# NEW EUROPEAN PATENT SPECIFICATION

(11) **EP 0 546 090 B2**
(45) Date of publication and mention of the opposition decision: **19.07.2006**
(45) Mention of the grant of the patent: 19.06.1996
(21) Application number: 91917090.2
(22) Date of filing: 28.08.1991
(51) Int. Cl.: C12N 15/54, C12N 15/82, C12N 5/10, A01H 5/00

(54) **GLYPHOSATE TOLERANT 5-ENOLPYRUVYLSHIKIMATE-3-PHOSPHATE SYNTHASES**
GLYPHOSATTOLERANTE 5-ENOLPYRUVYL-3-PHOSPHOSHIKIMAT-SYNTHASEN
SYNTHASES DE 5-ENOLPYRULVYLSHIKIMATE-3-PHOSPHATE TOLERANT LE GLYPHOSATE

(30) Priority: 31.08.1990 US 576537
(43) Date of publication of application: 16.06.1993
(73) Proprietor: Monsanto Company, St. Louis, Missouri 63167 (US)
(72) Inventor: BARRY, Gerard, Francis, St. Louis, MO 63130 (US); KISHORE, Ganesh, Murthy, Chesterfield, MO 63017 (US); PADGETTE, Stephen, Rogers, Labadie, MO 63055 (US)
(74) Representative: UEXKÜLL & STOLBERG
(86) International application number: PCT/US1991/006148
(87) International publication number: WO 1992/004449

(56) References cited:
- EP-A- 0 218 571
- EP-A- 0 293 358
- EP-A- 0 426 641
- WO-A-91/04323
- US-A- 4 769 061
- PLANT PHYSIOLOGY vol. 89, no.4, April 1989, ROCKVILLE, MD, USA. page 47; EICHHOLTZ, D.,ET AL.: "Glyphosate tolerant variants of petunia EPSP synthase"
- CHEMICAL ABSTRACTS, vol. 103, 1985, Columbus, Ohio, US; abstrct no. 119839, see abstract & FEMS MICROBIOL LETT vol. 28, no. 3, 1985, pages 297-301; SCHULZ, A., ET AL.:"Differential sensitivity of bacterial 5- enolpyruylshikimate 3-phosphate syntases to the herbicede glyphosate"
- CHEMICAL ABSTRACTS, vol. 112, 1990, Columbus, Ohio, US, abstact no. 92785, page 196;see abstract & DISSERTATION 1988, AVAIL. UNIV. MICROFILMS INT., ORDER no. DA 8917814. From Diss. abstr. int. B 1989,50(5), 1770-1771 FITZGIBBON, JOSEPH E. :"Pseudomonas strain PG2982: uptake of glyphosate and cloning of a gene which confers increased resistance to glyphosate"
- SAAS BULLETIN vol. 1, 1988, pages 37-40; LARSON-KELLY, N.,ET AL.:"Chloroplast delivery of a bacterial EPSP synthase in transgenic plants and tolerance to glyphosate"

### SEQUENCE LISTING

(1) GENERAL INFORMATION:
   (i) APPLICANT:
      Barry, Gerard F.
      Kishore, Ganesh M.
      Padgette, Stephen R.
   (ii) TITLE OF INVENTION: Glyphosate Tolerant
      5-Enolpyruvylshikimate-3-Phosphate Synthases
   (iii) NUMBER OF SEQUENCES: 36
   (iv) CORRESPONDENCE ADDRESS:
      (A) ADDRESSEE: Dennis R. Hoerner, Jr., Monsanto Co. BB4F
      (B) STREET: 700 Chesterfield Village Parkway
      (C) CITY: St. Louis
      (D) STATE: Missouri
      (E) COUNTRY: USA
      (F) ZIP: 63198
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentln Release #1.0, Version #1.25
   (vi) CURRENT APPLICATION DATA:
      (A) APPLICATION NUMBER: US 07/576537
      (B) FILING DATE: 31-AUG-1990
      (C) CLASSIFICATION:
   (viii) ATTORNEY/AGENT INFORMATION:
      (A) NAME: Hoerner Jr., Dennis R.
      (B) REGISTRATION NUMBER: 30,914
      (C) REFERENCE/DOCKET NUMBER: 38-21 (10535)
   (ix) TELECOMMUNICATION INFORMATION:
      (A) TELEPHONE: (314)537-6099
      (B) TELEFAX: (314)537-6047
(2) INFORMATION FOR SEQ ID NO:1:
   (i) SEQUENCE CHARACTERISTICS:
      (A) LENGTH: 597 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID N0:1:
(2) INFORMATION FOR SEQ ID NO:2:
   (i) SEQUENCE CHARACTERISTICS:
      (A) LENGTH: 1982 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAMElKEY: CDS
      (B) LOCATION: 62..1426
   (xi) SEQUENCE DESCRIPTION: SEQ ID NO:2:
   AAGCCCGCGT TCTCTCCGGC GCTCCGCCCG GAGAGCCGTG GATAGATTAA GGAAGACGCC        60
   C ATG TCG CAC GGT GCA AGC AGC CGG CCC GCA ACC GCC CGC AAA TCC            106
     Met Ser His Gly Ala Ser Ser Arg Pro Ala Thr Ala Arg Lys Ser
       1               5                  10                  15
   TCT GGC CTT TCC GGA ACC GTC CGC ATT CCC GGC GAC AAG TCG ATC TCC          154
   Ser Gly Leu Ser Gly Thr Val Arg Ile Pro Gly Asp Lys Ser Ile Ser
                   20                  25                  30
   CAC CGG TCC TTC ATG TTC GGC GGT CTC GCG AGC GGT GAA ACG CGC ATC          202
   His Arg Ser Phe Met Phe Gly Gly Leu Ala Ser Gly Glu Thr Arg Ile
               35                  40                  45
   ACC GGC CTT CTG GAA GGC GAG GAC GTC ATC AAT ACG GGC AAG GCC ATG      250
   Thr Gly Leu Leu Glu Gly Glu Asp Val Ile Asn Thr Gly Lys Ala Met
           50                  55                  60
   CAG GCC ATG GGC GCC AGG ATC CGT AAG GAA GGC GAC ACC TGG ATC ATC      298
   Gln Ala Met Gly Ala Arg Ile Arg Lys Glu Gly Asp Thr Trp Ile Ile
       65                  70                  75
   GAT GGC GTC GGC AAT GGC GGC CTC CTG GCG CCT GAG GCG CCG CTC GAT      346
   Asp Gly Val Gly Asn Gly Gly Leu Leu Ala Pro Glu Ala Pro Leu Asp
    80                  85                  90                  95
   TTC GGC AAT GCC GCC ACG GGC TGC CGC CTG ACC ATG GGC CTC GTC GGG      394
   Phe Gly Asn Ala Ala Thr Gly Cys Arg Leu Thr Met Gly Leu Val Gly
                   100                 105                 110
   GTC TAC GAT TTC GAC AGC ACC TTC ATC GGC GAC GCC TCG CTC ACA AAG      442
   Val Tyr Asp Phe Asp Ser Thr Phe Ile Gly Asp Ala Ser Leu Thr Lys
               115                 120                 125
   CGC CCG ATG GGC CGC GTG TTG AAC CCG CTG CGC GAA ATG GGC GTG CAG      490
   Arg Pro Met Gly Arg Val Leu Asn Pro Leu Arg Glu Met Gly Val Gln
           130                 135                 140
   GTG AAA TCG GAA GAC GGT GAC CGT CTT CCC GTT ACC TTG CGC GGG CCG      538
   Val Lys Ser Glu Asp Gly Asp Arg Leu Pro Val Thr Leu Arg Gly Pro
       145                 150                 155
   AAG ACG CCG ACG CCG ATC ACC TAC CGC GTG CCG ATG GCC TCC GCA CAG      586
   Lys Thr Pro Thr Pro Ile Thr Tyr Arg Val Pro Met Ala Ser Ala Gln
   160                 165                 170                 175
   GTG AAG TCC GCC GTG CTG CTC GCC GGC CTC AAC ACG CCC GGC ATC ACG      634
   Val Lys Ser Ala Val Leu Leu Ala Gly Leu Asn Thr Pro Gly Ile Thr
                   180                 185                 190
   ACG GTC ATC GAG CCG ATC ATG ACG CGC GAT CAT ACG GAA AAG ATG CTG      682
   Thr Val Ile Glu Pro Ile Met Thr Arg Asp His Thr Glu Lys Met Leu
               195                 200                 205
   CAG GGC TTT GGC GCC AAC CTT ACC GTC GAG ACG GAT GCG GAC GGC GTG      730
   Gln Gly Phe Gly Ala Asn Leu Thr Val Glu Thr Asp Ala Asp Gly Val
           210                 215                 220
   CGC ACC ATC CGC CTG GAA GGC CGC GGC AAG CTC ACC GGC CAA GTC ATC      778
   Arg Thr Ile Arg Leu Glu Gly Arg Gly Lys Leu Thr Gly Gln Val Ile
       225                 230                 235
   GAC GTG CCG GGC GAC CCG TCC TCG ACG GCC TTC CCG CTG GTT GCG GCC      826
   Asp Val Pro Gly Asp Pro Ser Ser Thr Ala Phe Pro Leu Val Ala Ala
   240                 245                 250                 255
   CTG CTT GTT CCG GGC TCC GAC GTC ACC ATC CTC AAC GTG CTG ATG AAC      874
   Leu Leu Val Pro Gly Ser Asp Val Thr Ile Leu Asn Val Leu Met Asn
                   260                 265                 270
   CCC ACC CGC ACC GGC CTC ATC CTG ACG CTG CAG GAA ATG GGC GCC GAC        922
   Pro Thr Arg Thr Gly Leu Ile Leu Thr Leu Gln Glu Met Gly Ala Asp
               275                     280                     285
   ATC GAA GTC ATC AAC CCG CGC CTT GCC GGC GGC GAA GAC GTG GCG GAC        970
   Ile Glu Val Ile Asn Pro Arg Leu Ala Gly Gly Glu Asp Val Ala Asp
           290                     295                     300
   CTG CGC GTT CGC TCC TCC ACG CTG AAG GGC GTC ACG GTG CCG GAA GAC       1018
   Leu Arg Val Arg Ser Ser Thr Leu Lys Gly Val Thr Val Pro Glu Asp
       305                     310                     315
   CGC GCG CCT TCG ATG ATC GAC GAA TAT CCG ATT CTC GCT GTC GCC GCC       1066
   Arg Ala Pro Ser Met Ile Asp Glu Tyr Pro Ile Leu Ala Val Ala Ala
   320                     325                     330                     335
   GCC TTC GCG GAA GGG GCG ACC GTG ATG AAC GGT CTG GAA GAA CTC CGC       1114
   Ala Phe Ala Glu Gly Ala Thr Val Met Asn Gly Leu Glu Glu Leu Arg
                   340                     345                     350
   GTC AAG GAA AGC GAC CGC CTC TCG GCC GTC GCC AAT GGC CTC AAG CTC       1162
   Val Lys Glu Ser Asp Arg Leu Ser Ala Val Ala Asn Gly Leu Lys Leu
               355                     360                     365
   AAT GGC GTG GAT TGC GAT GAG GGC GAG ACG TCG CTC GTC GTG CGC GGC       1210
   Asn Gly Val Asp Cys Asp Glu Gly Glu Thr Ser Leu Val Val Arg Gly
           370                     375                     380
   CGC CCT GAC GGC AAG GGG CTC GGC AAC GCC TCG GGC GCC GCC GTC GCC       1258
   Arg Pro Asp Gly Lys Gly Leu Gly Asn Ala Ser Gly Ala Ala Val Ala
       385                     390                     395
   ACC CAT CTC GAT CAC CGC ATC GCC ATG AGC TTC CTC GTC ATG GGC CTC       1306
   Thr His Leu Asp His Arg Ile Ala Met Ser Phe Leu Val Met Gly Leu
   400                     405                     410                     415
   GTG TCG GAA AAC CCT GTC ACG GTG GAC GAT GCC ACG ATG ATC GCC ACG       1354
   Val Ser Glu Asn Pro Val Thr Val Asp Asp Ala Thr Met Ile Ala Thr
                   420                     425                     430
   AGC TTC CCG GAG TTC ATG GAC CTG ATG GCC GGG CTG GGC GCG AAG ATC       1402
   Ser Phe Pro Glu Phe Met Asp Leu Met Ala Gly Leu Gly Ala Lys Ile
               435                     440                     445
   GAA CTC TCC GAT ACG AAG GCT GCC TGATGACCTT CACAATCGCC ATCGATGGTC       1456
   Glu Leu Ser Asp Thr Lys Ala Ala
           450                     455
   CCGCTGCGGC CGGCAAGGGG ACGCTCTCGC GCCGTATCGC GGAGGTCTAT GGCTTTCATC      1516
   ATCTCGATAC GGGCCTGACC TATCGCGCCA CGGCCAAAGC GCTGCTCGAT CGCGGCCTGT      1576
   CGCTTGATGA CGAGGCGGTT GCGGCCGATG TCGCCCGCAA TCTCGATCTT GCCGGGCTCG      1636
   ACCGGTCGGT GCTGTCGGCC CATGCCATCG GCGAGGCGGC TTCGAAGATC GCGGTCATGC      1696
   CCTCGGTGCG GCGGGCGCTG GTCGAGGCGC AGCGCAGCTT TGCGGCGCGT GAGCCGGGCA      1756
   CGGTGCTGGA TGGACGCGAT ATCGGCACGG TGGTCTGCCC GGATGCGCCG GTGAAGCTCT 1816
   ATGTCACCGC GTCACCGGAA GTGCGCGCGA AACGCCGCTA TGACGAAATC CTCGGCAATG 1876
   GCGGGTTGGC CGATTACGGG ACGATCCTCG AGGATATCCG CCGCCGCGAC GAGCGGGACA 1936
   TGGGTCGGGC GGACAGTCCT TTGAAGCCCG CCGACGATGC GCACTT 1982
(2) INFORMATION FOR SEQ ID NO:3:
   (i) SEQUENCE CHARACTERISTICS:
      (A) LENGTH: 455 amino acids
      (B) TYPE: amino acid
      (D) TOPOLOGY: linear
   (ii) MOLECULE TYPE: protein
   (xi) SEQUENCE DESCRIPTION: SEQ ID NO:3:
   Met Ser His Gly Ala Ser Ser Arg Pro Ala Thr Ala Arg Lys Ser Ser
   1 5 10 15
   Gly Leu Ser Gly Thr Val Arg Ile Pro Gly Asp Lys Ser Ile Ser His
   20 25 30
   Arg Ser Phe Met Phe Gly Gly Leu Ala Ser Gly Glu Thr Arg Ile Thr
   35 40 45
   Gly Leu Leu Glu Gly Glu Asp Val Ile Asn Thr Gly Lys Ala Met Gln
   50 55 60
   Ala Met Gly Ala Arg Ile Arg Lys Glu Gly Asp Thr Trp Ile Ile Asp
   65 70 75 80
   Gly Val Gly Asn Gly Gly Leu Leu Ala Pro Glu Ala Pro Leu Asp Phe
   85 90 95
   Gly Asn Ala Ala Thr Gly Cys Arg Leu Thr Met Gly Leu Val Gly Val
   100 105 110
   Tyr Asp Phe Asp Ser Thr Phe Ile Gly Asp Ala Ser Leu Thr Lys Arg
   115 120 125
   Pro Met Gly Arg Val Leu Asn Pro Leu Arg Glu Met Gly Val Gln Val
   130 135 140
   Lys Ser Glu Asp Gly Asp Arg Leu Pro Val Thr Leu Arg Gly Pro Lys
   145 150 155 160
   Thr Pro Thr Pro Ile Thr Tyr Arg Val Pro Met Ala Ser Ala Gln Val
   165 170 175
   Lys Ser Ala Val Leu Leu Ala Gly Leu Asn Thr Pro Gly Ile Thr Thr
   180 185 190
   Val Ile Glu Pro Ile Met Thr Arg Asp His Thr Glu Lys Met Leu Gln
           195                 200                 205
   Gly Phe Gly Ala Asn Leu Thr Val Glu Thr Asp Ala Asp Gly Val Arg
       210                 215                 220
   Thr Ile Arg Leu Glu Gly Arg Gly Lys Leu Thr Gly Gln Val Ile Asp
   225                 230                 235                 240
   Val Pro Gly Asp Pro Ser Ser Thr Ala Phe Pro Leu Val Ala Ala Leu
                   245                 250                 255
   Leu Val Pro Gly Ser Asp Val Thr Ile Leu Asn Val Leu Met Asn Pro
               260                 265                 270
   Thr Arg Thr Gly Leu Ile Leu Thr Leu Gln Glu Met Gly Ala Asp Ile
           275                 280                 285
   Glu Val Ile Asn Pro Arg Leu Ala Gly Gly Glu Asp Val Ala Asp Leu
       290                 295                 300
   Arg Val Arg Ser Ser Thr Leu Lys Gly Val Thr Val Pro Glu Asp Arg
   305                 310                 315                 320
   Ala Pro Ser Met Ile Asp Glu Tyr Pro Ile Leu Ala Val Ala Ala Ala
                   325                 330                 335
   Phe Ala Glu Gly Ala Thr Val Met Asn Gly Leu Glu Glu Leu Arg Val
               340                 345                 350
   Lys Glu Ser Asp Arg Leu Ser Ala Val Ala Asn Gly Leu Lys Leu Asn
           355                 360                 365
   Gly Val Asp Cys Asp Glu Gly Glu Thr Ser Leu Val Val Arg Gly Arg
       370                 375                 380
   Pro Asp Gly Lys Gly Leu Gly Asn Ala Ser Gly Ala Ala Val Ala Thr
   385                 390                 395                 400
   His Leu Asp His Arg Ile Ala Met Ser Phe Leu Val Met Gly Leu Val
                   405                 410                 415
   Ser Glu Asn Pro Val Thr Val Asp Asp Ala Thr Met Ile Ala Thr Ser
               420                 425                 430
   Phe Pro Glu Phe Met Asp Leu Met Ala Gly Leu Gly Ala Lys Ile Glu
           435                 440                 445
   Leu Ser Asp Thr Lys Ala Ala
       450                 455
(2) INFORMATION FOR SEQ ID NO:4:
   (i) SEQUENCE CHARACTERISTICS:
      (A) LENGTH: 1673 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 86..1432
   (xi) SEQUENCE DESCRIPTION: SEQ ID NO:4:
   GTAGCCACAC ATAATTACTA TAGCTAGGAA GCCCGCTATC TCTCAATCCC GCGTGATCGC        60
   GCCAAAATGT GACTGTGAAA AATCC ATG TCC CAT TCT GCA TCC CCG AAA CCA         112
                               Met Ser His Ser Ala Ser Pro Lys Pro
                                 1               5
   GCA ACC GCC CGC CGC TCG GAG GCA CTC ACG GGC GAA ATC CGC ATT CCG         160
   Ala Thr Ala Arg Arg Ser Glu Ala Leu Thr Gly Glu Ile Arg Ile Pro
    10                  15                  20                  25
   GGC GAC AAG TCC ATC TCG CAT CGC TCC TTC ATG TTT GGC GGT CTC GCA         208
   Gly Asp Lys Ser Ile Ser His Arg Ser Phe Met Phe Gly Gly Leu Ala
                        30                  35                  40
   TCG GGC GAA ACC CGC ATC ACC GGC CTT CTG GAA GGC GAG GAC GTC ATC         256
   Ser Gly Glu Thr Arg Ile Thr Gly Leu Leu Glu Gly Glu Asp Val Ile
                45                  50                  55
   AAT ACA GGC CGC GCC ATG CAG GCC ATG GGC GCG AAA ATC CGT AAA GAG         304
   Asn Thr Gly Arg Ala Met Gln Ala Met Gly Ala Lys Ile Arg Lys Glu
            60                  65                  70
   GGC GAT GTC TGG ATC ATC AAC GGC GTC GGC AAT GGC TGC CTG TTG CAG         352
   Gly Asp Val Trp Ile Ile Asn Gly Val Gly Asn Gly Cys Leu Leu Gln
        75                  80                  85
   CCC GAA GCT GCG CTC GAT TTC GGC AAT GCC GGA ACC GGC GCG CGC CTC         400
   Pro Glu Ala Ala Leu Asp Phe Gly Asn Ala Gly Thr Gly Ala Arg Leu
    90                  95                 100                 105
   ACC ATG GGC CTT GTC GGC ACC TAT GAC ATG AAG ACC TCC TTT ATC GGC         448
   Thr Met Gly Leu Val Gly Thr Tyr Asp Met Lys Thr Ser Phe Ile Gly
                   110                 115                 120
   GAC GCC TCG CTG TCG AAG CGC CCG ATG GGC CGC GTG CTG AAC CCG TTG         496
   Asp Ala Ser Leu Ser Lys Arg Pro Met Gly Arg Val Leu Asn Pro Leu
               125                 130                 135
   CGC GAA ATG GGC GTT CAG GTG GAA GCA GCC GAT GGC GAC CGC ATG CCG         544
   Arg Glu Met Gly Val Gln Val Glu Ala Ala Asp Gly Asp Arg Met Pro
           140                 145                 150
   CTG ACG CTG ATC GGC CCG AAG ACG GCC AAT CCG ATC ACC TAT CGC GTG      592
   Leu Thr Leu Ile Gly Pro Lys Thr Ala Asn Pro Ile Thr Tyr Arg Val
       155                 160                 165
   CCG ATG GCC TCC GCG CAG GTA AAA TCC GCC GTG CTG CTC GCC GGT CTC      640
   Pro Met Ala Ser Ala Gln Val Lys Ser Ala Val Leu Leu Ala Gly Leu
   170                 175                 180                 185
   AAC ACG CCG GGC GTC ACC ACC GTC ATC GAG CCG GTC ATG ACC CGC GAC      688
   Asn Thr Pro Gly Val Thr Thr Val Ile Glu Pro Val Met Thr Arg Asp
                   190                 195                 200
   CAC ACC GAA AAG ATG CTG CAG GGC TTT GGC GCC GAC CTC ACG GTC GAG      736
   His Thr Glu Lys Met Leu Gln Gly Phe Gly Ala Asp Leu Thr Val Glu
               205                 210                 215
   ACC GAC AAG GAT GGC GTG CGC CAT ATC CGC ATC ACC GGC CAG GGC AAG      784
   Thr Asp Lys Asp Gly Val Arg His Ile Arg Ile Thr Gly Gln Gly Lys
           220                 225                 230
   CTT GTC GGC CAG ACC ATC GAC GTG CCG GGC GAT CCG TCA TCG ACC GCC      832
   Leu Val Gly Gln Thr Ile Asp Val Pro Gly Asp Pro Ser Ser Thr Ala
       235                 240                 245
   TTC CCG CTC GTT GCC GCC CTT CTG GTG GAA GGT TCC GAC GTC ACC ATC      880
   Phe Pro Leu Val Ala Ala Leu Leu Val Glu Gly Ser Asp Val Thr Ile
   250                 255                 260                 265
   CGC AAC GTG CTG ATG AAC CCG ACC CGT ACC GGC CTC ATC CTC ACC TTG      928
   Arg Asn Val Leu Met Asn Pro Thr Arg Thr Gly Leu Ile Leu Thr Leu
                   270                 275                 280
   CAG GAA ATG GGC GCC GAT ATC GAA GTG CTC AAT GCC CGT CTT GCA GGC      976
   Gln Glu Met Gly Ala Asp Ile Glu Val Leu Asn Ala Arg Leu Ala Gly
               285                 290                 295
   GGC GAA GAC GTC GCC GAT CTG CGC GTC AGG GCT TCG AAG CTC AAG GGC     1024
   Gly Glu Asp Val Ala Asp Leu Arg Val Arg Ala Ser Lys Leu Lys Gly
           300                 305                 310
   GTC GTC GTT CCG CCG GAA CGT GCG CCG TCG ATG ATC GAC GAA TAT CCG     1072
   Val Val Val Pro Pro Glu Arg Ala Pro Ser Met Ile Asp Glu Tyr Pro
       315                 320                 325
   GTC CTG GCG ATT GCC GCC TCC TTC GCG GAA GGC GAA ACC GTG ATG GAC     1120
   Val Leu Ala Ile Ala Ala Ser Phe Ala Glu Gly Glu Thr Val Met Asp
   330                 335                 340                 345
   GGG CTC GAC GAA CTG CGC GTC AAG GAA TCG GAT CGT CTG GCA GCG GTC     1168
   Gly Leu Asp Glu Leu Arg Val Lys Glu Ser Asp Arg Leu Ala Ala Val
                   350                 355                 360
   GCA CGC GGC CTT GAA GCC AAC GGC GTC GAT TGC ACC GAA GGC GAG ATG     1216
   Ala Arg Gly Leu Glu Ala Asn Gly Val Asp Cys Thr Glu Gly Glu Met
               365                 370                 375
   TCG CTG ACG GTT CGC GGC CGC CCC GAC GGC AAG GGA CTG GGC GGC GGC 1264
   Ser Leu Thr Val Arg Gly Arg Pro Asp Gly Lys Gly Leu Gly Gly Gly
   380 385 390
   ACG GTT GCA ACC CAT CTC GAT CAT CGT ATC GCG ATG AGC TTC CTC GTG 1312
   Thr Val Ala Thr His Leu Asp His Arg Ile Ala Met Ser Phe Leu Val
   395 400 405
   ATG GGC CTT GCG GCG GAA AAG CCG GTG ACG GTT GAC GAC AGT AAC ATG 1360
   Met Gly Leu Ala Ala Glu Lys Pro Val Thr Val Asp Asp Ser Asn Met
   410 415 420 425
   ATC GCC ACG TCC TTC CCC GAA TTC ATG GAC ATG ATG CCG GGA TTG GGC 1408
   Ile Ala Thr Ser Phe Pro Glu Phe Met Asp Met Met Pro Gly Leu Gly
   430 435 440
   GCA AAG ATC GAG TTG AGC ATA CTC TAGTCACTCG ACAGCGAAAA TATTATTTGC 1462
   Ala Lys Ile Glu Leu Ser Ile Leu
   445
   GAGATTGGGC ATTATTACCG GTTGGTCTCA GCGGGGGTTT AATGTCCAAT CTTCCATACG 1522
   TAACAGCATC AGGAAATATC AAAAAAGCTT TAGAAGGAAT TGCTAGAGCA GCGACGCCGC 1582
   CTAAGCTTTC TCAAGACTTC GTTAAAACTG TACTGAAATC CCGGGGGGTC CGGGGATCAA 1642
   ATGACTTCAT TTCTGAGAAA TTGGCCTCGC A 1673
(2) INFORMATION FOR SEQ ID NO:5:
   (i) SEQUENCE CHARACTERISTICS:
      (A) LENGTH: 449 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Met Ser His Ser Ala Ser Pro Lys Pro Ala Thr Ala Arg Arg Ser Glu
   1 5 10 15
   Ala Leu Thr Gly Glu Ile Arg Ile Pro Gly Asp Lys Ser Ile Ser His
   20 25 30
   Arg Ser Phe Met Phe Gly Gly Leu Ala Ser Gly Glu Thr Arg Ile Thr
   35 40 45
   Gly Leu Leu Glu Gly Glu Asp Val Ile Asn Thr Gly Arg Ala Met Gln
   50 55 60
   Ala Met Gly Ala Lys Ile Arg Lys Glu Gly Asp Val Trp Ile Ile Asn
   65 70 75 80
   Gly Val Gly Asn Gly Cys Leu Leu Gln Pro Glu Ala Ala Leu Asp Phe
   85 90 95
   Gly Asn Ala Gly Thr Gly Ala Arg Leu Thr Met Gly Leu Val Gly Thr
   100 105 110
   Tyr Asp Met Lys Thr Ser Phe Ile Gly Asp Ala Ser Leu Ser Lys Arg
   115 120 125
   Pro Met Gly Arg Val Leu Asn Pro Leu Arg Glu Met Gly Val Gln Val
   130 135 140
   Glu Ala Ala Asp Gly Asp Arg Met Pro Leu Thr Leu Ile Gly Pro Lys
   145 150 155 160
   Thr Ala Asn Pro Ile Thr Tyr Arg Val Pro Met Ala Ser Ala Gln Val
   165 170 175
   Lys Ser Ala Val Leu Leu Ala Gly Leu Asn Thr Pro Gly Val Thr Thr
   180 185 190
   Val Ile Glu Pro Val Met Thr Arg Asp His Thr Glu Lys Met Leu Gln
   195 200 205
   Gly Phe Gly Ala Asp Leu Thr Val Glu Thr Asp Lys Asp Gly Val Arg
   210 215 220
   His Ile Arg Ile Thr Gly Gln Gly Lys Leu Val Gly Gln Thr Ile Asp
   225 230 235 240
   Val Pro Gly Asp Pro Ser Ser Thr Ala Phe Pro Leu Val Ala Ala Leu
   245 250 255
   Leu Val Glu Gly Ser Asp Val Thr Ile Arg Asn Val Leu Met Asn Pro
   260 265 270
   Thr Arg Thr Gly Leu Ile Leu Thr Leu Gln Glu Met Gly Ala Asp Ile
   275 280 285
   Glu Val Leu Asn Ala Arg Leu Ala Gly Gly Glu Asp Val Ala Asp Leu
   290 295 300
   Arg Val Arg Ala Ser Lys Leu Lys Gly Val Val Val Pro Pro Glu Arg
   305 310 315 320
   Ala Pro Ser Met Ile Asp Glu Tyr Pro Val Leu Ala Ile Ala Ala Ser
   325 330 335
   Phe Ala Glu Gly Glu Thr Val Met Asp Gly Leu Asp Glu Leu Arg Val
   340 345 350
   Lys Glu Ser Asp Arg Leu Ala Ala Val Ala Arg Gly Leu Glu Ala Asn
   355 360 365
   Gly Val Asp Cys Thr Glu Gly Glu Met Ser Leu Thr Val Arg Gly Arg
   370 375 380
   Pro Asp Gly Lys Gly Leu Gly Gly Gly Thr Val Ala Thr His Leu Asp
   385 390 395 400
   His Arg Ile Ala Met Ser Phe Leu Val Met Gly Leu Ala Ala Glu Lys
   405 410 415
   Pro Val Thr Val Asp Asp Ser Asn Met Ile Ala Thr Ser Phe Pro Glu
   420 425 430
   Phe Met Asp Met Met Pro Gly Leu Gly Ala Lys Ile Glu Leu Ser Ile
   435 440 445
   Leu
(2) INFORMATION FOR SEQ ID NO:6:
   (i) SEQUENCE CHARACTERISTICS:
      (A) LENGTH: 1500 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 34..1380
   (xi) SEQUENCE DESCRIPTION: SEQ ID NO:6:
   GTGATCGCGC CAAAATGTGA CTGTGAAAAA TCC ATG TCC CAT TCT GCA TCC CCG        54
                                        Met Ser His Ser Ala Ser Pro
                                         1               5
   AAA CCA GCA ACC GCC CGC CGC TCG GAG GCA CTC ACG GGC GAA ATC CGC        102
   Lys Pro Ala Thr Ala Arg Arg Ser Glu Ala Leu Thr Gly Glu Ile Arg
           10                  15                  20
   ATT CCG GGC GAC AAG TCC ATC TCG CAT CGC TCC TTC ATG TTT GGC GGT        150
   Ile Pro Gly Asp Lys Ser Ile Ser His Arg Ser Phe Met Phe Gly Gly
       25                  30                  35
   CTC GCA TCG GGC GAA ACC CGC ATC ACC GGC CTT CTG GAA GGC GAG GAC        198
   Leu Ala Ser Gly Glu Thr Arg Ile Thr Gly Leu Leu Glu Gly Glu Asp
    40                  45                  50                  55
   GTC ATC AAT ACA GGC CGC GCC ATG CAG GCC ATG GGC GCG AAA ATC CGT        246
   Val Ile Asn Thr Gly Arg Ala Met Gln Ala Met Gly Ala Lys Ile Arg
                    60                  65                  70
   AAA GAG GGC GAT GTC TGG ATC ATC AAC GGC GTC GGC AAT GGC TGC CTG        294
   Lys Glu Gly Asp Val Trp Ile Ile Asn Gly Val Gly Asn Gly Cys Leu
               75                  80                  85
   TTG CAG CCC GAA GCT GCG CTC GAT TTC GGC AAT GCC GGA ACC GGC GCG        342
   Leu Gln Pro Glu Ala Ala Leu Asp Phe Gly Asn Ala Gly Thr Gly Ala
           90                  95                 100
   CGC CTC ACC ATG GGC CTT GTC GGC ACC TAT GAC ATG AAG ACC TCC TTT      390
   Arg Leu Thr Met Gly Leu Val Gly Thr Tyr Asp Met Lys Thr Ser Phe
       105                 110                 115
   ATC GGC GAC GCC TCG CTG TCG AAG CGC CCG ATG GGC CGC GTG CTG AAC      438
   Ile Gly Asp Ala Ser Leu Ser Lys Arg Pro Met Gly Arg Val Leu Asn
   120                 125                 130                 135
   CCG TTG CGC GAA ATG GGC GTT CAG GTG GAA GCA GCC GAT GGC GAC CGC      486
   Pro Leu Arg Glu Met Gly Val Gln Val Glu Ala Ala Asp Gly Asp Arg
                   140                 145                 150
   ATG CCG CTG ACG CTG ATC GGC CCG AAG ACG GCC AAT CCG ATC ACC TAT      534
   Met Pro Leu Thr Leu Ile Gly Pro Lys Thr Ala Asn Pro Ile Thr Tyr
               155                 160                 165
   CGC GTG CCG ATG GCC TCC GCG CAG GTA AAA TCC GCC GTG CTG CTC GCC      582
   Arg Val Pro Met Ala Ser Ala Gln Val Lys Ser Ala Val Leu Leu Ala
           170                 175                 180
   GGT CTC AAC ACG CCG GGC GTC ACC ACC GTC ATC GAG CCG GTC ATG ACC      630
   Gly Leu Asn Thr Pro Gly Val Thr Thr Val Ile Glu Pro Val Met Thr
       185                 190                 195
   CGC GAC CAC ACC GAA AAG ATG CTG CAG GGC TTT GGC GCC GAC CTC ACG      678
   Arg Asp His Thr Glu Lys Met Leu Gln Gly Phe Gly Ala Asp Leu Thr
   200                 205                 210                 215
   GTC GAG ACC GAC AAG GAT GGC GTG CGC CAT ATC CGC ATC ACC GGC CAG      726
   Val Glu Thr Asp Lys Asp Gly Val Arg His Ile Arg Ile Thr Gly Gln
                   220                 225                 230
   GGC AAG CTT GTC GGC CAG ACC ATC GAC GTG CCG GGC GAT CCG TCA TCG      774
   Gly Lys Leu Val Gly Gln Thr Ile Asp Val Pro Gly Asp Pro Ser Ser
               235                 240                 245
   ACC GCC TTC CCG CTC GTT GCC GCC CTT CTG GTG GAA GGT TCC GAC GTC      822
   Thr Ala Phe Pro Leu Val Ala Ala Leu Leu Val Glu Gly Ser Asp Val
           250                 255                 260
   ACC ATC CGC AAC GTG CTG ATG AAC CCG ACC CGT ACC GGC CTC ATC CTC      870
   Thr Ile Arg Asn Val Leu Met Asn Pro Thr Arg Thr Gly Leu Ile Leu
       265                 270                 275
   ACC TTG CAG GAA ATG GGC GCC GAT ATC GAA GTG CTC AAT GCC CGT CTT      918
   Thr Leu Gln Glu Met Gly Ala Asp Ile Glu Val Leu Asn Ala Arg Leu
   280                 285                 290                 295
   GCA GGC GGC GAA GAC GTC GCC GAT CTG CGC GTC AGG GCT TCG AAG CTC      966
   Ala Gly Gly Glu Asp Val Ala Asp Leu Arg Val Arg Ala Ser Lys Leu
                   300                 305                 310
   AAG GGC GTC GTC GTT CCG CCG GAA CGT GCG CCG TCG ATG ATC GAC GAA     1014
   Lys Gly Val Val Val Pro Pro Glu Arg Ala Pro Ser Met Ile Asp Glu
               315                 320                 325
   TAT CCG GTC CTG GCG ATT GCC GCC TCC TTC GCG GAA GGC GAA ACC GTG        1062
   Tyr Pro Val Leu Ala Ile Ala Ala Ser Phe Ala Glu Gly Glu Thr Val
           330                 335                 340
   ATG GAC GGG CTC GAC GAA CTG CGC GTC AAG GAA TCG GAT CGT CTG GCA        1110
   Met Asp Gly Leu Asp Glu Leu Arg Val Lys Glu Ser Asp Arg Leu Ala
       345                 350                 355
   GCG GTC GCA CGC GGC CTT GAA GCC AAC GGC GTC GAT TGC ACC GAA GGC        1158
   Ala Val Ala Arg Gly Leu Glu Ala Asn Gly Val Asp Cys Thr Glu Gly
   360                 365                 370                 375
   GAG ATG TCG CTG ACG GTT CGC GGC CGC CCC GAC GGC AAG GGA CTG GGC        1206
   Glu Met Ser Leu Thr Val Arg Gly Arg Pro Asp Gly Lys Gly Leu Gly
                   380                 385                 390
   GGC GGC ACG GTT GCA ACC CAT CTC GAT CAT CGT ATC GCG ATG AGC TTC        1254
   Gly Gly Thr Val Ala Thr His Leu Asp His Arg Ile Ala Met Ser Phe
               395                 400                 405
   CTC GTG ATG GGC CTT GCG GCG GAA AAG CCG GTG ACG GTT GAC GAC AGT        1302
   Leu Val Met Gly Leu Ala Ala Glu Lys Pro Val Thr Val Asp Asp Ser
           410                 415                 420
   AAC ATG ATC GCC ACG TCC TTC CCC GAA TTC ATG GAC ATG ATG CCG GGA        1350
   Asn Met Ile Ala Thr Ser Phe Pro Glu Phe Met Asp Met Met Pro Gly
       425                 430                 435
   TTG GGC GCA AAG ATC GAG TTG AGC ATA CTC TAGTCACTCG ACAGCGAAAA          1400
   Leu Gly Ala Lys Ile Glu Leu Ser Ile Leu
   440                 445
   TATTATTTGC GAGATTGGGC ATTATTACCG GTTGGTCTCA GCGGGGGTTT AATGTCCAAT      1460
   CTTCCATACG TAACAGCATC AGGAAATATC AAAAAAGCTT                            1500
(2) INFORMATION FOR SEQ ID NO:7:
   (i) SEQUENCE CHARACTERISTICS:
      (A) LENGTH: 449 amino acids
      (B) TYPE: amino acid
      (D) TOPOLOGY: linear
   (ii) MOLECULE TYPE: protein
   (xi) SEQUENCE DESCRIPTION: SEQ ID NO:7:
   Met Ser His Ser Ala Ser Pro Lys Pro Ala Thr Ala Arg Arg Ser Glu
   1               5                   10                  15
   Ala Leu Thr Gly Glu Ile Arg Ile Pro Gly Asp Lys Ser Ile Ser His
               20                  25                  30
   Arg Ser Phe Met Phe Gly Gly Leu Ala Ser Gly Glu Thr Arg Ile Thr
           35                  40                  45
   Gly Leu Leu Glu Gly Glu Asp Val Ile Asn Thr Gly Arg Ala Met Gln
   50 55 60
   Ala Met Gly Ala Lys Ile Arg Lys Glu Gly Asp Val Trp Ile Ile Asn
   65 70 75 80
   Gly Val Gly Asn Gly Cys Leu Leu Gln Pro Glu Ala Ala Leu Asp Phe
   85 90 95
   Gly Asn Ala Gly Thr Gly Ala Arg Leu Thr Met Gly Leu Val Gly Thr
   100 105 110
   Tyr Asp Met Lys Thr Ser Phe Ile Gly Asp Ala Ser Leu Ser Lys Arg
   115 120 125
   Pro Met Gly Arg Val Leu Asn Pro Leu Arg Glu Met Gly Val Gln Val
   130 135 140
   Glu Ala Ala Asp Gly Asp Arg Met Pro Leu Thr Leu Ile Gly Pro Lys
   145 150 155 160
   Thr Ala Asn Pro Ile Thr Tyr Arg Val Pro Met Ala Ser Ala Gln Val
   165 170 175
   Lys Ser Ala Val Leu Leu Ala Gly Leu Asn Thr Pro Gly Val Thr Thr
   180 185 190
   Val Ile Glu Pro Val Met Thr Arg Asp His Thr Glu Lys Met Leu Gln
   195 200 205
   Gly Phe Gly Ala Asp Leu Thr Val Glu Thr Asp Lys Asp Gly Val Arg
   210 215 220
   His Ile Arg Ile Thr Gly Gln Gly Lys Leu Val Gly Gln Thr Ile Asp
   225 230 235 240
   Val Pro Gly Asp Pro Ser Ser Thr Ala Phe Pro Leu Val Ala Ala Leu
   245 250 255
   Leu Val Glu Gly Ser Asp Val Thr Ile Arg Asn Val Leu Met Asn Pro
   260 265 270
   Thr Arg Thr Gly Leu Ile Leu Thr Leu Gln Glu Met Gly Ala Asp Ile
   275 280 285
   Glu Val Leu Asn Ala Arg Leu Ala Gly Gly Glu Asp Val Ala Asp Leu
   290 295 300
   Arg Val Arg Ala Ser Lys Leu Lys Gly Val Val Val Pro Pro Glu Arg
   305 310 315 320
   Ala Pro Ser Met Ile Asp Glu Tyr Pro Val Leu Ala Ile Ala Ala Ser
   325 330 335
   Phe Ala Glu Gly Glu Thr Val Met Asp Gly Leu Asp Glu Leu Arg Val
   340 345 350
   Lys Glu Ser Asp Arg Leu Ala Ala Val Ala Arg Gly Leu Glu Ala Asn
   355 360 365
   Gly Val Asp Cys Thr Glu Gly Glu Met Ser Leu Thr Val Arg Gly Arg
   370 375 380
   Pro Asp Gly Lys Gly Leu Gly Gly Gly Thr Val Ala Thr His Leu Asp
   385 390 395 400
   His Arg Ile Ala Met Ser Phe Leu Val Met Gly Leu Ala Ala Glu Lys
   405 410 415
   Pro Val Thr Val Asp Asp Ser Asn Met Ile Ala Thr Ser Phe Pro Glu
   420 425 430
   Phe Met Asp Met Met Pro Gly Leu Gly Ala Lys Ile Glu Leu Ser Ile
   435 440 445
   Leu
(2) INFORMATION FOR SEQ ID NO:8:
   (i) SEQUENCE CHARACTERISTICS:
      (A) LENGTH: 423 amino acids.
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:8:
   Ser Leu Thr Leu Gln Pro Ile Ala Arg Val Asp Gly Thr Ile Asn Leu
   1 5 10 15
   Pro Gly Ser Lys Thr Val Ser Asn Arg Ala Leu Leu Leu Ala Ala Leu
   20 25 30
   Ala His Gly Lys Thr Val Leu Thr Asn Leu Leu Asp Ser Asp Asp Val
   35 40 45
   Arg His Met Leu Asn Ala Leu Thr Ala Leu Gly Val Ser Tyr Thr Leu
   50 55 60
   Ser Ala Asp Arg Thr Arg Cys Glu Ile Ile Gly Asn Gly Gly Pro Leu
   65 70 75 80
   His Ala Glu Gly Ala Leu Glu Leu Phe Leu Gly Asn Ala Gly Thr Ala
   85 90 95
   Met Arg Pro Leu Ala Ala Ala Leu Cys Leu Gly Ser Asn Asp Ile Val
   100 105 110
   Leu Thr Gly Glu Pro Arg Met Lys Glu Arg Pro Ile Gly His Leu Val
   115 120 125
   Asp Ala Leu Arg Leu Gly Gly Ala Lys Ile Thr Tyr Leu Glu Gln Glu
   130 135 140
   Asn Tyr Pro Pro Leu Arg Leu Gln Gly Gly Phe Thr Gly Gly Asn Val
   145 150 155 160
   Asp Val Asp Gly Ser Val Ser Ser Gln Phe Leu Thr Ala Leu Leu Met
   165 170 175
   Thr Ala Pro Leu Ala Pro Glu Asp Thr Val Ile Arg Ile Lys Gly Asp
   180 185 190
   Leu Val Ser Lys Pro Tyr Ile Asp Ile Thr Leu Asn Leu Met Lys Thr
   195 200 205
   Phe Gly Val Glu Ile Glu Asn Gln His Tyr Gln Gln Phe Val Val Lys
   210 215 220
   Gly Gly Gln Ser Tyr Gln Ser Pro Gly Thr Tyr Leu Val Glu Gly Asp
   225 230 235 240
   Ala Ser Ser Ala Ser Tyr Phe Leu Ala Ala Ala Ala Ile Lys Gly Gly
   245 250 255
   Thr Val Lys Val Thr Gly Ile Gly Arg Asn Ser Met Gln Gly Asp Ile
   260 265 270
   Arg Phe Ala Asp Val Leu Glu Lys Met Gly Ala Thr Ile Cys Trp Gly
   275 280 285
   Asp Asp Tyr Ile Ser Cys Thr Arg Gly Glu Leu Asn Ala Ile Asp Met
   290 295 300
   Asp Met Asn His Ile Pro Asp Ala Ala Met Thr Ile Ala Thr Ala Ala
   305 310 315 320
   Leu Phe Ala Lys Gly Thr Thr Arg Leu Arg Asn Ile Tyr Asn Trp Arg
   325 330 335
   Val Lys Glu Thr Asp Arg Leu Phe Ala Met Ala Thr Glu Leu Arg Lys
   340 345 350
   Val Gly Ala Glu Val Glu Glu Gly His Asp Tyr Ile Arg Ile Thr Pro
   355 360 365
   Pro Glu Lys Leu Asn Phe Ala Glu Ile Ala Thr Tyr Asn Asp His Arg
   370 375 380
   Met Ala Met Cys Phe Ser Leu Val Ala Leu Ser Asp Thr Pro Val Thr
   385 390 395 400
   Ile Leu Asp Pro Lys Cys Thr Ala Lys Thr Phe Pro Asp Tyr Phe Glu
   405 410 415
   Gln Leu Ala Arg Ile Ser Gln
   420
(2) INFORMATION FOR SEQ ID NO:9:
   (i) SEQUENCE CHARACTERISTICS:
      (A) LENGTH: 1377 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:9:
   CCATGGCTCA CGGTGCAAGC AGCCGTCCAG CAACTGCTCG TAAGTCCTCT GGTCTTTCTG     60
   GAACCGTCCG TATTCCAGGT GACAAGTCTA TCTCCCACAG GTCCTTCATG TTTGGAGGTC    120
   TCGCTAGCGG TGAAACTCGT ATCACCGGTC TTTTGGAAGG TGAAGATGTT ATCAACACTG    180
   GTAAGGCTAT GCAAGCTATG GGTGCCAGAA TCCGTAAGGA AGGTGATACT TGGATCATTG    240
   ATGGTGTTGG TAACGGTGGA CTCCTTGCTC CTGAGGCTCC TCTCGATTTC GGTAACGCTG    300
   CAACTGGTTG CCGTTTGACT ATGGGTCTTG TTGGTGTTTA CGATTTCGAT AGCACTTTCA    360
   TTGGTGACGC TTCTCTCACT AAGCGTCCAA TGGGTCGTGT GTTGAACCCA CTTCGCGAAA    420
   TGGGTGTGCA GGTGAAGTCT GAAGACGGTG ATCGTCTTCC AGTTACCTTG CGTGGACCAA    480
   AGACTCCAAC GCCAATCACC TACAGGGTAC CTATGGCTTC CGCTCAAGTG AAGTCCGCTG    540
   TTCTGCTTGC TGGTCTCAAC ACCCCAGGTA TCACCACTGT TATCGAGCCA ATCATGACTC    600
   GTGACCACAC TGAAAAGATG CTTCAAGGTT TTGGTGCTAA CCTTACCGTT GAGACTGATG    660
   CTGACGGTGT GCGTACCATC CGTCTTGAAG GTCGTGGTAA GCTCACCGGT CAAGTGATTG    720
   ATGTTCCAGG TGATCCATCC TCTACTGCTT TCCCATTGGT TGCTGCCTTG CTTGTTCCAG    780
   GTTCCGACGT CACCATCCTT AACGTTTTGA TGAACCCAAC CCGTACTGGT CTCATCTTGA    840
   CTCTGCAGGA AATGGGTGCC GACATCGAAG TGATCAACCC ACGTCTTGCT GGTGGAGAAG    900
   ACGTGGCTGA CTTGCGTGTT CGTTCTTCTA CTTTGAAGGG TGTTACTGTT CCAGAAGACC    960
   GTGCTCCTTC TATGATCGAC GAGTATCCAA TTCTCGCTGT TGCAGCTGCA TTCGCTGAAG   1020
   GTGCTACCGT TATGAACGGT TTGGAAGAAC TCCGTGTTAA GGAAAGCGAC CGTCTTTCTG   1080
   CTGTCGCAAA CGGTCTCAAG CTCAACGGTG TTGATTGCGA TGAAGGTGAG ACTTCTCTCG   1140
   TCGTGCGTGG TCGTCCTGAC GGTAAGGGTC TCGGTAACGC TTCTGGAGCA GCTGTCGCTA   1200
   CCCACCTCGA TCACCGTATC GCTATGAGCT TCCTCGTTAT GGGTCTCGTT TCTGAAAACC   1260
   CTGTTACTGT TGATGATGCT ACTATGATCG CTACTAGCTT CCCAGAGTTC ATGGATTTGA   1320
   TGGCTGGTCT TGGAGCTAAG ATCGAACTCT CCGACACTAA GGCTGCTTGA TGAGCTC      1377
(2) INFORMATION FOR SEQ ID NO:10:
   (i) SEQUENCE CHARACTERISTICS:
      (A) LENGTH: 318 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 87..317
   (xi) SEQUENCE DESCRIPTION: SEQ ID NO:10:
   AGATCTATCG ATAAGCTTGA TGTAATTGGA GGAAGATCAA AATTTTCAAT CCCCATTCTT       60
   CGATTGCTTC AATTGAAGTT TCTCCG ATG GCG CAA GTT AGC AGA ATC TGC AAT       113
                                Met Ala Gln Val Ser Arg Ile Cys Asn
                                 1               5
   GGT GTG CAG AAC CCA TCT CTT ATC TCC AAT CTC TCG AAA TCC AGT CAA        161
   Gly Val Gln Asn Pro Ser Leu Ile Ser Asn Leu Ser Lys Ser Ser Gln
    10                 15                  20                  25
   CGC AAA TCT CCC TTA TCG GTT TCT CTG AAG ACG CAG CAG CAT CCA CGA        209
   Arg Lys Ser Pro Leu Ser Val Ser Leu Lys Thr Gln Gln His Pro Arg
                   30                  35                  40
   GCT TAT CCG ATT TCG TCG TCG TGG GGA TTG AAG AAG AGT GGG ATG ACG        257
   Ala Tyr Pro Ile Ser Ser Ser Trp Gly Leu Lys Lys Ser Gly Met Thr
               45                  50                  55
   TTA ATT GGC TCT GAG CTT CGT CCT CTT AAG GTC ATG TCT TCT GTT TCC        305
   Leu Ile Gly Ser Glu Leu Arg Pro Leu Lys Val Met Ser Ser Val Ser
           60                  65                  70
   ACG GCG TGC ATG C                                                      318
   Thr Ala Cys Met
       75
(2) INFORMATION FOR SEQ ID NO:11:
   (i) SEQUENCE CHARACTERISTICS:
      (A) LENGTH: 77 amino acids
      (B) TYPE: amino acid
      (D) TOPOLOGY: linear
   (ii) MOLECULE TYPE: protein
   (xi) SEQUENCE DESCRIPTION: SEQ ID NO:11:
   Met Ala Gln Val Ser Arg Ile Cys Asn Gly Val Gln Asn Pro Ser Leu
    1               5                  10                  15
   Ile Ser Asn Leu Ser Lys Ser Ser Gln Arg Lys Ser Pro Leu Ser Val
               20                  25                  30
   Ser Leu Lys Thr Gln Gln His Pro Arg Ala Tyr Pro Ile Ser Ser Ser
           35                  40                  45
   Trp Gly Leu Lys Lys Ser Gly Met Thr Leu Ile Gly Ser Glu Leu Arg
       50                  55                  60
   Pro Leu Lys Val Met Ser Ser Val Ser Thr Ala Cys Met
   65                  70                  75
(2) INFORMATION FOR SEQ ID NO:12:
   (i) SEQUENCE CHARACTERISTICS:
      (A) LENGTH: 402 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 87..401
   (xi) SEQUENCE DESCRIPTION: SEQ ID NO:12:
   AGATCTATCG ATAAGCTTGA TGTAATTGGA GGAAGATCAA AATTTTCAAT CCCCATTCTT      60
   CGATTGCTTC AATTGAAGTT TCTCCG ATG GCG CAA GTT AGC AGA ATC TGC AAT     113
                                Met Ala Gln Val Ser Arg Ile Cys Asn
                                 1               5
   GGT GTG CAG AAC CCA TCT CTT ATC TCC AAT CTC TCG AAA TCC AGT CAA      161
   Gly Val Gln Asn Pro Ser Leu Ile Ser Asn Leu Ser Lys Ser Ser Gln
    10                  15                  20                  25
   CGC AAA TCT CCC TTA TCG GTT TCT CTG AAG ACG CAG CAG CAT CCA CGA      209
   Arg Lys Ser Pro Leu Ser Val Ser Leu Lys Thr Gln Gln His Pro Arg
                    30                  35                  40
   GCT TAT CCG ATT TCG TCG TCG TGG GGA TTG AAG AAG AGT GGG ATG ACG      257
   Ala Tyr Pro Ile Ser Ser Ser Trp Gly Leu Lys Lys Ser Gly Met Thr
                45                  50                  55
   TTA ATT GGC TCT GAG CTT CGT CCT CTT AAG GTC ATG TCT TCT GTT TCC      305
   Leu Ile Gly Ser Glu Leu Arg Pro Leu Lys Val Met Ser Ser Val Ser
            60                  65                  70
   ACG GCG GAG AAA GCG TCG GAG ATT GTA CTT CAA CCC ATT AGA GAA ATC      353
   Thr Ala Glu Lys Ala Ser Glu Ile Val Leu Gln Pro Ile Arg Glu Ile
        75                  80                  85
   TCC GGT CTT ATT AAG TTG CCT GGC TCC AAG TCT CTA TCA AAT AGA ATT      401
   Ser Gly Leu Ile Lys Leu Pro Gly Ser Lys Ser Leu Ser Asn Arg Ile
    90                  95                 100                 105
   C                                                                    402
(2) INFORMATION FOR SEQ ID NO:13:
   (i) SEQUENCE CHARACTERISTICS:
      (A) LENGTH: 105 amino acids
      (B) TYPE: amino acid
      (D) TOPOLOGY: linear
   (ii) MOLECULE TYPE: protein
   (xi) SEQUENCE DESCRIPTION: SEQ ID NO:13:
   Met Ala Gln Val Ser Arg Ile Cys Asn Gly Val Gln Asn Pro Ser Leu
     1               5                  10                  15
   Ile Ser Asn Leu Ser Lys Ser Ser Gln Arg Lys Ser Pro Leu Ser Val
                20                  25                  30
   Ser Leu Lys Thr Gln Gln His Pro Arg Ala Tyr Pro Ile Ser Ser Ser
            35                  40                  45
   Trp Gly Leu Lys Lys Ser Gly Met Thr Leu Ile Gly Ser Glu Leu Arg
        50                  55                  60
   Pro Leu Lys Val Met Ser Ser Val Ser Thr Ala Glu Lys Ala Ser Glu
    65                  70                  75                  80
   Ile Val Leu Gln Pro Ile Arg Glu Ile Ser Gly Leu Ile Lys Leu Pro
                    85                  90                  95
   Gly Ser Lys Ser Leu Ser Asn Arg Ile
               100                 105
(2) INFORMATION FOR SEQ ID NO:14:
   (i) SEQUENCE CHARACTERISTICS:
      (A) LENGTH: 233 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 14..232
   (xi) SEQUENCE DESCRIPTION: SEQ ID NO:14:
   AGATCTTTCA AGA ATG GCA CAA ATT AAC AAC ATG GCT CAA GGG ATA CAA        49
                  Met Ala Gln Ile Asn Asn Met Ala Gln Gly Ile Gln
                    1               5                  10
   ACC CTT AAT CCC AAT TCC AAT TTC CAT AAA CCC CAA GTT CCT AAA TCT       97
   Thr Leu Asn Pro Asn Ser Asn Phe His Lys Pro Gln Val Pro Lys Ser
           15                  20                  25
   TCA AGT TTT CTT GTT TTT GGA TCT AAA AAA CTG AAA AAT TCA GCA AAT      145
   Ser Ser Phe Leu Val Phe Gly Ser Lys Lys Leu Lys Asn Ser Ala Asn
       30                  35                  40
   TCT ATG TTG GTT TTG AAA AAA GAT TCA ATT TTT ATG CAA AAG TTT TGT      193
   Ser Met Leu Val Leu Lys Lys Asp Ser Ile Phe Met Gln Lys Phe Cys
    45                 50                  55                  60
   TCC TTT AGG ATT TCA GCA TCA GTG GCT ACA GCC TGC ATG C                233
   Ser Phe Arg Ile Ser Ala Ser Val Ala Thr Ala Cys Met
                   65                  70
(2) INFORMATION FOR SEQ ID NO:15:
   (i) SEQUENCE CHARACTERISTICS:
      (A) LENGTH: 73 amino acids
      (B) TYPE: amino acid
      (D) TOPOLOGY: linear
   (ii) MOLECULE TYPE: protein
   (xi) SEQUENCE DESCRIPTION: SEQ ID NO:15:
   Met Ala Gln Ile Asn Asn Met Ala Gln Gly Ile Gln Thr Leu Asn Pro
     1               5                  10                  15
   Asn Ser Asn Phe His Lys Pro Gln Val Pro Lys Ser Ser Ser Phe Leu
                20                  25                  30
   Val Phe Gly Ser Lys Lys Leu Lys Asn Ser Ala Asn Ser Met Leu Val
            35                  40                  45
   Leu Lys Lys Asp Ser Ile Phe Met Gln Lys Phe Cys Ser Phe Arg Ile
        50                  55                  60
   Ser Ala Ser Val Ala Thr Ala Cys Met
    65                  70
(2) INFORMATION FOR SEQ ID NO:16:
   (i) SEQUENCE CHARACTERISTICS:
      (A) LENGTH: 352 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 49..351
   (xi) SEQUENCE DESCRIPTION: SEQ ID NO:16:
   AGATCTGCTA GAAATAATTT TGTTTAACTT TAAGAAGGAG ATATATCC ATG GCA CAA      57
                                                        Met Ala Gln
                                                          1
   ATT AAC AAC ATG GCT CAA GGG ATA CAA ACC CTT AAT CCC AAT TCC AAT     105
   Ile Asn Asn Met Ala Gln Gly Ile Gln Thr Leu Asn Pro Asn Ser Asn
        5                  10                  15
   TTC CAT AAA CCC CAA GTT CCT AAA TCT TCA AGT TTT CTT GTT TTT GGA     153
   Phe His Lys Pro Gln Val Pro Lys Ser Ser Ser Phe Leu Val Phe Gly
    20                  25                  30                  35
   TCT AAA AAA CTG AAA AAT TCA GCA AAT TCT ATG TTG GTT TTG AAA AAA     201
   Ser Lys Lys Leu Lys Asn Ser Ala Asn Ser Met Leu Val Leu Lys Lys
                    40                  45                  50
   GAT TCA ATT TTT ATG CAA AAG TTT TGT TCC TTT AGG ATT TCA GCA TCA     249
   Asp Ser Ile Phe Met Gln Lys Phe Cys Ser Phe Arg Ile Ser Ala Ser
                55                  60                  65
   GTG GCT ACA GCA CAG AAG CCT TCT GAG ATA GTG TTG CAA CCC ATT AAA     297
   Val Ala Thr Ala Gln Lys Pro Ser Glu Ile Val Leu Gln Pro Ile Lys
            70                  75                  80
   GAG ATT TCA GGC ACT GTT AAA TTG CCT GGC TCT AAA TCA TTA TCT AAT     345
   Glu Ile Ser Gly Thr Val Lys Leu Pro Gly Ser Lys Ser Leu Ser Asn
        85                  90                  95
   AGA ATT C                                                           352
   Arg Ile
   100
(2) INFORMATION FOR SEQ ID NO:17:
   (i) SEQUENCE CHARACTERISTICS:
      (A) LENGTH: 101 amino acids
      (B) TYPE: amino acid
      (D) TOPOLOGY: linear
   (ii) MOLECULE TYPE: protein
   (xi) SEQUENCE DESCRIPTION: SEQ ID NO:17:
   Met Ala Gln Ile Asn Asn Met Ala Gln Gly Ile Gln Thr Leu Asn Pro
     1               5                  10                  15
   Asn Ser Asn Phe His Lys Pro Gln Val Pro Lys Ser Ser Ser Phe Leu
                20                  25                  30
   Val Phe Gly Ser Lys Lys Leu Lys Asn Ser Ala Asn Ser Met Leu Val
            35                  40                  45
   Leu Lys Lys Asp Ser Ile Phe Met Gln Lys Phe Cys Ser Phe Arg Ile
        50                  55                  60
   Ser Ala Ser Val Ala Thr Ala Gln Lys Pro Ser Glu Ile Val Leu Gln
    65                  70                  75                  80
   Pro Ile Lys Glu Ile Ser Gly Thr Val Lys Leu Pro Gly Ser Lys Ser
                    85                  90                  95
   Leu Ser Asn Arg Ile
               100
(2) INFORMATION FOR SEQ ID NO:18:
   (i) SEQUENCE CHARACTERISTICS:
      (A) LENGTH: 28 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:18:
   Xaa His Gly Ala Ser Ser Arg Pro Ala Thr Ala Arg Lys Ser Ser Gly
   1               5                   10                  15
   Leu Xaa Gly Thr Val Arg Ile Pro Gly Asp Lys Met
               20                  25
(2) INFORMATION FOR SEQ ID NO:19:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:19:
   Ala Pro Ser Met Ile Asp Glu Tyr Pro Ile Leu Ala Val
   1 5 10
(2) INFORMATION FOR SEQ ID NO:20:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:20:
   Ile Thr Gly Leu Leu Glu Gly Glu Asp Val Ile Asn Thr Gly Lys
   1 5 10 15
(2) INFORMATION FOR SEQ ID NO:21:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:21:
      ATGATHGAYG ARTAYCC 17
(2) INFORMATION FOR SEQ ID NO:22:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:22:
      **GARGAYGTNA THAACAC 17**
(2) INFORMATION FOR SEQ ID NO:23:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:23:
      **GARGAYGTNA THAATAC 17**
(2) INFORMATION FOR SEQ ID NO:24:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:24:
      **CGTGGATAGA TCTAGGAAGA CAACCATGGC TCACGGTC 38**
(2) INFORMATION FOR SEQ ID NO:25:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:25:
      **GGATAGATTA AGGAAGACGC GCATGCTTCA CGGTGCAAGC AGCC 44**
(2) INFORMATION FOR SEQ ID NO:26:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO-.26-.
      **GGCTGCCTGA TGAGCTCCAC AATCGCCATC GATGG 35**
(2) INFORMATION FOR SEQ ID NO:27:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:27:
      **CGTCGCTCGT CGTGCGTGGC CGCCCTGACG GC 32**
(2) INFORMATION FOR SEQ ID No:28:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:28:
      **CGCGCAAGCC CATGCAGGCT ATGGGCGCC 29**
(2) INFORMATION FOR SEQ ID NO:29:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:29:
      **CGGGCTGCCG CCTGACTATG GGCCTCGTCG G 31**
(2) INFORMATION FOR SEQ ID NO:30:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID No:30:
   Xaa His Ser Ala Ser Pro Lys Pro Ala Thr Ala Arg Arg Ser Glu
   1 5 10 15
(2) INFORMATION FOR SEGl ID NO:31:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:31:
      **GCGGTBGCSG GYTTSCG** **17**
(2) INFORMATION FOR SEQ ID NO:32:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:32:
   Pro Gly Asp Lys Ser Ile Ser His Arg Ser Phe Met Phe Gly Gly Leu
   1               5                   10                  15
(2) INFORMATION FOR SEQ ID NO:33:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:33:
   Leu Asp Phe Gly Asn Ala Ala Thr Gly Cys Arg Leu Thr
   1               5                   10
(2) INFORMATION FOR SEQ ID NO:34:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:34:
      **CGGCAATGCC GCCACCGGCG CGCGCC 26**
(2) INFORMATION FOR SEQ ID NO:35:
   (i) SEQUENCE CHARACTERISTICS:
      (A) LENGTH: 49 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:35:
      **GGACGGCTGC TTGCACCGTG AAGCATGCTT AAGCTTGGCG TAATCATGG 49**
(2) INFORMATION FOR SEQ ID NO:36:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:36:
      **GCAAGACGCC CAGAATTCAC GGTGCAAGCA GCCGG 35**

## Description

This is a continuation-in-part of a copending U.S. patent application having serial number 07/576,537, filed August 31, 1990 and entitled "Glyphosate Tolerant 5-Enolpyruvylshikimate.3.Phosphate Synthases."

This invention relates in general to plant molecular biology and, more particularly, to a new class of glyphosate tolerant 5-enolpyruvylshikimate-3-phosphate synthases.

Recent advances in genetic engineering have provided the requisite tools to transform plants to contain foreign genes. It is now possible to produce plants which have unique characteristics of agronomic importance. Certainly, one such advantageous trait is more cost effective, environmentally compatible weed control via herbicide tolerance. Herbicide-tolerant plants may reduce the need for tillage to control weeds thereby effectively reducing soil erosion.

One herbicide which is the subject of much investigation in this regard is N-phosphonomethylglycine commonly referred to as glyphosate. Glyphosate inhibits the shikimic acid pathway which leads to the biosynthesis of aromatic compounds including amino acids, plant hormones and vitamins. Specifically, glyphosate curbs the conversion of phosphoenolpyruvic acid (PEP) and 3-phosphoshikimic acid to 5-enolpyruvyl-3-phosphoshikimic acid by inhibiting the enzyme 5-enolpyruvylshikimate-3-phosphate synthase (hereinafter referred to as EPSP synthase or EPSPS).

It has been shown that glyphosate tolerant plants can be produced by inserting into the genome of the plant the capacity to produce a higher level of EPSP synthase in the chloroplast of the cell (Shah et al., 1986) which enzyme is preferably glyphosate tolerant (Kishore et al. 1988). Variants of the wild-type EPSPS enzyme have been isolated which are glyphosate tolerant as a result of alterations in the EPSPS amino acid coding sequence (Kishore and Shah, 1988; Schulz et al., 1984; Sost et al., 1984; Kishore et al., 1986). These variants typically have a higher Kᵢ for glyphosate than the wild-type EPSPS enzyme which confers the glyphosate tolerant phenotype, but these variants are also characterized by a high Kₘ for PEP which makes the enzyme kinetically less efficient (Kishore and Shah, 1988: Sost et al., 1984; Schulz et al., 1984: Kishore et al., 1986); Sost and Amrhein, 1990). For example, the apparent Kₘ for PEP and the apparent Kᵢ for glyphosate for the native EPSPS from *E. coli* are 10 µM and 0.5 µM while for a glyphosate tolerant isolate having a single amino acid substitution of an alanine for the glycine at position 96 these values are 220 µM and 4.0 mM, respectively. A number of glyphosate tolerant plant variant EPSPS genes have been constructed by musagenesis. Again, the glyphosate tolerant EPSPS was impaired due to an increase in the Km for PEP and a slight reduction of the Vₘₐₓ of the native plant enzyme (Kishore and Shah, 1988) thereby lowering the catalytic efficiency (Vₘₐₓ/Kₘ) of the enzyme. Since the kinetic constants of the variant enzymes are impaired with respect to PEP, it has been proposed that high levels of overproduction of the variant enzyme, 40-80 fold, would be required to maintain normal catalytic activity in plants in the presence of glyphosate (Kishore et al., 1988).

While such variant EPSP synthases have proved useful in obtaining transgenic plants tolerant to glyphosate, it would be increasingly beneficial to obtain an EPSP synthase that is highly glyphosate tolerant while still kinetically' efficient such that the amount of the glyphosate tolerant EPSPS needed to be produced to maintain normal catalytic activity in the plant is reduced or that improved tolerance be obtained with the same expression level.

Previous studies have shown that EPSPS enzymes from different sources vary widely with respect to their degree of sensitivity to inhibition by glyphosate. A study of plant and bacterial EPSPS enzyme activity as a function of glyphosate concentration showed that there was a very wide range in the degree of sensitivity to glyphosate. The degree of sensitivity showed no correlation with any genus or species tested (Schulz et al., 1985). Insensitivity to glyphosate inhibition of the activity of the EPSPS from the *Pseudomonas* sp. PG2982 has also been reported but with no details of the studies (Fitzgibbon, 1988). In general, while such natural tolerance has been reported, there is no report suggesting the kinetic superiority of the naturally occurring bacterial glyphosate tolerant EPSPS enzymes over those of mutated EPSPS enzymes nor have any of the genes been characterized. Similarly, there are no reports on the expression of naturally glyphosate tolerant EPSPS enzymes in plants to confer glyphosate tolerance.

### SUMMARY OF THE INVENTION

A DNA molecule comprising DNA encoding a kinetically efficient, glyphosate tolerant EPSP synthase is presented. The EPSP synthases of the present invention reduce the amount of overproduction of the EPSPS enzyme in a transgenic plant necessary for the enzyme to maintain catalytic activity while still conferring glyphosate tolerance. This and other EPSP synthases described herein represent a new class of EPSPS enzymes, referred to hereinafter as Class II EPSPS enzymes. Class II EPSPS enzymes share little homology to known bacterial or plant EPSPS enzymes and exhibit tolerance to glyphosate while maintaining suitable Kₘ (PEP) ranges. Suitable ranges of Kₘ (PEP) for EPSPS for enzymes of the present invention are between 1-150 µM, with a more preferred range of between 1-35 µM, and a most preferred range between 2-25 µM. These kinetic constants are determined under the assay conditions specified hereinafter. The Vₘₐₓ of the enzyme should preferably be at least 15% of the uninhibited plant enzyme and more preferably greater than 25%. An EPSPS of the present invention preferably has a Kᵢ for glyphosate range of between 25-10000 µM. The Kᵢ/Kₘ ratio should be between 3-500, and more preferably between 6-250. The Vₘₐₓ should preferably be in the range of 2-100 units/mg (µmoles/minute.mg at 25°C) and the Kₘ for shikimate-3-phosphate should preferably be in the range of 0.1 to 50 µM.

Genes coding for Class II EPSPS enzymes have been isolated from three (3) different bacteria: *Agrobacterium tumefaciens* sp. strain CP4, *Achromobacter* sp. strain LBAA, and *Pseudomonas* sp. strain PG2982. The LBAA and PG2982 Class II EPSPS genes have been determined to be identical and the proteins encoded by these two genes are very similar to the CP4 protein and share approximately 84% amino acid identity with it. Class II EPSPS enzymes can be readily distinguished from Class I EPSPS's by their inability to react with polyclonal antibodies prepared from Class I EPSPS enzymes under conditions where other Class I EPSPS enzymes would readily react with the Class I antibodies.

Other Class II EPSPS enzymes can be readily isolated and identified by utilizing a nucleic acid probe from one of the Class II EPSPS genes disclosed herein using standard hybridization techniques. Such a probe from the CP4 strain has been prepared and utilized to isolate the Class II EPSPS genes from strains LBAA and PG2982. These genes may also be adapted for enhanced expression in plants by known methodology. Such a probe has also been used to identify homologous genes in bacteria isolated *de novo* from soil.

The Class II EPSPS enzymes are preferably fused to a chloroplast transit peptide (CTP) to target the protein to the chloroplasts of the plant into which it may be introduced. Chimeric genes encoding this CTP-Class II EPSPS fusion protein may be prepared with an appropriate promoter and 3' polyadenylation site for introduction into a desired plant by standard methods.

Therefore, in one aspect, the present invention provides a new class of EPSP synthases that exhibit a low Kₘ for phosphoenolpyruvate (PEP), a high Vₘₐₓ/Kₘ ratio, and a high Kᵢ for glyphosate such that when introduced into a plant, the plant is made glyphosate tolerant such that the catalytic activity of the enzyme and plant metabolism are maintained in a substantially normal state. For purposes of this discussion, a highly efficient EPSPS refers to its efficiency in the presence of glyphosate.

In another aspect of the present invention, a double-stranded DNA molecule comprising DNA encoding a Class II EPSPS enzyme is disclosed. A Class II EPSPS enzyme DNA sequence is disclosed from three sources: *Agrobacterium* sp. strain designated CP4, *Achromobacter* sp. strain LBAA and *Pseudomonas* sp. strain PG2982.

In a further aspect of the present invention, a nucleic acid probe from an EPSPS Class II gene is presented that is suitable for use in screening for Class II EPSPS genes in other sources by assaying for the ability of a DNA sequence from the other source to hybridize to the probe.

In yet another aspect of the present invention, transgenic plants and transformed plant cells are disclosed that are made glyphosate tolerant by the introduction of a Class II EPSPS gene into the plant's genome.

In a still further aspect of the invention, a recombinant, double-stranded DNA molecule comprising in sequence:
a) a promoter which functions in plant cells to cause the production of an RNA sequence;
b) a structural DNA sequence that causes the production of an RNA sequence which encodes a Class II EPSPS enzyme: and
c) a 3' nontranslated region which functions in plant cells to cause the addition of a stretch of polyadenyl nucleotides to the 3' end of the RNA sequence
where the promoter is heterologous with respect to the structural DNA sequence and adapted to cause sufficient expression of the fusion polypeptide to enhance the glyphosate tolerance of a plant cell transformed with said DNA molecule.

In still another aspect of the present invention, a method for selectively controlling weeds in a crop field is presented by planting crop seeds or crop plants transformed with a Class II EPSPS gene to confer glyphosate tolerance to the plants which allows for glyphosate containing herbicides to be applied to the crop to selectively kill the glyphosate sensitive weeds, but not the crops.

Other and further objects, advantages and aspects of the invention will become apparent from the accompanying drawing figures and the description of the invention.

### BRIEF DESCRIPTION OF THE DRAWINGS

Figure 1 shows the DNA sequence (SEQ ID NO:1) for the full-length promoter of figwort mosaic virus (FMV35S).
Figure 2 shows the cosmid cloning vector pMON17020.
Figure 3 shows the structural DNA sequence (SEQ ID NO:2) for the Class II EPSPS gene from bacterial isolate *Agrobacterium* sp. strain CP4 and the deduced amino acid sequence (SEQ ID NO:3).
Figure 4 shows the structural DNA sequence (SEQ ID NO:4) for the Class II EPSPS gene from the bacterial isolate *Achromobacter sp.* strain LBAA and the deduced amino acid sequence (SEQ ID NO:5).
Figure 5 shows the structural DNA sequence (SEQ ID NO:6) for the Class II EPSPS gene from the bacterial isolate *Pseudomonas sp.* strain PG2982 and the deduced amino acid sequence (SEQ ID NO:7).
Figure 6 shows the Bestfit comparison of the *E. coli* EPSPS amino acid sequence (SEQ ID NO:8) with that for the CP4 EPSPS (SEQ ID NO:3).
Figure 7 shows the Bestfit comparison of the CP4 EPSPS amino acid sequence (SEQ ID NO:3) with that for the LBAA EPSPS (SEQ ID NO:5).
Figure 8 shows the structural DNA sequence (SEQ ID NO:9) for the synthetic CP4 Class II EPSPS gene.
Figure 9 shows the DNA sequence (SEQ ID NO:10) of the chloroplast transit peptide (CTP) and encoded amino acid sequence (SEQ ID NO:11) derived from the *Arabidopsis thaliana* EPSPS CTP and containing a *Sphl* restriction site at the chloroplast processing site, hereinafter referred to as CTP2.
Figure 10 shows the DNA sequence (SEQ ID N0:12) of the chloroplast transit peptide and encoded amino acid sequence (SEQ ID NO: 13) derived from the *Arabidopsis thaliana* EPSPS gene and containing an EcoRl restriction site within the mature region of the EPSPS, hereinafter referred to as CTP3.
Figure 11 shows the DNA sequence (SEQ ID NO:14) of the chloroplast transit peptide and encoded amino acid sequence (SEQ ID NO:15) derived from the *Petunia hybrida* EPSPS CTP and containing a Sphl restriction site at the chloroplast processing site and in which the amino acids at the processing site are changed to -Cys-Met-, hereinafter referred to as CTP4.
Figure 12 shows the DNA sequence (SEQ ID NO:16) of the chloroplast transit peptide and encoded amino acid sequence (SEQ ID NO:17) derived from the *Petunia hybrida* EPSPS gene with the naturally occurring *Eco*RI site in the mature region of the EPSPS gene, hereinafter referred to as CTP5.
Figure 13 shows a plasmid map of CP4 plant transformation/ expression vector pMON17110.
Figure 14 shows a plasmid map of CP4 synthetic EPSPS gene plant transformation/expression vector pMON17131.
Figure 15 shows a plasmid map of CP4 EPSPS free DNA plant transformation expression vector pMON1 3640.
Figure 16 shows a plasmid map of CP4 plant transformation/direct selection vector pMON17227.
Figure 17 shows a plasmid map of CP4 plant transformation/expression vector pMON19653.

The expression of a plant gene which exists in double-stranded DNA form involves synthesis of messenger RNA (mRNA) from one strand of the DNA by RNA polymerase enzyme, and the subsequent processing of the mRNA primary transcript inside the nucleus. This processing involves a 3' non-translated region which adds polyadenylate nucleotides to the 3' end of the RNA.

Transcription of DNA into mRNA is regulated by a region of DNA usually referred to as the "promoter. " The promoter region contains a sequence of bases that signals RNA polymerase to associate with the DNA, and to initiate the transcription into mRNA using one of the DNA strands as a template to make a corresponding complementary strand of RNA.

A number of promoters which are active in plant cells have been described in the literature. These include the nopaline synthase (NOS) and octopine synthase (OCS) promoters (which are carried on tumor-inducing plasmids of *Agrobacterium tumefaciens*), the cauliflower mosaic virus (CaMV) 19S and 35S promoters, the light-inducible promoter from the small subunit of ribulose bis-phosphate carboxylase (ssRUBISCO, a very abundant plant polypeptide) and the full-length transcript promoter from the figwort mosaic virus (FMV35S). All of these promoters have been used to create various types of DNA constructs which have been expressed in plants; see, e.g., PCT publication WO 84/02913 (Rogers et al., Monsanto).

Promoters which are known or are found to cause transcription of DNA in plant cells can be used in the present invention. Such promoters may be obtained from a variety of sources such as plants and plant DNA viruses and include, but are not limited to, the CaMV35S and FMV35S promoters and promoters isolated from plant genes such as ssRUBISCO genes. As described below, it is preferred that the particular promoter selected should be capable of causing sufficient expression to result in the production of an effective amount of a Class II EPSPS to render the plant substantially tolerant to glyphosate herbicides. The amount of Class II EPSPS needed to induce the desired tolerance may vary with the plant species. It is preferred that the promoters utilized have relatively high expression in all meristematic tissues in addition to other tissues inasmuch as it is now known that glyphosate is translocated and accumulated in this type of plant tissue. Alternatively, a combination of chimeric genes can be used to cumulatively result in the necessary overall expression level of the selected Class II EPSPS enzyme to result in the glyphosate tolerant phenotype.

The mRNA produced by a DNA construct of the present invention also contains a 5'non-translated leader sequence. This sequence can be derived from the promoter selected to express the gene, and can be specifically modified so as to increase translation of the mRNA. The 5' non-translated regions can also be obtained from viral RNAs, from suitable eukaryotic genes, or from a synthetic gene sequence. The present invention is not limited to constructs, as presented in the following examples, wherein the non-translated region is derived from both the 5'non-translated sequence that accompanies the promoter sequence and part of the 5' non-translated region of the virus coat protein gene. Rather, the non-translated leader sequence can be derived from an unrelated promoter or coding sequence as discussed above.

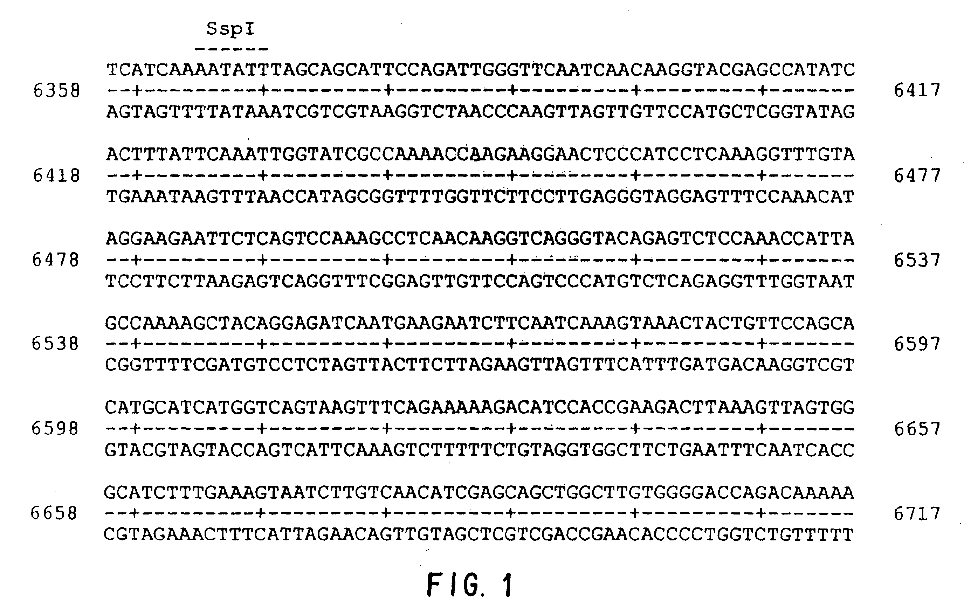

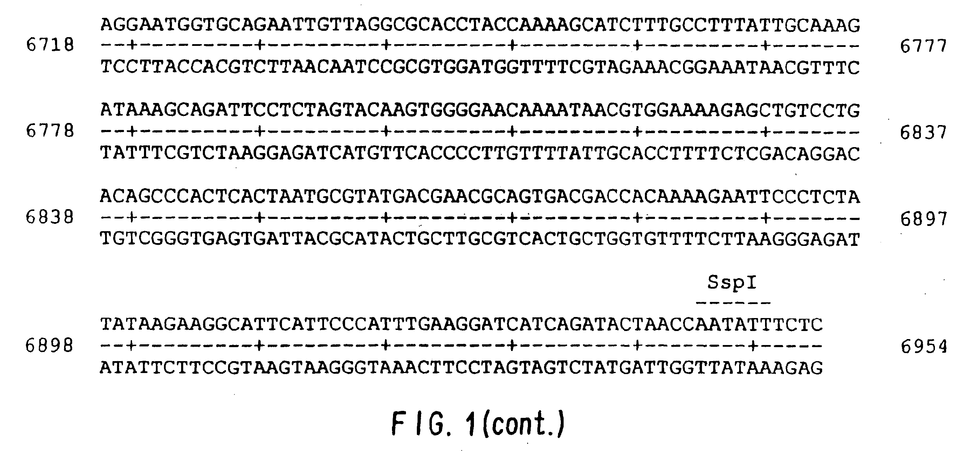

A preferred promoter for use in the present invention is the full-length transcript (SEO I D NO:1) promoter from the figwort mosaic virus (FMV35S) which functions as a strong and uniform promoter with particularly good expression in meristematic tissue for chimeric genes inserted into plants, particularly dicotyledons. The resulting transgenic plant in general expresses the protein encoded by the inserted gene at a higher and more uniform level throughout the tissues and cells of the transformed plant than the same gene driven by an enhanced CaMV35S promoter. Referring to Figure 1, the DNA sequence (SEQ ID NO:1) of the FMV35S promoter is located between nucleotides 6368 and 6930 of the FMV genome. A 5' non-translated leader sequence is preferably coupled with the promoter. The leader sequence can be from the FMV35S genome itself or can be from a source other than FMV35S.

The 3' non-translated region of the chimeric plant gene contains a polyadenylation signal which functions in plants to cause the addition of polyadenylate nucleotides to the 3' end of the viral RNA. Examples of suitable 3'regions are (1) the 3' transcribed, non-translated regions containing the polyadenylated signal of *Agrobacterium* tumor-inducing (Ti) plasmid genes, such as the nopaline synthase (NOS) gene, and (2) plant genes like the soybean storage protein genes and the small subunit of the ribulose-1,5-bisphosphate carboxylase (ssRUBISCO) gene. An example of a preferred 3'region is that from the ssRUBISCO gene from pea (E9), described in greater detail below.

The DNA constructs of the present invention also contain a structural coding sequence in double-stranded DNA form which encodes a glyphosate tolerant, highly efficient Class II EPSPS enzyme.

### Identification of glyphosate tolerant, highly efficient EPSPS enzymes

In an attempt to identify and isolate glyphosate tolerant, highly efficient EPSPS enzymes, kinetic analysis of the EPSPS enzymes from a number of bacteria exhibiting tolerance to glyphosate or that had been isolated from suitable sources was undertaken. It was discovered that in some cases the EPSPS enzymes showed no tolerance to inhibition by glyphosate and it was concluded that the tolerance phenotype of the bacterium was due to an impermeability to glyphosate or other factors. In a number of cases, however, microorganisms were identified whose EPSPS enzyme showed a greater degree of tolerance to inhibition by glyphosate and that displayed a low Kₘ for PEP when compared to that previously reported for other microbial and plant sources. The EPSPS enzymes from these microorganisms were then subjected to further study and analysis.

Table I displays the data obtained for the EPSPS enzymes identified and isolated as a result of the above described analysis. Table I includes data for three identified Class II EPSPS enzymes that were observed to have a high tolerance to inhibition to glyphosate and a low Kₘ for PEP as well as data for the native Petunia EPSPS and a glyphosate tolerant variant of the Petunia EPSPS referred to as GA101. The GA101 variant is so named because it exhibits the substitution of an alanine residue for a glycine residue at position 101 (with respect to Petunia) in the invariant region. When the change introduced into the Petunia EPSPS (GA101) was introduced into a number of other EPSPS enzymes, similar changes in kinetics were observed, an elevation of the Kᵢ for glyphosate and of the Kₘ for PEP.

**Table I**

| **Kinetic characterization of EPSPS enzymes** | | | |
|---|---|---|---|
| ENZYME SOURCE | Kₘ PEP (µM) | Kᵢ Glyphosate (µM) | Kᵢ/Kₘ |
| Petunia | 5 | 0.4 | 0.08 |
| Petunia GA101 | 200 | 2000 | 10 |
| PG2982 | 2.1-3.1¹ | 25-82 | ~8-40 |
| LBAA | ~7.3-8² | 60(est) | ~7.9 |
| CP4 | 12³ | 2720 | 227 |

| | | | |
|---|---|---|---|
| ¹ Range of PEP tested = 1-40 µM | | | |
| ² Range of PEP tested = 5-80 µM | | | |
| ³ Range of PEP tested = 1.5-40 µM | | | |

The *Agrobacterium* sp. strain CP4 was initially identified by its ability to grow on glyphosate as a carbon source (10 mM) in the presence of 1 mM phosphate. The strain CP4 was identified from a collection obtained from a fixed-bed immobilized cell column that employed Mannville R-635 diatomaceous earth beads. The column had been run for three months on a waste-water feed from a glyphosate production plant. The column contained 50 mg/ml glyphosate and NH₃ as NH₄Cl. Total organic carbon was 300 mg/ml and BOD's (Biological Oxygen Demand - a measure of "soft" carbon availability) were less than 30 mg/ml. This treatment column has been described (Heitkamp et al., 1990). Dworkin-Foster minimal salts medium containing glyphosate at 10 mM and with phosphate at 1 mM was used to select for microbes from a wash of this column that were capable of growing on glyphosate as sole carbon source. Dworkin-Foster minimal medium was made up by combining in 1 liter (with autoclaved H₂O), 1 ml each of A, B and C and 10 ml of D (as per below) and thiamine HCl (5 mg).

| | | |
|---|---|---|
| A. | D-F Salts (1000X stock: per 100 ml: autoclaved): | |
| | H₃BO₃ | 1 mg |
| | MnSO₄.7H₂O | 1 mg |
| | ZnSO₄.7H₂O | 12.5 mg |
| | CuSO₄.5H₂O | 8 mg |
| | NaMoO₃.3H₂O | 1.7 mg |
| | | |
| B. | FeSO₄.7H₂O (1000X stock; per 100 ml: autoclaved) | 0.1 g |
| | | |
| C. | MgSO₄.7H₂O (1000X stock; per 100 ml; autoclaved) | 20 g |
| | | |
| D. | (NH₄)₂SO₄ (100X stock; per 100 ml; autoclaved) | 20 g |

Yeast Extract (YE; Difco) was added to a final concentration of 0.01 or 0.001%. The strain CP4 was also grown on media composed of D-F salts, amended as described above, containing glucose, gluconate and citrate (each at 0.1 %) as carbon sources and with inorganic phosphate (0.2 - 1.0 mM) as the phosphorous source.

Other Class II EPSPS containing microorganisms were identified as *Achromobacter* sp. strain LBAA, which was from a collection of bacteria previously described (Hallas et al., 1988), and *Pseudomonas* sp. strain PG2982 which has been described in the literature (Moore et al. 1983; Fitzgibbon 1988). It had been reported previously, from measurements in crude lysates, that the EPSPS enzyme from strain PG2982 was less sensitive to inhibition to glyphosate than that of *E. coli,* but there has been no report of the details of this lack of sensitivity and there has been no report on the Kₘ for PEP for this enzyme or of the DNA sequence for the gene for this enzyme (Fitzgibbon, 1988: Fitzgibbon and Braymer, 1990)..

### Relationship of the Class 11 EPSPS to those previously studied

All EPSPS proteins studied to date have shown a remarkable degree of homology. For example, bacterial and plant EPSPS's are about 54% identical and with similarity as high as 80%. Within bacterial EPSPS's and plant EPSPS's themselves the degree of identity and similarity is much greater (see Table II).

**Table II**

| **Comparison between exemplary Class I EPSPS protein sequences**^{**1**} | | |
|---|---|---|
| | similarity | identity |
| *E. coli* vs. *S. typhimurium* | 93.0 | 88.3 |
| *P hybrida* vs. *E. coli* | 71.9 | 54.5 |
| *P. hybrida* vs. Tomato | 92.8 | 88.2 |

| | | |
|---|---|---|
| ¹ The EPSPS sequences compared here were obtained from the following references: *E. coli.* Rogers et al.. 1983: S. *typhimurium.* Stalker et al.. 1985: *Petunia hybrida,* Shah et al.. 1986: and Tomato. Gasser et al.. 1988. | | |

When crude extracts of CP4 and LBAA bacteria (50 µg protein) were probed using rabbit anti-EPSPS antibody (Padgette et al. 1987) to the Petunia EPSPS protein in a Western analysis, no positive signal could be detected, even with extended exposure times (Protein A - ¹²⁵I development system) and under conditions where the control EPSPS (Petunia EPSPS, 20 ng: a Class I EPSPS) was readily detected. The presence of EPSPS activity in these extracts was confirmed by enzyme assay. This surprising result, indicating a lack of similarity between the EPSPS's from these bacterial isolates and those previously studied, coupled with the combination of a low Kₘ for PEP and a high Kᵢ for glyphosate, illustrates that these new EPSPS enzymes are different from known EPSPS enzymes (now referred to as Class I EPSPS).

### Glyphosate Tolerant Enzymes in Microbial Isolates

For clarity and brevity of disclosure, the following description of the isolation of genes encoding Class II EPSPS enzymes is directed to the isolation of such a gene from a bacterial isolate. Those skilled in the art will recognize that the same or similar strategy can be utilized to isolate such genes from other microbial isolates, plant or fungal sources.

### Cloning of the Agrobacterium sp. strain CP4 EPSPS Gene(s) in E. coli

Having established the existence of a suitable EPSPS in *Agrobacterium* sp. strain CP4, two parallel approaches were undertaken to clone the gene: cloning based on the expected phenotype for a glyphosate tolerant EPSPS; and purification of the enzyme to provide material to raise antibodies and to obtain amino acid sequences from the protein to facilitate the verification of clones. Cloning and genetic techniques, unless otherwise indicated, are generally those described in Maniatis et al., 1982 or Sambrook et al., 1987. The cloning strategy was as follows: introduction of a cosmid bank of strain *Agrobacterium* sp. strain CP4 into *E. coli* and selection for the EPSPS gene by selection for growth on inhibitory concentrations of glyphosate.

Chromosomal DNA was prepared from strain *Agrobacterium* sp. strain CP4 as follows: The cell pellet from a 200 ml L-Broth (Miller, 1972), late log phase culture of *Agrobacterium* sp. strain CP4 was resuspended in 10 ml of Solution I: 50 mM Glucose, 10 mM EDTA, 25 mM Tris -CL pH 8.0 (Birnboim and Doly, 1979). SDS was added to a final concentration of 1 % and the suspension was subjected to three freeze-thaw cycles, each consisting of immersion in dry ice for 15 minutes and in water at 70°C for 10 minutes. The lysate was then extracted four times with equal volumes of phenol:chloroform (1:1; phenol saturated with TE; TE = 10 mM Tris pH8.0: 1.0 mM EDTA) and the phases separated by centrifugation (15000g; 10 minutes). The ethanol-precipitable material was pelleted from the supernatant by brief centrifugation (8000g; 5 minutes) following addition of two volumes of ethanol. The pellet was resuspended in 5 ml TE and dialyzed for 16 hours at 4°C against 2 liters TE. This preparation yielded a 5 ml DNA solution of 552 µg/ml.

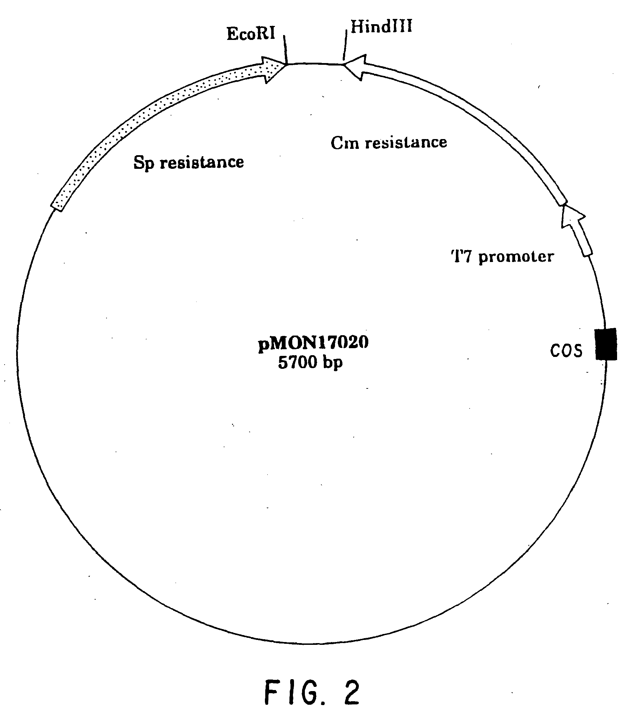

Partially-restricted DNA was prepared as follows. Three 100 µg aliquot samples of CP4 DNA were treated for 1 hour at 37°C with restriction endonuclease *Hin*dIII at rates of 4, 2 and 1 enzyme unit/µg DNA, respectively. The DNA samples were pooled, made 0.25 mM with EDTA and extracted with an equal volume of phenol:chloroform. Following the addition of sodium acetate and ethanol, the DNA was precipitated with two volumes of ethanol and pelleted by centrifugation (12000 g; 10 minutes). The dried DNA pellet was resuspended in 500 µl TE and layered on a 10-40% Sucrose gradient (in 5% increments of 5.5 ml each) in 0.5 M NaCl, 50 mM Tris pH8.0, 5 mM EDTA. Following centrifugation for 20 hours at 26,000 rpm in a SW28 rotor, the tubes were punctured and ~1.5 ml fractions collected. Samples (20 µl) of each second fraction were run on 0.7% agarose gel and the size of the DNA determined by comparison with linearized lambda DNA and Hindlll-digested lambda DNA standards. Fractions containing DNA of 25-35 kb fragments were pooled, desalted on AMICON10 columns (7000 rpm; 20°C; 45 minutes) and concentrated by precipitation. This procedure yielded 15 µg of CP4 DNA of the required size. A cosmid bank was constructed using the vector pMON17020. This vector, a map of which is presented in Figure 2, is based on the pBR327 replicon and contains the spectinomycin/streptomycin (Sp^{r}:*spc*) resistance gene from Tn7 (Fling et al., 1985), the chloramphenicol resistance gene (Cm^{r}: *cat*) from Tn9 (Alton et al., 1979), the gene 10 promoter region from phage T7 (Dunn et al., 1983), and the 1.6 kb *Bgl*II phage lambda cos fragment from pHC79 (Hohn and Collins, 1980). A number of cloning sites are located downstream of the *cat* gene. Since the predominant block to the expression of genes from other microbial sources in *E. coli* appears to be at the level of transcription, the use of the T7 promoter and supplying the T7 polymerase *in trans* from the pGP1-2 plasmid (Tabor and Richardson, 1985), enables the expression of large DNA segments of foreign DNA, even those containing RNA polymerase transcription termination sequences. The expression of the spc gene is impaired by transcription from the T7 promoter such that only Cm^{r} can be selected in strains containing pGP1-2. The use of antibiotic resistances such as Cm resistance which do not employ a membrane component is preferred due to the observation that high level expression of resistance genes that involve a membrane component, i.e. β-lactamase and Amp resistance, give rise to a glyphosate tolerant phenotype. Presumably, this is due to the exclusion of glyphosate from the cell by the membrane localized resistance protein. It is also preferred that the selectable marker be oriented in the same direction as the T7 promoter.

The vector was then cut with *Hin*dIII and treated with calf alkaline phosphatase (CAP) in preparation for cloning. Vector and target sequences were ligated by combining the following:

| | |
|---|---|
| Vector DNA (*Hin*dIII/CAP) | 3 µg |
| Size fractionated CP4 *Hin*dIII fragments | 1.5 µg |
| 10X ligation buffer | 2.2 µl |
| T4 DNA ligase (New England Biolabs) (400 U/µl) | 1.0 µl |

and adding H₂O 22.0 µl. This mixture was incubated for 1B hours at 16°C. 10X ligation buffer is 250 mM Tris-HCl, pH 8.0: 100 mM MgCl₂: 100 mM Dithiothreitol; 2 mM Spermidine. The ligated DNA (5 µl) was packaged into lambda phage particles (Stratagene: Gigapack Gold) using the manufacturer's procedure.

A sample (200 µl) of *E. coli* HB101 (Boyer and Rolland-Dussoix, 1973) containing the T7 polymerase expression plasmid pGP1-2 (Tabor and Richardson, 1985) and grown overnight in L-Broth (with maltose at 0.2% and kanamycin at 50 µg/ml) was infected with 50 µl of the packaged DNA. Transformants were selected at 30°C on M9 (Miller, 1972) agar containing kanamycin (50 µg/ml), chloramphenicol (25 µg/ml), L-proline (50 µg/ml), L-leucine (50 µg/ml) and B1 (5 µg/ml), and with glyphosate at 3.0 mM. Aliquot samples were also plated on the same media lacking glyphosate to titer the packaged cosmids. Cosmid transformants were isolated on this latter medium at a rate of ~5 x 10⁵ per µg CP4 *Hin*dIII DNA after 3 days at 30°C. Colonies arose on the glyphosate agar from day 3 until day 15 with a final rate of ~1 per 200 cosmids. DNA was prepared from 14 glyphosate tolerant clones and, following verification of this phenotype, was transformed into *E. coli* GB100/pGP1-2 (*E. coli* GB100 is an *aroA* derivative of MM294 [Talmadge and Gilbert, 1980]) and tested for complementation for growth in the absence of added aromatic amino acids and aminobenzoic acids. Other *aroA* strains such as SR481 (Bachman et al. 1980; Padgette et al., 1987), could be used and would be suitable for this experiment. The use of GB100 is merely exemplary and should not be viewed in a limiting sense. This *aroA* strain usually requires that growth media be supplemented with L-phenylalanine, L-tyrosine and L-tryptophan each at 100 µg/ml and with para-hydroxybehzoic acid, 2,3-dihydroxybenzoic acid and para-aminobenzoic acid each at 5 µg/ml for growth in minimal media. Of the fourteen cosmids tested only one showed complementation of the *aroA-* phenotype. Transformants of this cosmid, pMON1 7076, showed weak but uniform growth on the unsupplemented minimal media after 10 days.

The proteins encoded by the cosmids were determined *in vivo* using a T7 expression system (Tabor and Richardson, 1985). Cultures of *E. coli* containing pGP1-2 (Tabor and Richardson, 1985) and test and control cosmids were grown at 30°C in L-broth (2 ml) with chloramphenicol and kanamycin (25 and 50 µg/ml, respectively) to a Klett reading of ~ 50. An aliquot was removed and the cells collected by centrifugation, washed with M9 salts (Miller, 1972) and resuspended in 1 ml M9 medium containing glucose at 0.2%, thiamine at 20 µg/ml and containing the 18 amino acids at 0.01 % (minus cysteine and methionine). Following incubation at 30°C for 90 minutes, the cultures were transferred to a 42°C water bath and held there for 15 minutes. Rifampicin (Sigma) was added to 200 µg/ml and the cultures held at 42°C for 10 additional minutes and then transferred to 30°C for 20 minutes. Samples were pulsed with 10 µCi of ³⁵S-methionine for 5 minutes at 30°C. The cells were collected by centrifugation and suspended in 60-120 µl cracking buffer (60 mM Tris-HCl 6.8, 1% SDS, 1% 2-mercaptoethanol, 10% glycerol, 0.01 % bromophenol blue). Aliquot samples were electrophoresed on 12.5% SDS-PAGE and following soaking for 60 minutes in 10 volumes of Acetic Acid-Methanol-water (10:30:60), the gel was soaked in ENLIGHTNING^{™} (DUPONT) following manufacturer's directions, dried, and exposed at -70°C to X-Ray film. Proteins of about 45 kd in size, labeled with ³⁵S-methionine, were detected in number of the cosmids, including pMON17076.

### Purification of EPSPS from Agrobacterium sp. strain CP4

All protein purification procedures were carried out at 3-5°C. EPSPS enzyme assays were performed using either the phosphate release or radioactive HPLC method, as previously described in Padgette et al. 1987, using 1 mM phosphoenol pyruvate (PEP, Boehringer) and 2 mM shikimate-3-phosphate (S3P) substrate concentrations. For radioactive HPLC assays, ¹⁴C-PEP (Amersham) was utilized. S3P was synthesized as previously described in Wibbenmeyer et al. 198B. N-terminal amino acid sequencing was performed by loading samples onto a Polybrene precycled filter in aliquots while drying. Automated Edman degradation chemistry was used to determine the N-terminal protein sequence, using an Applied Biosytems Model 470A gas phase sequencer (Hunkapiller et al. 1983) with an Applied Biosystems 120A PTH analyzer.

Five 10-litre fermentations were carried out on a spontaneous "smooth" isolate of strain CP4 that displayed less clumping when grown in liquid culture. This reduced clumping and smooth colony morphology may be due to reduced polysaccharide production by this isolate. In the following section dealing with the purification of the EPSPS enzyme, CP4 refers to the "smooth" isolate - CP4-S1. The cells from the three batches showing the highest specific activities were pooled. Cell paste of *Agrobacterium* sp. CP4 (300 g) was washed twice with 0.5 L of 0.9% saline and collected by centrifugation (30 minutes, 8000 rpm in a GS3 Sorvall rotor). The cell pellet was suspended in 0.9 L extraction buffer (100 mM TrisCl, 1 mM EDTA, 1 mM BAM (Benzamidine), 5 mM DTT, 10% glycerol, pH 7.5) and lysed by 2 passes through a Manton Gaulin cell. The resulting solution was centrifuged (30 minutes, 8000 rpm) and the supernatant was treated with 0.21 L of 1.5% protamine sulfate (in 100 mM TrisCl, pH 7.5, 0.2% w/v final protamine sulfate concentration). After stirring for 1 hour, the mixture was centrifuged (50 minutes, 8000 rpm) and the resulting supernatant treated with solid ammonium sulfate to 40% saturation and stirred for 1 hour. After centrifugation (50 minutes, 8000 rpm), the resulting supernatant was treated with solid ammonium sulfate to 70% saturation, stirred for 50 minutes, and the insoluble protein was collected by centrifugation (1 hour, 8000 rpm). This 40-70% ammonium sulfate fraction was then dissolved in extraction buffer to give a final volume of 0.2 L, and dialyzed twice (Spectrum 10,000 MW cutoff dialysis tubing) against 2 L of extraction buffer for a total of 12 hours.

To the resulting dialyzed 40-70% ammonium sulfate fraction (0.29 L) was added solid ammonium sulfate to give a final concentration of 1 M. This material was loaded (2 ml/min) onto a column (5 cm x 15 cm, 295 ml) packed with phenyl Sepharose CL-4B (Pharmacia) resin equilibrated with extraction buffer containing 1 M ammonium sulfate, and washed with the same buffer (1.5 L, 2 ml/min). EPSPS was eluted with a linear gradient of extraction buffer going from 1 M to 0.00 M ammonium sulfate (total volume of 1.5 L, 2 ml/min). Fractions were collected (20 ml) and assayed for EPSPS activity by the phosphate release assay. The fractions with the highest EPSPS activity (fractions 36-50) were pooled and dialyzed against 3 x 2 L (18 hours) of 10 mM TrisCl, 25 mM KCI, 1 mM EDTA, 5 mM DTT, 10% glycerol, pH 7.8.

The dialyzed EPSPS extract (350 ml) was loaded (5 ml/min) onto a column (2.4 cm x 30 cm, 136 ml) packed with Q-Sepharose Fast Flow (Pharmacia) resin equilibrated with 10 mM TrisCl, 25 mM KCI, 5 mM DTT, 10% glycerol, pH 7.8 (Q Sepharose buffer), and washed with 1 L of the same buffer. EPSPS was eluted with a linear gradient of Q Sepharose buffer going from 0.025 M to 0.40 M KCI (total volume of 1.4 L, 5 ml/min). Fractions were collected (15 ml) and assayed for EPSPS activity by the phosphate release assay. The fractions with the highest EPSPS activity (fractions 47-60) were pooled and the protein was precipitated by adding solid ammonium sulfate to 80% saturation and stirring for 1 hour. The precipitated protein was collected by centrifugation (20 minutes, 12000 rpm in a GSA Sorvall rotor), dissolved in Q Sepharose buffer (total volume of 14 ml), and dialyzed against the same buffer (2 x 1 L, 18 hours).

The resulting dialyzed partially purified EPSPS extract (19 ml) was loaded (1.7 ml/min) onto a Mono Q 10/10 column (Pharmacia) equilibrated with Q Sepharose buffer, and washed with the same buffer (35 ml). EPSPS was eluted with a linear gradient of 0.025 M to 0.35 M KCI (total volume of 119 ml, 1.7 ml/min). Fractions were collected (1.7 ml) and assayed for EPSPS activity by the phosphate release assay. The fractions with the highest EPSPS activity (fractions 30-37) were pooled (6 ml).

The Mono Q pool was made 1 M in ammonium sulfate by the addition of solid ammonium sulfate and 2 ml aliquots were chromatographed on a Phenyl Superose 5/5 column (Pharmacia) equilibrated with 100 mM TrisCl, 5 mM DTT, 1 M ammonium sulfate, 10% glycerol, pH 7.5 (Phenyl Superose buffer). Samples were loaded (1 ml/min), washed with Phenyl Superose buffer (10 ml), and eluted with a linear gradient of Phenyl Superose buffer going from 1 M to 0.00 M ammonium sulfate (total volume of 60 ml, 1 ml/min). Fractions were collected (1 ml) and assayed for EPSPS activity by the phosphate release assay. The fractions from each run with the highest EPSPS activity (fractions ~36-40) were pooled together (10 ml, 2.5 mg protein). For N-terminal amino acid sequence determination, a portion of one fraction (#39 from run 1) was dialyzed against 50 mM NaHCO₃ (2 x 1 L). The resulting pure EPSPS sample (0.9 ml, 77 µg protein) was found to exhibit a single N-terminal amino acid sequence of:

**XH(G)ASSRPATARKSS(G)LX(GXT)V(R)IPG(D)(K)(M)** **(SEQ ID NO:18).**

In this and all amino acid sequences to follow, the standard single letter nomenclature is used. All peptide structures represented in the following description are shown in conventional format wherein the amino group at the N-terminus appears to the left and the carboxyl group at the C-terminus at the right. Likewise, amino acid nomenclature for the naturally occurring amino acids found in protein is as follows: alanine (Ala-,A), asparagine (Asn:N), aspartic acid (Asp: D), arginine (Arg:R), cysteine (Cys:C), glutamic acid (Glu:E), glutamine (Gln-,Q), glycine (Gty:G), histidine (His:H), isoleucine (Ile:l), leucine (Leu:L), lysine (Lys:K), methionine (Met:M), phenylalanine (Phe;F), proline (Pro:P), serine (Ser:S), threonine (Thr:T), tryptophan (Trp:W), tyrosine (Tyr:Y), and valine (Val:V). An "X" is used when the amino acid residue is unknown and parentheses designate that an unambiguous assignment is not possible and the amino acid designation within the parentheses is the most probable estimate based on known information.

The remaining Phenyl Superose EPSPS pool was dialyzed against 50 mM TrisCl, 2 mM DTT, 10 mM KCl. 10% glycerol, pH 7.5 (2 x 1 L). An aliquot (0.55 ml, 0.61 mg protein) was loaded (1 ml/min) onto a Mono Q 5/5 column (Pharmacia) equilibrated with Q Sepharose buffer, washed with the same buffer (5 ml), and eluted with a linear gradient of Q Sepharose buffer going from 0-0.14 M KCI in 10 minutes, then holding at 0.14 M KCI (1 ml/min). Fractions were collected (1 ml) and assayed for EPSPS activity by the phosphate release assay and were subjected to SDSPAGE (10-15%, Phast System, Pharmacia, with silver staining) to determine protein purity. Fractions exhibiting a single band of protein by SDS-PAGE (22-25, 222 µg) were pooled and dialyzed against 100 mM ammonium bicarbonate, pH 8.1 (2 x 1 L, 9 hours).

### Tryspinolysis and peptide sequencing of Agrobacterium sp strain CP4 EPSPS

To the resulting pure *Agrobacterium* sp. strain CP4 EPSPS (111 µg) was added 3 µg of trypsin (Calbiochem), and the trypsinolysis reaction was allowed to proceed for 16 hours at 37°C. The tryptic digest was then chromatographed (1ml/min) on a C18 reverse phase HPLC column (Vydac) as previously described in Padgette et al. 1988 for *E. coli* EPSPS. For all peptide purifications, 0.1% trifluoroacetic acid (TFA, Pierce) was designated buffer "RP-A" and 0.1% TFA in acetonitrile was buffer "RP-B". The gradient used for elution of the trypsinized *Agrobacterium* sp. CP4 EPSPS was: 0-8 minutes, 0% RP-B; 8-28 minutes, 0-15% RP-B; 28-40 minutes, 15-21% RP-B; 40-68 minutes, 21-49% RP-B: 68-72 minutes, 49-75% RP-B; 72-74 minutes, 75-100% RP-B. Fractions were collected (1 ml) and, based on the elution profile at 210 nm, at least 70 distinct peptides were produced from the trypsinized EPSPS. Fractions 40-70 were evaporated to dryness and redissolved in 150 µl each of 10% acetonitrile, 0.1 % trifluoroacetic acid.

The fraction 61 peptide was further purified on the C18 column by the gradient: 0-5 minutes, 0% RP-B: 5-10 minutes, 0-38% RP-B; 10-30 minutes, 38-45% B. Fractions were collected based on the UV signal at 210 nm. A large peptide peak in fraction 24 eluted at 42% RP-B and was dried down, resuspended as described above, and rechromatographed on the C18 column with the gradient: 0-5 minutes, 0% RP-B; 5-12 min, 0-38% RP-B; 12-15 min, 38-39% RP-B; 15-18 minutes, 39% RP-B; 18-20 minutes, 39-41% RP-B; 20-24 minutes, 41% RP-B; 24-28 minutes, 42% RP-B. The peptide in fraction 25, eluting at 41% RP-B and designated peptide 61-24-25, was subjected to N-terminal amino acid sequencing, and the following sequence was determined:

**APSM(I)(D)EYPILAV** **(SEQ ID NO:19).**

The CP4 EPSPS fraction 53 tryptic peptide was further purified by C18HPLC by the gradient 0% B (5 minutes), 0-30% B (5-17 minutes), 30-40% B (17-37 minutes). The peptide in fraction 28, eluting at 34% B and designated peptide 53-28, was subjected to N-terminal amino acid sequencing, and the following sequence was determined:

**ITGLLEGEDVINTGK** **(SEQ ID NO: 20).**

In order to verify the CP4 EPSPS cosmid clone, a number of oligonucleotide probes were designed on the basis of the sequence of two of the tryptic sequences from the CP4 enzyme (Table III). The probe identified as MID was very low degeneracy and was used for initial screening. The probes identified as EDV C and EDV-T were based on the same amino acid sequences and differ in one position (underlined in Table III below) and were used as confirmatory probes, with a positive to be expected only from one of these two probes. In the oligonucleotides below, alternate acceptable nucleotides at a particular position are designated by a "/" such as A/C/T.

**Table III Selected CP4 EPSPS peptide sequence and DNA probes**

| | |
|---|---|
| **PEPTIDE 61-24-25 APSM(I)(XD)EYPILAV (SEQ ID NO:19)** | |
| | **Probe MID; 17-mer, mixed probe; 24-fold degenerate** |
| | **ATGATA/C/TGAC/TGAG/ATAC/TCC (SEQ ID NO:21)** |
| **PEPTIDE 53-28 ITGLLEGEDVINTGK (SEQ ID NO:20)** | |
| | **Probe EDV-C; 17-mer; mixed probe; 48-fold degenerate** |
| | **GAAJGGACITGTA/C/G/TATA/C/TAACAC (SEQ ID NO:22)** |
| | **Probe EDV-T; 17-mer, mixed probe; 48-fold degenerate** |
| | **GAAlGGAC/TGTA/C/G/TATA/C/TAATAC (SEQ ID NO:23)** |

The probes were labeled using gamma-³²P-ATP and polynucleotide kinase. DNA from fourteen of the cosmids described above was restricted with *Eco*RI, transferred to membrane and probed with the olignucleotide probes. The conditions used were as follows: prehybridization was carried out in 6X SSC, 10X Denhardt's for 2-18 hour periods at 60°C, and hybridization was for 48-72 hours in 6X SSC, 10X Denhardt's, 100 µg/ml tRNA at 10°C below the T_{d} for the probe. The T_{d} of the probe was approximated by the formula 2°C x (A+T) + 4°C x (G+C). The filters were then washed three times with 6X SSC for ten minutes each at room temperature, dried and autoradiographed. Using the MID probe, an ~9.9 kb fragment in the pMON17076 cosmid gave the only positive signal. This cosmid DNA was then probed with the EDV-C (SEQ ID NO:22) and EDV-T (SEO ID NO:23) probes separately and again this ~9.9 kb band gave a signal and only with the EDV-T probe.

The combined data on the glyphosate tolerant phenotype, the complementation of the *E. coli aroA-* phenotype, the expression of a ~45 Kd protein, and the hybridization to two probes derived from the CP4 EPSPS amino acid sequence strongly suggested that the pMON17076 cosmid contained the EPSPS gene.

### Localization and subcloning of the CP4 EPSPS gene

The CP4 EPSPS gene was further localized as follows: a number of additional Southern analyses were carried out on different restriction digests of pMON17076 using the MID (SEQ ID NO:21) and EDV-T (SEQ ID NO:23) probes separately. Based on these analyses and on subsequent detailed restriction mapping of the pBlueScript (Stratagene) subclones of the ~9.9 kb fragment from pMON17076, a 3.8 kb *EcoR*I *-Sal*I fragment was identified to which both probes hybridized. This analysis also showed that MID (SEQ ID NO:21) and EDV-T (SEQ ID NO:23) probes hybridized to different sides of *BamHl, Cla*I, and *Sac*II sites. This 3.8 kb fragment was cloned in both orientations in pBlueScript to form pMON17081 and pMON17082. The phenotypes imparted to *E. coli* by these clones were then determined. Glyphosate tolerance was determined following transformation into *E. coli* MM294 containing pGP1-2 (pBlueScript also contains a T7 promoter) on M9 agar media containing glyphosate at 3 mM. Both pMON17081 and pMON17082 showed glyphosate tolerant colonies at three days at 30°C at about half the size of the controls on the same media lacking glyphosate. This result suggested that the 3.8 kb fragment contained an intact EPSPS gene. The apparent lack of orientation-dependence of this phenotype could be explained by the presence of the T7 promoter at one side of the cloning sites and the *lac* promoter at the other. The *aroA* phenotype was determined in transformants of *E*. *coli* GB100 on M9 agar media lacking aromatic supplements. In this experiment, carried out with and without the *Plac* inducer IPTG, pMON17082 showed much greater growth than pMON17081, suggesting that the EPSPS gene was expressed from the *Sal*I site towards the *Eco*RI site.

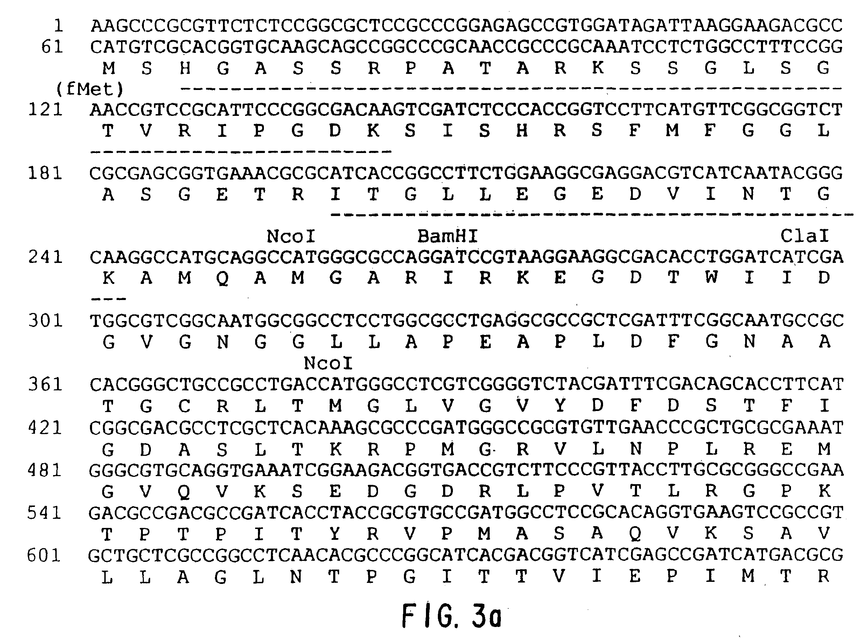

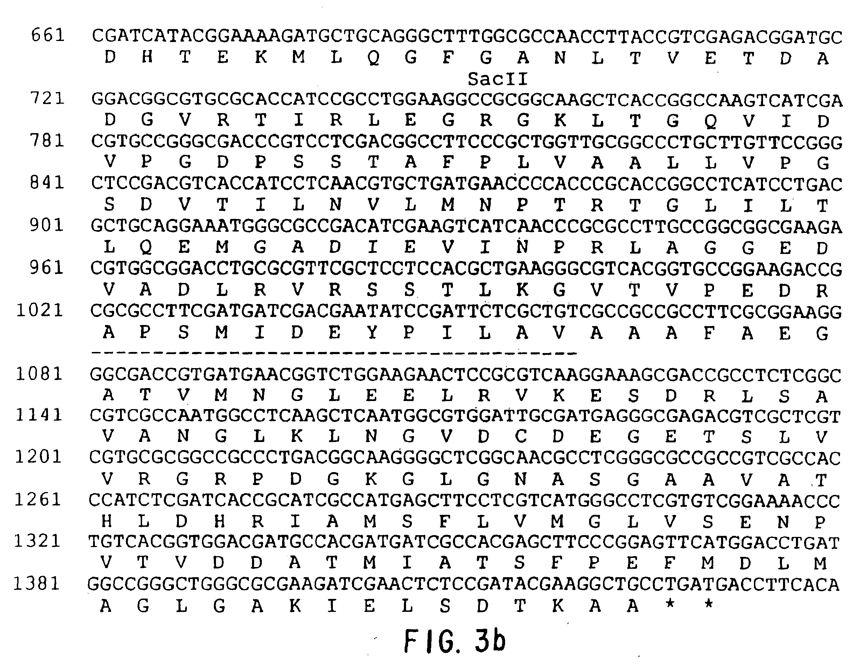

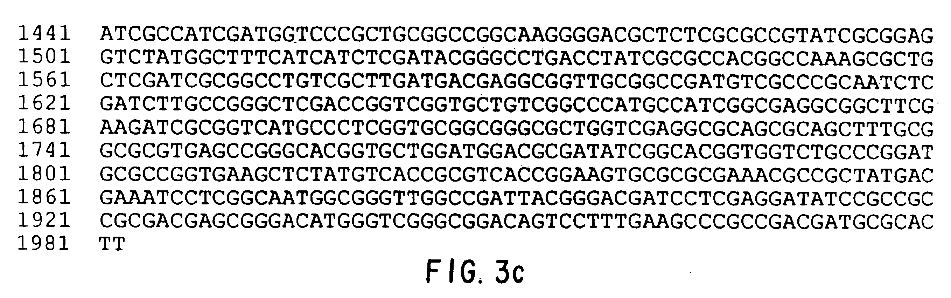

Nucleotide sequencing was begun from a number of restriction site ends, including the *Bam*HI site discussed above. Sequences encoding protein sequences that closely matched the N-terminus protein sequence and that for the tryptic fragment 53-28 (SEQ ID NO:20) (the basis of the EDV-T probe) (SEQ ID NO:23) were localized to the *Sal*I side of this *Bam*HI site. These data provided conclusive evidence for the cloning of the CP4 EPSPS gene and for the direction of transcription of this gene. These data coupled with the restriction mapping data also indicated that the complete gene was located on an ~2.3 kb *Xho*I fragment and this fragment was subcloned into pBlueScript. The nucleotide sequence of almost 2 kb of this fragment was determined by a combination of sequencing from cloned restriction fragments and by the use of specific primers to extend the sequence. The nucleotide sequence of the CP4 EPSPS gene and flanking regions is shown in Figure 3 (SEQ ID NO:2). The sequence corresponding to peptide 61-24-25 (SED ID NO:19) was also located. The sequence was determined using both the Sequenase kit from IBI (International Biotechnologies Inc.) and the T7 sequencing /Deaza Kit from Pharmacia.

That the cloned gene encoded the EPSPS activity purified from the *Agrobacterium* sp. strain CP4 was verified in the following manner: By a series of site directed mutageneses, *Bgl*II and *Nco*I sites were placed at the N-terminus with the fMet contained within the *Nco*I recognition sequence, the first internal *Nco*I site was removed (the second internal Ncol site was removed later), and a Sad site was placed after the stop codons. At a later stage the internal *Not*I site was also removed by site-directed mutagenesis. The following list includes the primers for the site-directed mutagenesis (addition or removal of restriction sites) of the CP4 EPSPS gene. Mutagenesis was carried out by the procedures of Kunkel et al. (1987), essentially as described in Sambrook et al. (1989).
PRIMER BgNc (addition *of Bgl*II and *Nco*I sites to N-terminus)
   **CGTGGATAGATCTAGGAAGACAACCATGGCTCACGGTC (SEQ ID NO:24)**
PRIMER Sph2 (addition of *Sph*I site to N-terminus)
   **GGATAGATTAAGGAAGACGCGCATGCTTCACGGTGCAAGC AGCC (SEQ ID NO:25)**
PRIMER S1 (addition *of Sac*I site immediately after stop codons)
   **GGCTGCCTGATGAGCTCCACAATCGCCATCGATGG (SEQ ID NO:26)**
PRIMER N1 (removal of internal *Not*l recognition site)
   **CGTCGCTCGTCGTGCGTGGCCGCCCTGACGGC (SEQ ID NO:27)**
PRIMER Nco1 (removal of first internal *Nco*I recognition site)
   **CGGGCAAGGCCATGCAGGCTATGGGCGCC (SEQ ID NO:28)**
PRIMER Nco2 (removal of second internal *Nco*I recognition site)
   **CGGGCTGCCGCCTGACTATGGGCCTCGTCGG (SEQ ID NO:29)**

This CP4 EPSPS gene was then cloned as a *Nco*I*-BamH*I N-terminal fragment plus a *BamH*I*-Sac*I C-terminal fragment into a *PrecA-gene10L* expression vector similar to those described (Wong et al., 1988; Olins et al., 1988) to form pMON17101. The Kₘ for PEP and the Kᵢ for glyphosate were determined for the EPSPS activity in crude lysates of pMON17101/GB100 transformants following induction with nalidixic acid (Wong et al., 1988) and found to be the same as that determined for the purified and crude enzyme preparations from *Agrobacterium* sp. strain CP4.

### Characterization of the EPSPS gene from Achromobacter sp. strain LBAA and from Pseudomonas sp. strain PG2982

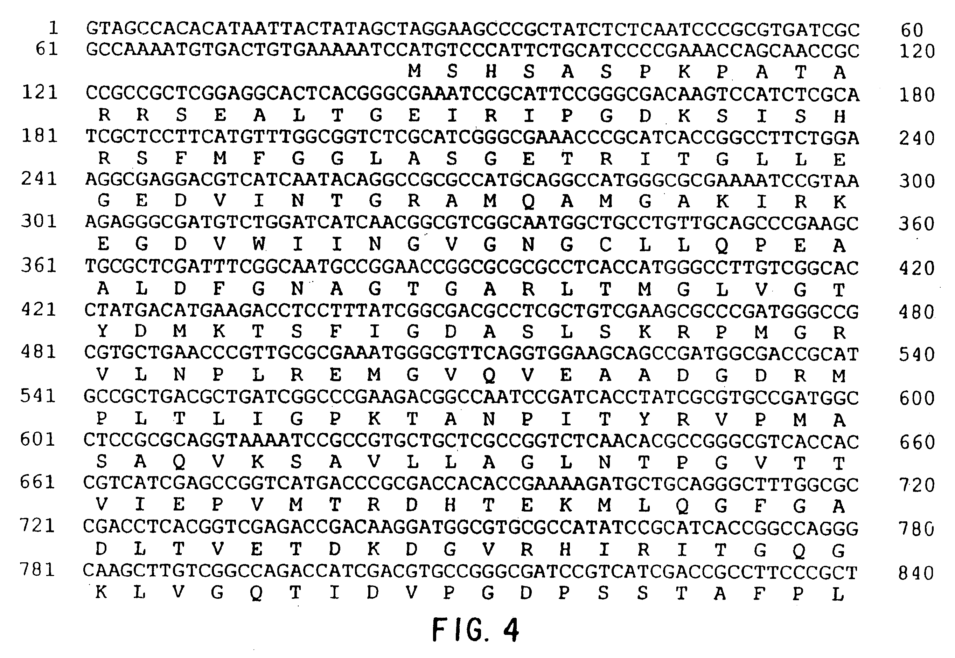

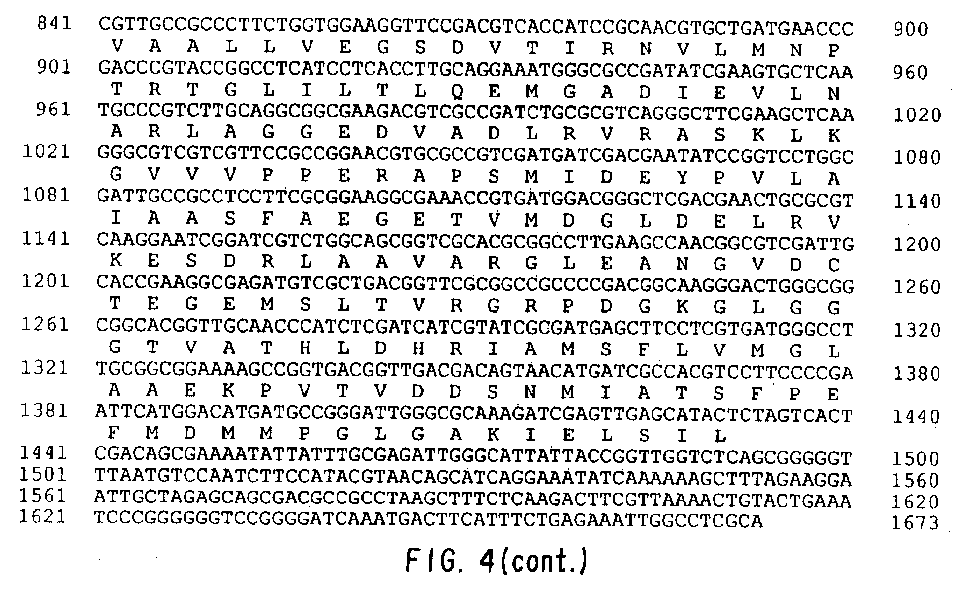

A cosmid bank of partially *Hin*dIII-restricted LBAA DNA was constructed in *E. coli* MM294 in the vector pHC79 (Hohn and Collins, 1980). This bank was probed with a full length CP4 EPSPS gene probe by colony hybridization and positive clones were identified at a rate of ~1 per 400 cosmids. The LBAA EPSPS gene was further localized in these cosmids by Southern analysis. The gene was located on an ~2.8 kb Xhol fragment and by a series of sequencing steps, both from restriction fragment ends and by using the oligonucleotide primers from the sequencing of the CP4 EPSPS gene, the nucleotide sequence of the LBAA EPSPS gene was completed and is presented in Figure 4 (SEQ ID NO:4).

The EPSPS gene from PG2982 was also cloned. The EPSPS protein was purified, essentially as described for the CP4 enzyme, with the following differences: Following the Sepharose CL-4B column, the fractions with the highest EPSPS activity were pooled and the protein precipitated by adding solid ammonium sulfate to 85% saturation and stirring for 1 hour. The precipitated protein was collected by centrifugation, resuspended in Q Sepharose buffer and following dialysis against the same buffer was loaded onto the column (as for the CP4 enzyme). After purification on the Q Sepharose column, ~40 mg of protein in 100 mM Tris pH 7.8, 10% glycerol, 1 mM EDTA, 1 mM DTT, and 1 M ammonium sulfate, was loaded onto a Phenyl Superose (Pharmacia) column. The column was eluted at 1.0 ml/minutes with a 40 ml gradient from 1.0 M to 0.00 M ammonium sulfate in the above buffer.

Approximately 1.0 mg of protein from the active fractions of the Phenyl Superose 10/10 column was loaded onto a Pharmacia Mono P 5/10 Chromatofocusing column with a flow rate of 0.75 ml/minutes. The starting buffer was 25 mM bis-Tris at pH 6.3, and the column was eluted with 39 ml of Polybuffer 74, pH 4.0. Approximately 50 µg of the peak fraction from the Chromatofocusing column was dialyzed into 25 mM ammonium bicarbonate. This sample was then used to determine the N-terminal amino acid sequence.

The N-terminal sequence obtained was:
XHSASPKPATARRSE (where X = an unidentified residue) (SEQ ID NO:30). A number of degenerate oligonucleotide probes were designed based on this sequence and used to probe a library of PG2982 partial-*Hind*III DNA in the cosmid pHC79 (Hohn and Collins, 1980) by colony hybridization under nonstringent conditions. Final washing conditions were 15 minutes with 1X SSC, 0.1% SDS at 55°C. One probe with the sequence GCGGTBGCSG-GYTTSGG (where B = C, G, or T: S = C or G, and Y = C or T) (SEQ ID NO:31) identified a set of cosmid clones.

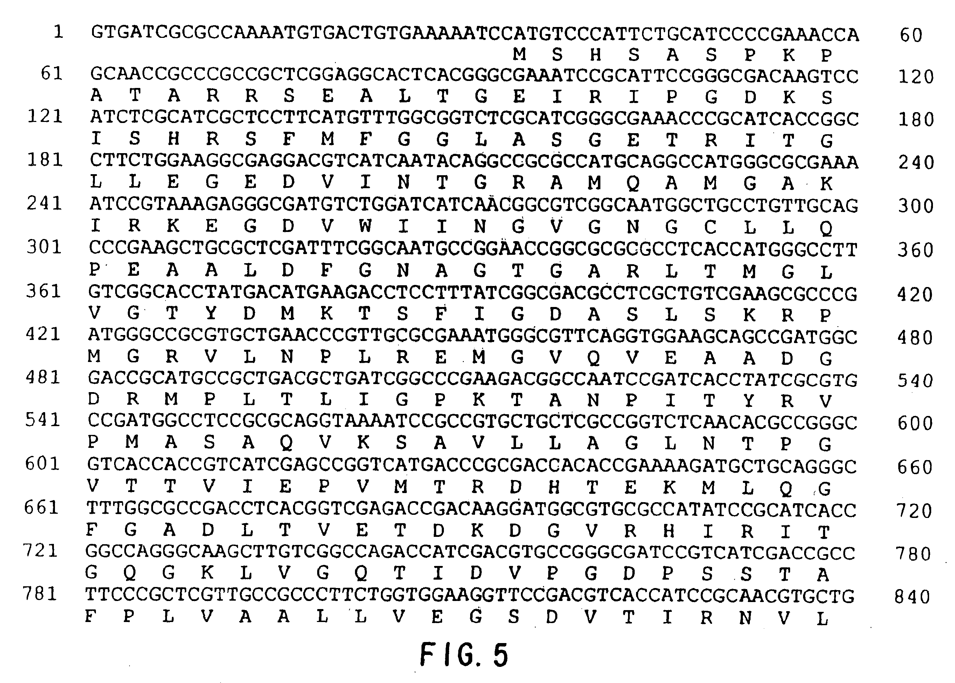

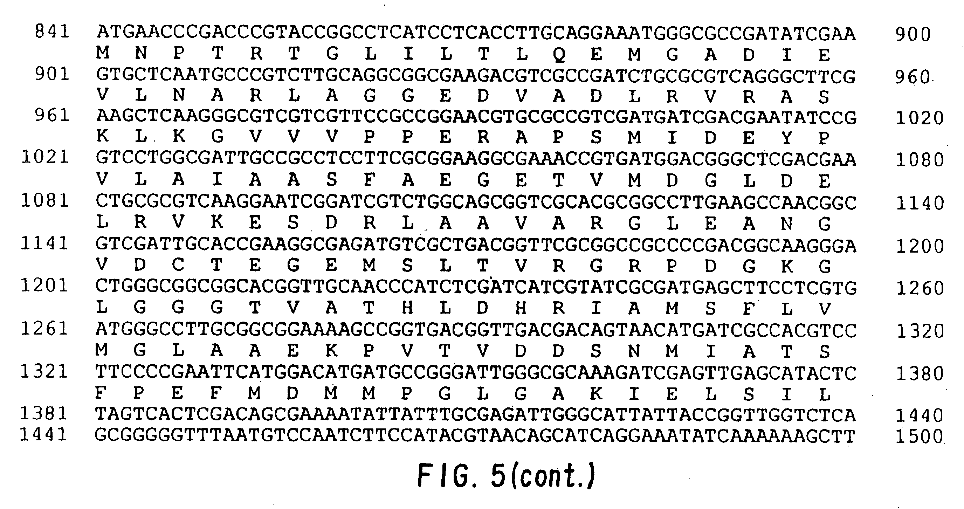

The cosmid set identified in this way was made up of cosmids of diverse Hindlll fragments. However, when this set was probed with the CP4 EPSPS gene probe, a cosmid containing the PG2982 EPSPS gene was identified (designated as cosmid 9C1 originally and later as pMON20107). By a series of restriction mappings and Southern analysis this gene was localized to a ~2.8 kb Xhol fragment and the nucleotide sequence of this gene was determined. This DNA sequence (SEQ ID NO:6) is shown in Figure 5. There are no nucleotide differences between the EPSPS gene sequences from LBAA (SEQ ID NO:4) and PG2982 (SEQ ID NO:6). The kinetic parameters of the two enzymes are within the range of experimental error.

A gene from PG2982 that imparts glyphosate tolerance in *E. coli* has been sequenced (Fitzgibbon, 1988: Fitzgibbon and Braymer, 1990). The sequence of the PG2982 EPSPS Class II gene shows no homology to the previously reported sequence suggesting that the glyphosate tolerant phenotype of the previous work is not related to EPSPS.

### Alternative Isolation Protocols for Other Class II EPSPS Structural Genes

A number of Class II genes have been isolated and described here. It is clear that the initial gene cloning, that of the gene from CP4, was difficult due to the low degree of similarity between the Class I and Class II enzymes and genes. The identification of the other genes however was greatly facilitated by the use of this first gene as a probe. In the cloning of the LBAA EPSPS gene, the CP4 gene probe allowed the rapid identification of cosmid clones and the localization of the intact gene to a small restriction fragment and some of the CP4 sequencing primers were also used to sequence the LBAA (and PG2982) EPSPS gene(s). The CP4 gene probe was also used to confirm the PG2982 gene clone. The high degree of similarity of the Class II EPSPS genes may be used to identify and clone additional genes in much the same way that Class I EPSPS gene probes have been used to clone other Class I genes. An example of the latter was in the cloning of the *A. thaliana* EPSPS gene using the *P. hybrida* gene as a probe (Klee et al., 1987).

Glyphosate tolerant EPSPS activity has been reported previously for EPSP synthases from a number of sources. These enzymes have not been characterized to any extent in most cases. The use of Class I and Class II EPSPS gene probes or antibody probes provide a rapid means of initially screening for the nature of the EPSPS and provide tools for the rapid cloning and characterization of the genes for such enzymes.

Two of the three genes described were isolated from bacteria that were isolated from a glyphosate treatment facility (Strains CP4 and LBAA). The third (PG2982) was from a bacterium that had been isolated from a culture collection strain. This latter isolation suggests that exposure to glyphosate may not be a prerequisite for the isolation of high glyphosate tolerant EPSPS enzymes and that the screening of collections of bacteria could yield additional isolates. It is possible to enrich for glyphosate degrading or glyphosate resistant microbial populations (Quinn et al., 1988; Talbot et al., 1984) in cases where it was felt that enrichment for such microorganisms would enhance the isolation frequency of Class II EPSPS microorganisms. Additional bacteria containing class II EPSPS gene have also been identified. A bacterium called C12, isolated from the same treatment column beads as CP4 (see above) but in a medium in which glyphosate was supplied as both the carbon and phosphorus source, was shown by Southern analysis to hybridize with a probe consisting of the CP4 EPSPS coding sequence. This result, in conjunction with that for strain LBAA, suggests that this enrichment method facilitates the identification of Class II EPSPS isolates. New bacterial isolates containing Class II EPSPS genes have also been identified from environments other than glyphosate waste treatment facilities. An inoculum was prepared by extracting soil (from a recently harvested soybean field in Jerseyville, Illinois) and a population of bacteria selected by growth at 28°C in Dworkin-Foster medium containing glyphosate at 10 mM as a source of carbon (and with cycloheximide at 100 µg/ml to prevent the growth of fungi). Upon plating on L-agar media, five colony types were identified. Chromosomal DNA was prepared from 2ml L-broth cultures of these isolates and the presence of a Class II EPSPS gene was probed using a the CP4 EPSPS coding sequence probe by Southern analysis under stringent hybridization and washing conditions. One of the soil isolates, S2, was positive by this screen.

### Relationships between different EPSPS genes

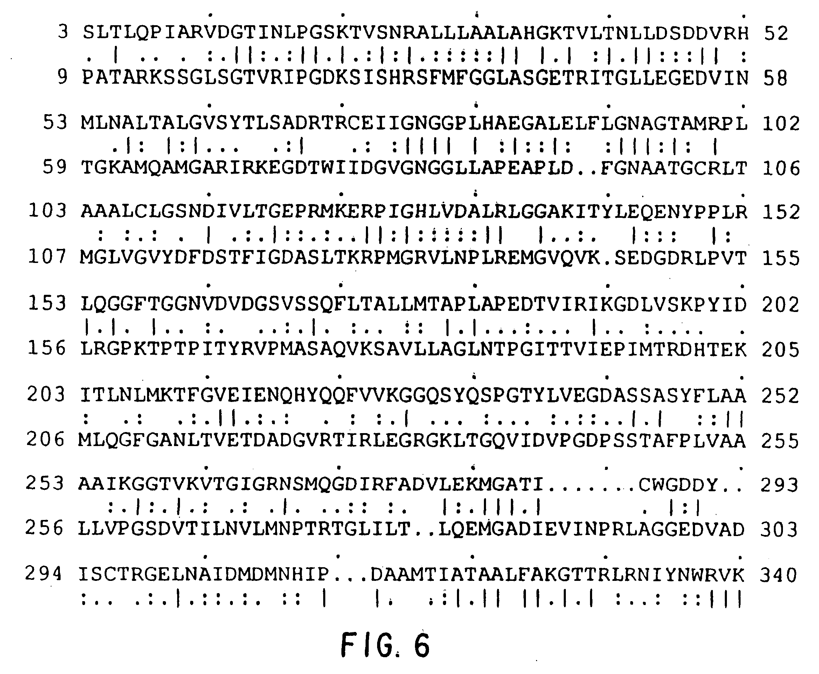

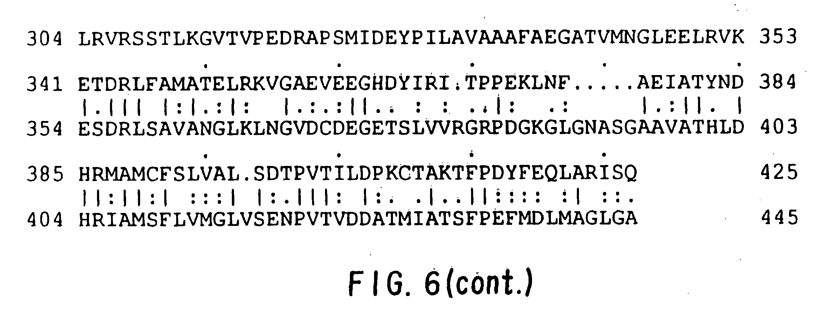

The deduced amino acid sequences of a number of Class I and the Class II EPSPS enzymes were compared using the Bestfit computer program provided in the UWGCG package (Devereux et al. 1984). The degree of similarity and identity as determined using this program is reported. The degree of similarity/identity determined within Class I and Class II protein sequences is remarkably high, for instance, comparing *E. coli* with *S. typhimurium* (similarity/identity = 93%/88%) and even comparing *E. coli* with a plant EPSPS i*Petunia hybrida; 72%*/*55%).* This data is shown in Table IV. The comparison of sequences between Class I and Class II, however, shows only a very low degree of relatedness between the Classes (similarity/identity = 50-53%/23-30%). The display of the Bestfit analysis for the *E.coli* (SEQ ID NO:8) and CP4 (SEQ ID NO:3) sequences shows the positions of the conserved residues and is presented in Figure 6. Previous analyses of EPSPS sequences had noted the high degree of conservation of sequences of the enzymes and the almost invariance of sequences in two regions - the "20-35" and "95-107" regions (Gasser et al., 1988: numbered according to the Petunia EPSPS sequence) - and these regions are less conserved in the case of CP4 and LBAA when compared to Class I bacterial and plant EPSPS sequences (see Figure 6 for a comparison of the *E. coli* and CP4 EPSPS sequences with the *E. coli* sequence appearing as the top sequence in the Figure). The corresponding sequences in the CP4 Class 11 EPSPS are:
**PGDKSISHRSFMFGGL (SEQ ID NO:32) and LDFGNAATGCRLT (SEQ ID NO:33).**

These comparisons show that the overall relatedness of Class I and Class II is EPSPS proteins is low and that sequences in putative conserved regions have also diverged considerably.

In the CP4 EPSPS an alanine residue is present at the "glycine101" position. The replacement of the conserved glycine (from the "95-107" region) by an alanine results in an elevated Kᵢ for glyphosate and in an elevation in the Kₘ for PEP in Class I EPSPS. In the case of the CP4 EPSPS, which contains an alanine at this position, the Kₘ for PEP is in the low range, indicating that the Class II enzymes differ in many aspects from the EPSPS enzymes heretofore characterized.

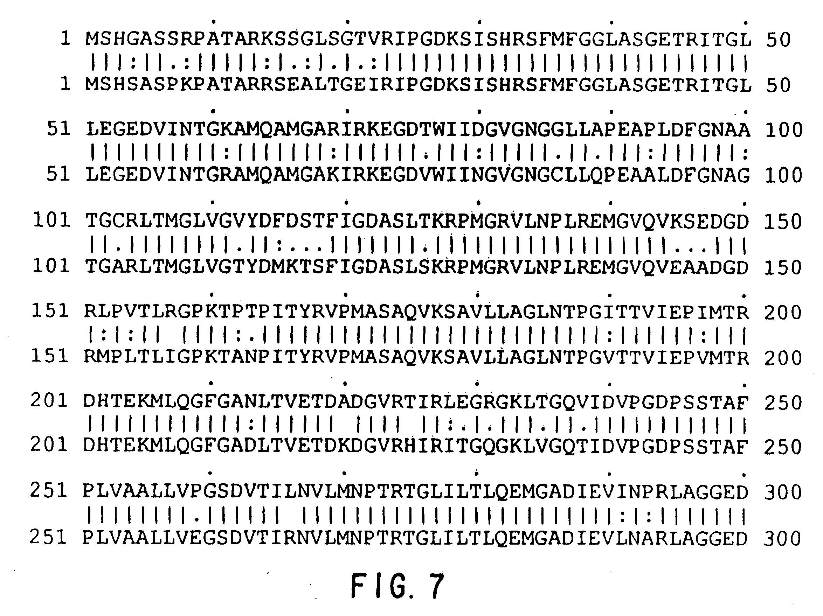

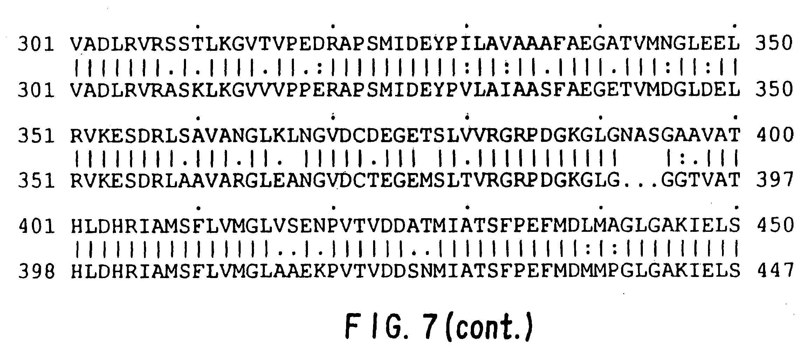

Within the Class II isolates, the degree of similarity/identity is as high as that noted for that within Class I (Table IV). Figure 7 displays the Bestfit computer program alignment of the CP4 (SEQ ID NO:3) and LBAA (SEQ ID NO:5) EPSPS deduced amino acid sequences with the CP4 sequence appearing as the top sequence in the Figure. The symbols used in Figures 6 and 7 are the standard symbols used in the Bestfit computer program to designate degrees of similarity and identity.

**Table IV**

| **Comparison of relatedness of EPSPS protein sequences**^{**1**} **Comparison between Class I and Class II EPSPS protein sequences** | | |
|---|---|---|
| | similarity | identity |
| *E. coli* vs. CP4 | 52.8 | 26.3 |
| *E. coli* vs*.* LBAA | 52.1 | 26.7 |
| *S. typhimurium* vs. CP4 | 51.8 | 25.8 |
| *B. pertussis* vs. CP4 | 52.8 | 27.3 |
| *S. cerevisiae* vs. CP4 | 53.5 | 29.9 |
| *P. hybrida* vs. CP4 | 50.2 | 23.4 |

| **Comparison between Class I EPSPS protein sequences** | | |
|---|---|---|
| | similarity | identity |
| *E. coli* vs. *S. typhimurium* | 93.0 | 88.3 |
| *P. hybrida* vs. *E*. *coli* | 71.9 | 54.5 |

| **Comparison between Class II EPSPS protein sequences** | | |
|---|---|---|
| | similarity | identity |
| *Agrobacterium* sp. strain CP4vs. *Achromobacter* sp. strain LBAA | 89.9 | 83.7 |

| | | |
|---|---|---|
| ¹ The EPSPS sequences compared here were obtained from the following references: *E. coli,* Rogers et al., 1983: S. *typhimurium.* Stalker et al., 1985: *Petunia hybrida,* Shah et al.. 1986: *B. pertussis,* Maskell et al., 1988; and *S. cerevisiae,* Duncan et al., 1987. | | |

One difference that may be noted between the deduced amino acid sequences of the CP4 and LBAA EPSPS proteins is at position 100 where an Alanine is found in the case of the CP4 enzyme and a Glycine is found in the case of the LBAA enzyme. In the Class IEPSPS enzymes a Glycine is usually found in the equivalent position, i.e Glycine96 in *E. coli* and *K. pneumoniae* and Glycine101 in Petunia. In the case of these three enzymes it has been reported that converting that Glycine to an Alanine results in an elevation of the appKi for glyphosate and a concomitant elevation in the appKm for PEP (Kishore et al. 1986; Kishore and Shah, 1988: Sost and Amrhein, 1990), which, as discussed above, makes the enzyme less efficient especially under conditions of lower PEP concentrations. The Glycine 100 of the LBAA EPSPS was converted to an Alanine and both the appKm for PEP and the appKi for glyphosate were determined for the variant. The Glycinel00Alanine change was introduced by mutagenesis using the following primer:
**CGGCAATGCCGCCACCGGCGCGCGCC (SEQ ID NO:34)**
and both the wild type and variant genes were expressed in *E. coli* in a *RecA* promoter expression vector (pMON17201 and pMON1 7264, respectively) and the appKm's and appKi's determined in crude lysates. The data indicate that the appKi(glyphosate) for the G100A variant is elevated about 16-fold (Table V). This result is in agreement with the observation of the importance of this G-A change in raising the appKi(glyphosate) in the Class I EPSPS enzymes. However, in contrast to the results in the Class I G-A variants, the appKm(PEP) in the Class II (LBAA) G-A variant is unaltered. This provides yet another distinction between the Class II and Class I EPSPS enzymes.

**Table V**

| | appKm(PEP) | appKi(glyphosate) |
|---|---|---|
| Lysate prepared from: | | |
| *E. coli*/pMON17201 (wild type) | 5.3 µM | 28 µM⁻ |
| *E. coli*/pMON17264 (G100A variant) | 5.5 µM | 459 µM# |
| @ range of PEP: 2-40 µM | | |

| | | |
|---|---|---|
| range of glyphosate: 0-310 µM: # range of glyphosate: 0-5000 µM. | | |

The LBAA G100A variant, by virtue of its superior kinetic properties, is capable of imparting improved glyphosate *in planta.*

### Modification and Resynthesis of the Agrobacterium sp. strain CP4 EPSPS Gene Sequence

The EPSPS gene from *Agrobacterium* sp. strain CP4 contains sequences that could be inimical to high expression of the gene in plants. These sequences include potential polyadenylation sites that are often and A+T rich, a higher G+C% than that frequently found in plant genes (63% *versus ∼*50%), concentrated stretches of G and C residues, and codons that are not used frequently in plant genes. The high G+C% in the CP4 EPSPS gene has a number of potential consequences including the following: a higher usage of G or C than that found in plant genes in the third position in codons, and the potential to form strong hair-pin structures that may affect expression or stability of the RNA. The reduction in the G+C content of the CP4 EPSPS gene, the disruption of stretches of G's and C's, the elimination of potential polyadenylation sequences, and improvements in the codon usage to that used more frequently in plant genes, could result in higher expression of the CP4 EPSPS gene in plants.

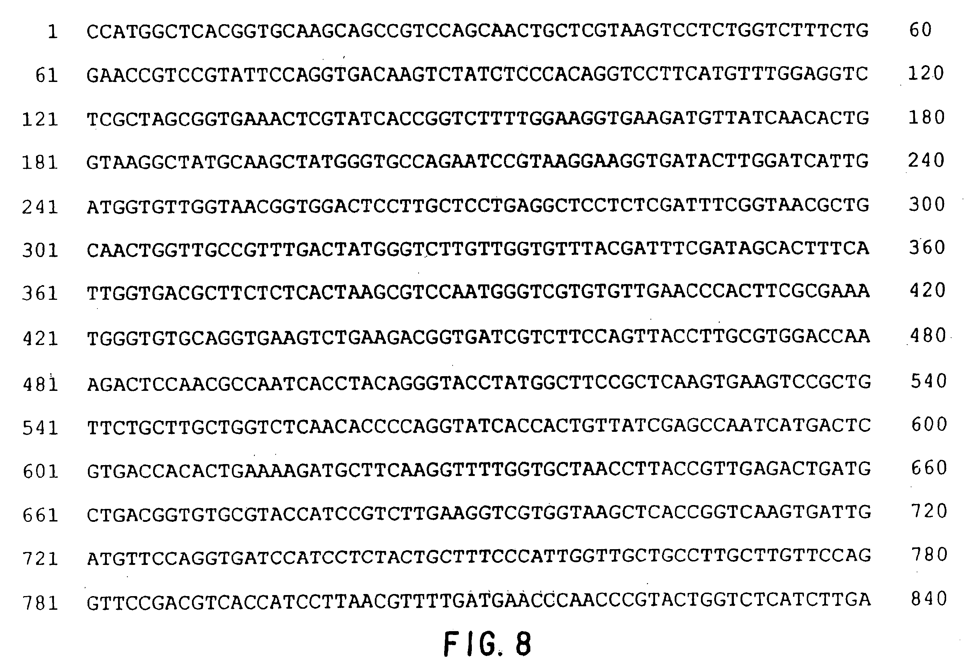

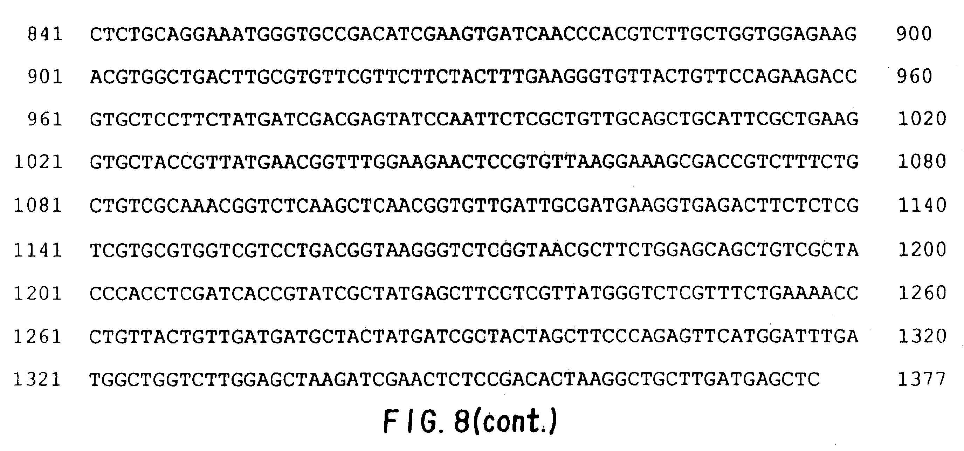

A synthetic CP4 gene was designed to change as completely as possible those inimical sequences discussed above. In summary, the gene sequence was redesigned to eliminate as much as possible the following sequences or sequence features (while avoiding the introduction of unnecessary restriction sites): stretches of G's and C's of 5 or greater; and A+T rich regions (predominantly) that could function as polyadenylation sites or potential RNA destabilization region The sequence of this gene is shown in Figure 8 (SEQ ID NO:9). This coding sequence was expressed in *E. coli* from the *RecA* promoter and assayed for EPSPS activity and compared with that from the native CP4 EPSPS gene. The apparent Km for PEP for the native and synthetic genes was 11.8 and 12.7, respectively, indicating that the enzyme expressed from the synthetic gene was unaltered. The N-terminus of the coding sequence was mutagenized to place an Sphl site at the ATG to permit the construction of the CTP2-CP4 synthetic fusion for chloroplast import. The following primer was used to accomplish this mutagenesis:

**GGACGGCTGCTTGCACCGTGAAGCATGCTTAAGCTTGGCGT AATCATGG** **(SEQ ID NO:35).**

### Expression of Chloroplast Directed CP4 EPSPS

The glyphosate target in plants, the 5-*enol*pyruvyl-shikimate-3-phosphate synthase (EPSPS) enzyme, is located in the chloroplast. Many chloroplast-localized proteins, including EPSPS, are expressed from nuclear genes as precursors and are targeted to the chloroplast by a chloroplast transit peptide (CTP) that is removed during the import steps. Examples of other such chloroplast proteins include the small subunit (SSU) of Ribulose-1,5-bisphosphate carboxylase (RUBISCO), Ferredoxin, Ferredoxin oxidoreductase, the Light-harvesting-complex protein I and protein II, and Thioredoxin F. It has been demonstrated *in vivo* and *in vitro* that non-chloroplast proteins may be targeted to the chloroplast by use of protein fusions with a CTP and that a CTP sequence is sufficient to target a protein to the chloroplast.

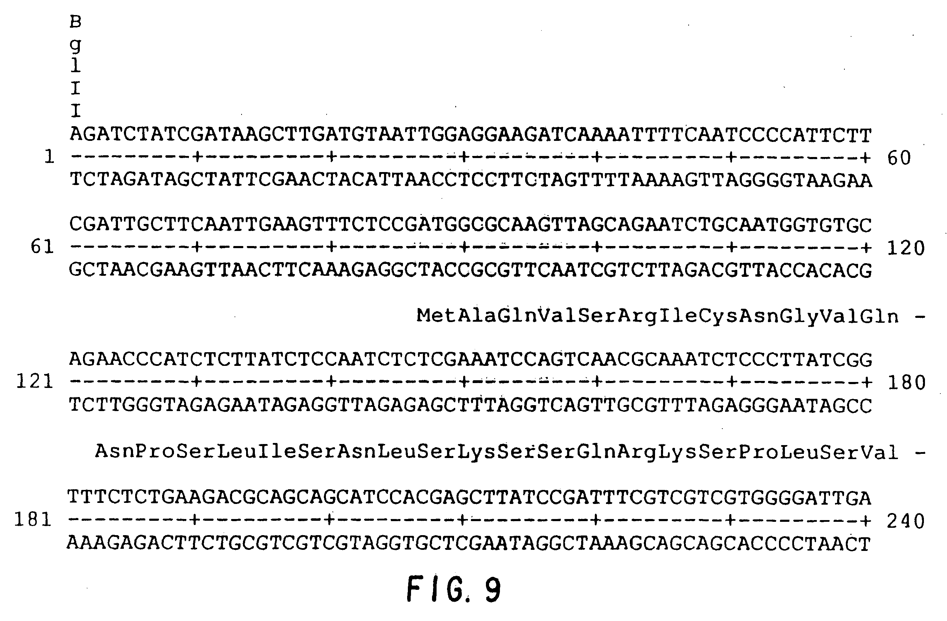

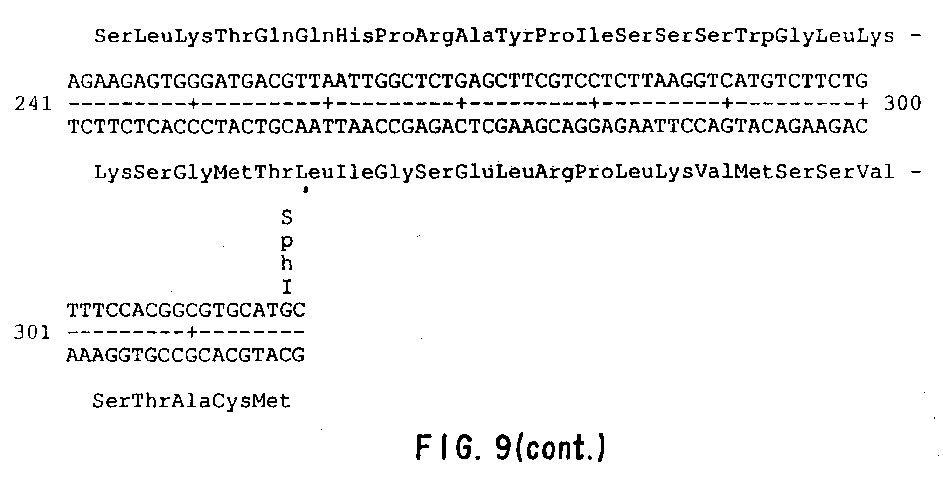

A CTP-CP4 EPSPS fusion was constructed between the *Arabidopsis thaliana* EPSPS CTP (Klee et al., 1987) and the CP4 EPSPS coding sequences. The *Arabidopsis* CTP was engineered by site-directed mutagenesis to place a *Sph*l restriction site at the CTP processing site. This mutagenesis replaced the Glu-Lys at this location with Cys-Met. The sequence of this CTP, designated as CTP2 (SEQ ID NO:10), is shown in Figure 9. The N-terminus of the CP4 EPSPS gene was modified to place a *Sph*l site that spans the Met codon. The second codon was converted to one for leucine in this step also. This change had no apparent effect on the *in vivo* activity of CP4 EPSPS in *E. coli* as judged by rate of complementation of the *aroA* allele. This modified N-terminus was then combined with the *Sac*l C-terminus and cloned downstream of the CTP2 sequences. The CTP2-CP4 EPSPS fusion was cloned into pBlueScript KS(+). This vector may be transcribed *in vitro* using the T7 polymerase and the RNA translated with ³⁵S-Methionine to provide material that may be evaluated for import into chloroplasts isolated from *Lactuca sativa* using the methods described hereinafter (della-Cioppa et al., 1986, 1987). This template was transcribed *in vitro* using T7 polymerase and the ³⁵S-methionine-labeled CTP2-CP4 EPSPS material was shown to import into chloroplasts with an efficiency comparable to that for the control Petunia EPSPS (control = ³⁵S labeled PreEPSPS [pMON6140: della-Cioppa et al., 1986]).

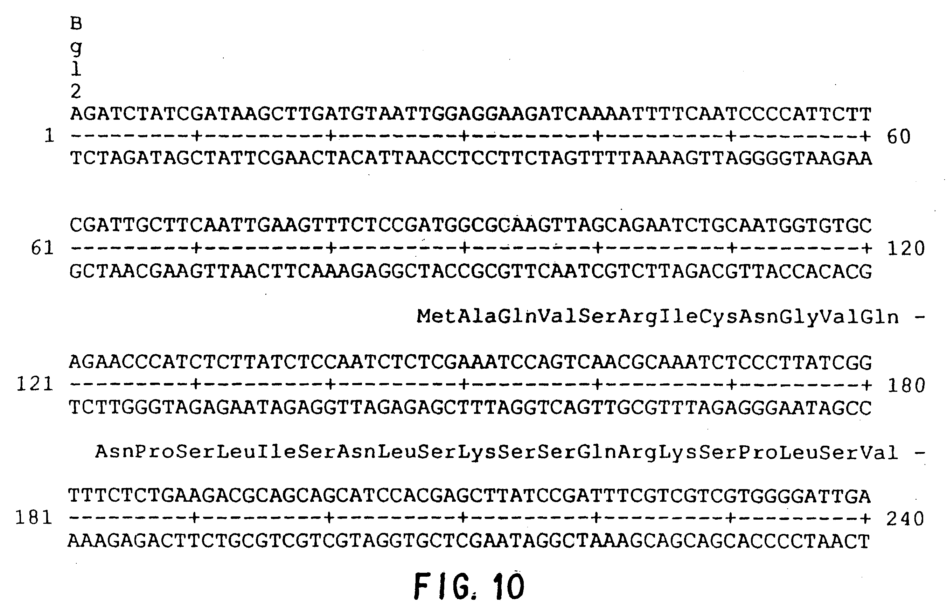

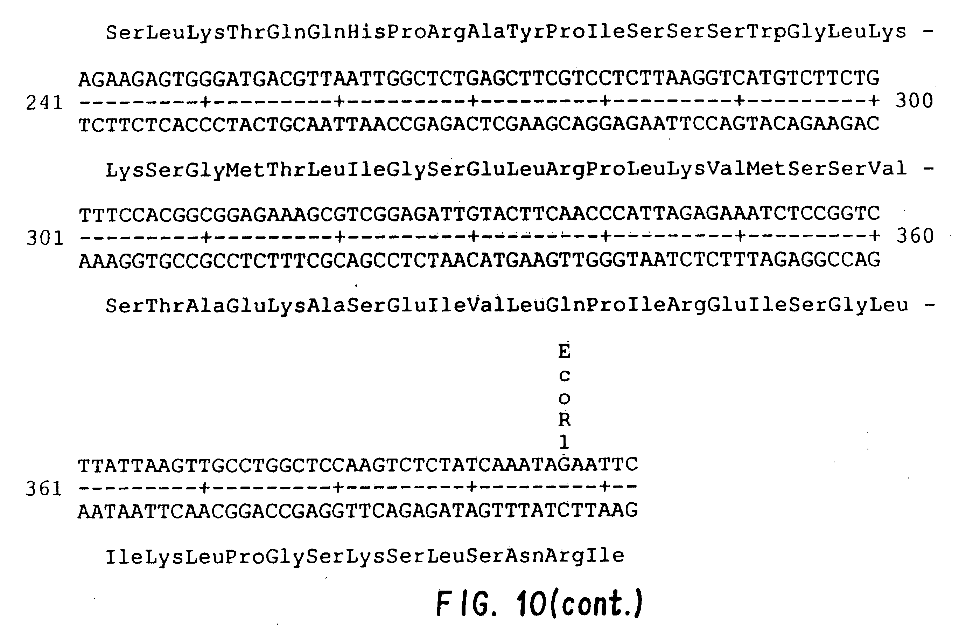

In another example the *Arabidopsis* EPSPS CTP, designated as CTP3, was fused to the CP4 EPSPS through an EcoRl site. The sequence of this CTP3 (SEQ ID NO:12) is shown in Figure 10. An *Eco*RI site was introduced into the *Arabidopsis* EPSPS mature region around amino acid 27, replacing the sequence -Arg-Ala-Leu-Leu- with -Arg-I le-Leu-Leu- in the process. The primer of the following sequence was used to modify the N-terminus of the CP4 EPSPS gene to add an *Eco*RI site to effect the fusion to the CTP3:
**GGAAGACGCCCAGAATTCACGGTGCAAGCAGCCGG (SEQ ID NO:36) (the *Eco*RI site is underlined).**
This CTP3-CP4 EPSPS fusion was also cloned into the pBlueScript vector and the T7 expressed fusion was found to also import into chloroplasts with an efficiency comparable to that for the control Petunia EPSPS (pMON6140).

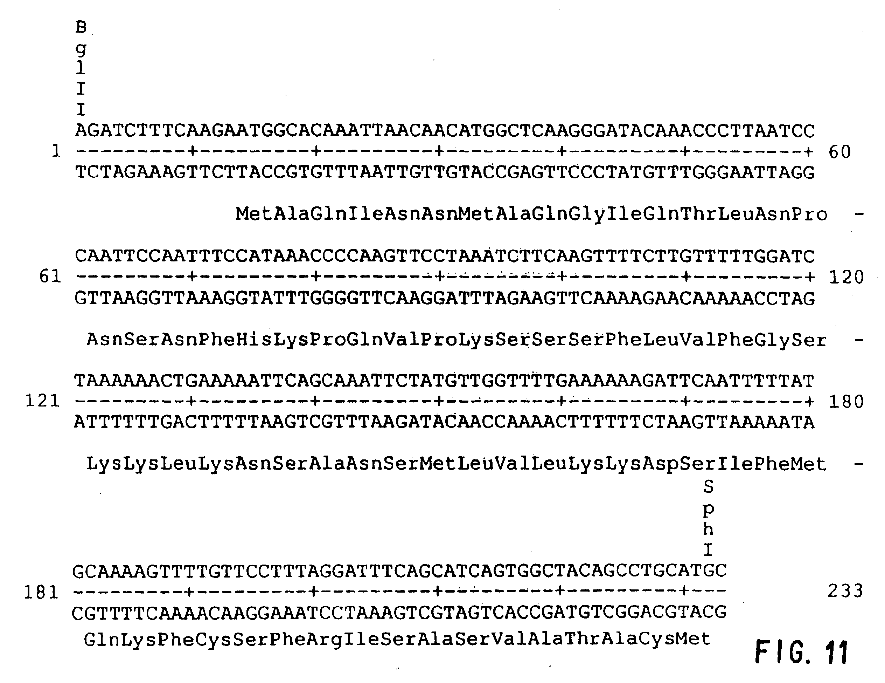

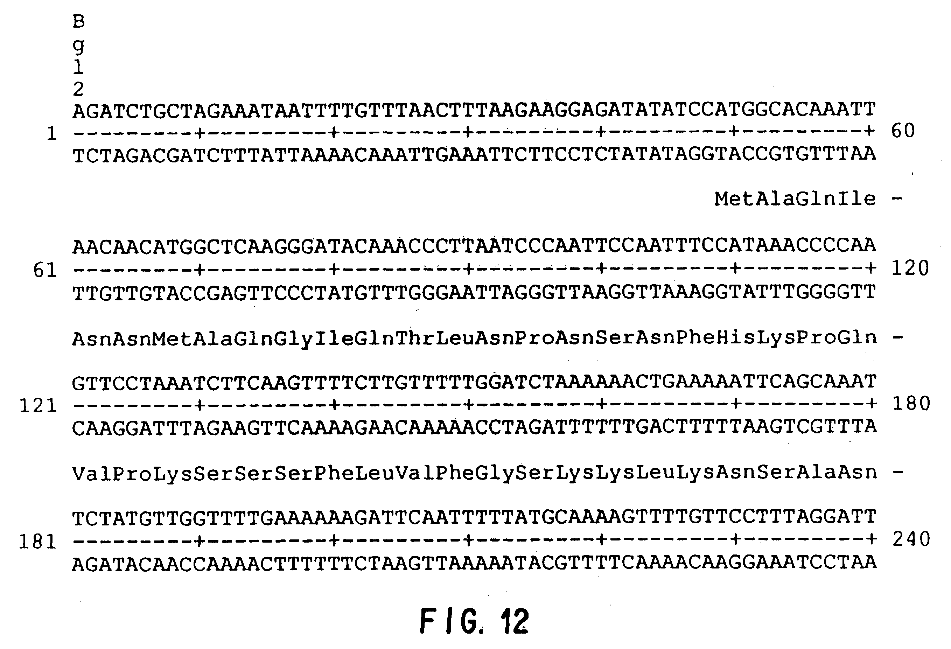

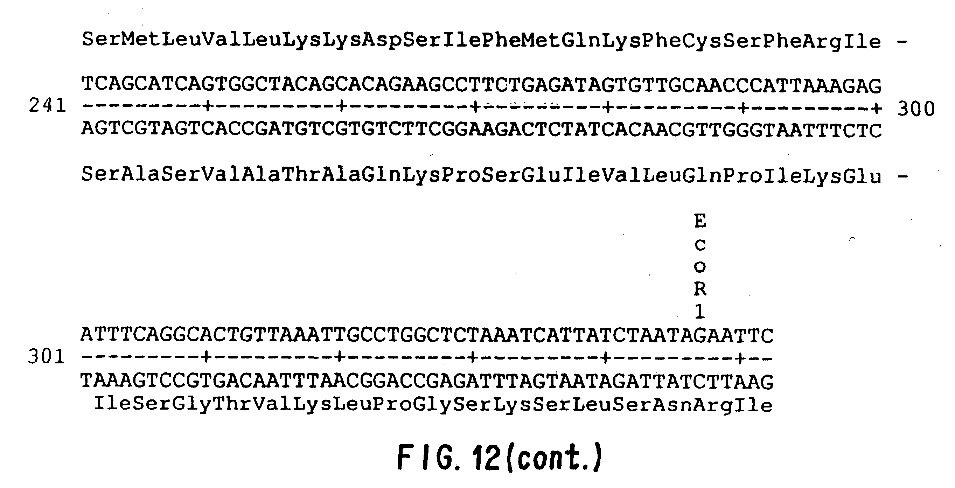

A related series of CTPs, designated as CTP4 (*Sph*I) and CTP5 (*Eco*RI), based on the Petunia EPSPS CTP and gene were also fused to the *Sph*I*-* and *Eco*RI-modified CP4 EPSPS gene sequences. The *Sph*I site was added by site-directed mutagenesis to place this restriction site (and change the amino acid sequence to -Cys-Met-) at the chloroplast processing site. All of the CTP-CP4 EPSPS fusions were shown to import into chloroplasts with approximately equal efficiency. The CTP4 (SEQ ID NO:14) and CTP5 (SEQ ID NO:16) sequences are shown in Figures 11 and 12.

A CTP2-LBAA EPSPS fusion was also constructed following the modification of the N-terminus of the LBAA EPSPS gene by the addition of a *Sph*I site. This fusion was also found to be imported efficiently into chloroplasts.

By similar approaches, the CTP2-CP4 EPSPS and the CTP4-CP4 EPSPS fusion have also been shown to import efficiently into chloroplasts prepared from the leaf sheaths of corn. These results indicate that these CTP-CP4 fusions could also provide useful genes to impart glyphosate tolerance in monocot species.

Those skilled in the art will recognize that various chimeric constructs can be made which utilize the functionality of a particular CTP to import a Class II EPSPS enzyme into the plant cell chloroplast. The chloroplast import of the Class II EPSPS can be determined using the following assay.

### Chloroplast Uptake Assay

Intact chloroplasts are isolated from lettuce (*Latuca sativa,* var. longifolia) by centrifugation in Percoll/ficoll gradients as modified from Bartlett et al (1982). The final pellet of intact chloroplasts is suspended in 0.5 ml of sterile 330 mM sorbitol in 50 mM Hepes-KOH, pH 7.7, assayed for chlorophyll (Arnon, 1949), and adjusted to the final chlorophyll concentration of 4 mg/ml (using sorbitol/Hepes). The yield of intact chloroplasts from a single head of lettuce is 3-6mg chlorophyll.

A typical 300 µl uptake experiment contained 5 mM ATP, 8.3 mM unlabeled methionine, 322 mM sorbitol, 58.3 mM Hepes-KOH (pH 8.0), 50 µl reticulocyte lysate translation products, and intact chloroplasts from *L. sativa* (200 µg chlorophyll). The uptake mixture is gently rocked at room temperature (in 10 x 75 mm glass tubes) directly in front of a fiber optic illuminator set at maximum light intensity (150 Watt bulb). Aliquot samples of the uptake mix (about 50 µl) are removed at various times and fractionated over 100 µl silicone-oil gradients (in 150 µl polyethylene tubes) by centrifugation at 11,000 X g for 30 seconds. Under these conditions, the intact chloroplasts form a pellet under the silicone-oil layer and the incubation medium (containing the reticulocyte lysate) floats on the surface. After centrifugation, the silicone-oil gradients are immediately frozen in dry ice. The chloroplast pellet is then resuspended in 50-100 µl of lysis buffer (10 mM Hepes-KOH pH 7.5, 1 mM PMSF, 1 mM benzamidine, 5 mM e-amino-n-caproic acid, and 30 µg/ml aprotinin) and centrifuged at 15,000 X g for 20 minutes to pellet the thylakoid membranes. The clear supernatant (stromal proteins) from this spin, and an aliquot of the reticulocyte lysate incubation medium from each uptake experiment, are mixed with an equal volume of 2X SDS-PAGE sample buffer for electrophoresis (Laemmli, 1970).

SDS-PAGE is carried out according to Laemmli (1970) in 3-17% (w/v) acrylamide slab gels (60 mm X 1.5 mm) with 3% (w/v) acrylamide stacking gels (5 mm X 1.5 mm).-The gel is fixed for 20-30 min in a solution with 40% methanol and 10% acetic acid. Then, the gel is soaked in EN³HANCE^{™} (DuPont) for 20-30 minutes, followed by drying the gel on a gel dryer. The gel is imaged by autoradiography, using an intensifying screen and an overnight exposure to determine whether the CP4 EPSPS is imported into the isolated chloroplasts.

### PLANT TRANSFORMATION

Plants which can be made glyphosate tolerant by practice of the present invention include, but are not limited to soybean, cotton, corn, canola, oil seed rape, flax, sugarbeet, sunflower, potato, tobacco, tomato, wheat, rice, alfalfa and lettuce as well as various tree, nut and vine species.

A double-stranded DNA molecule of the present invention ("chimeric gene") can be inserted into the genome of a plant by any suitable method. Suitable plant transformation vectors include those derived from a Ti plasmid of *Agrobacterium tumefaciens,* as well as those disclosed, e.g., by Herrera-Estrella (1983), Bevan (1984), Klee (1985) and EPO publication 120,516 (Schilperoort et al.). In addition to plant transformation vectors derived from the Ti or root-inducing (Ri) plasmids of *Agrobacterium,* alternative methods can be used to insert the DNA constructs of this invention into plant cells. Such methods may involve, for example, the use of liposomes, electroporation, chemicals that increase free DNA uptake, free DNA delivery via microprojectile bombardment, and transformation using viruses or pollen.

### Class II EPSPS Plant transformation vectors

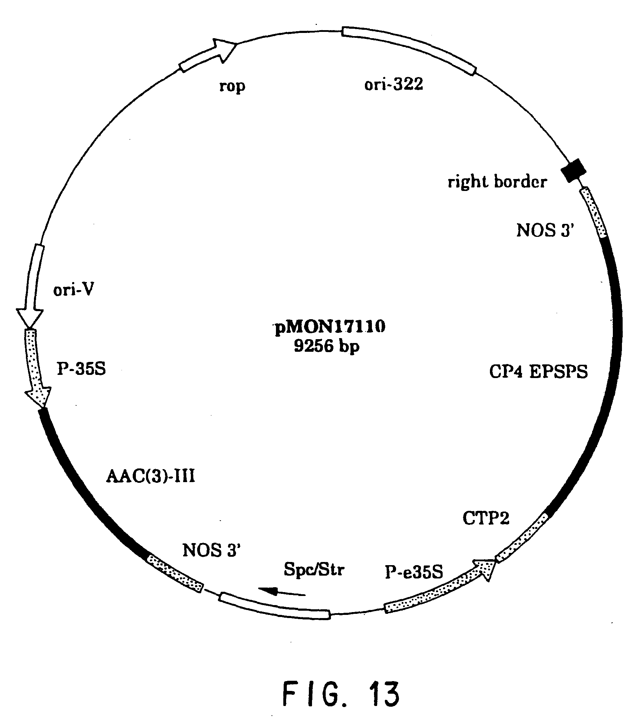

Class II EPSPS DNA sequences may be engineered into vectors capable of transforming plants by using known techniques. The following description is meant to be illustrative and not to be read in a limiting sense. One of ordinary skill in the art would know that other plasmids, vectors, markers, promoters, etc. would be used with suitable results. The CTP2-CP4 EPSPS fusion was cloned as a *Bgl*II*-Eco*RI fragment into the plant vector pMON979 (described below) to form pMON17110, a map of which is presented in Figure 13. In this vector the CP4 gene is expressed from the enhanced CaMV35S promoter (E35S; Kay et al. 1987). A FMV35S promoter construct (pMON17116) was completed in the following way: The *Sal*I-*Not*I and the *Not*I*-Bgl*II fragments from pMON979 containing the Spc/AAC(3)-III/onV and the pBR322/Right Border/NOS 3'/CP4 EPSPS gene segment from pMON17110 were ligated with the *Xho*I-*Bgl*II FMV35S promoter fragment from pMON981. These vectors were introduced into tobacco, cotton and canola..

A series of vectors was also completed in the vector pMON977 in which the CP4 EPSPS gene, the CTP2-CP4 EPSPS fusion, and the CTP3-CP4 fusion were cloned as *Bg*/II*-Sac*I fragments to form pMON17124, pMON17119, and pMON17120, respectively. These plasmids were introduced into tobacco. A pMON977 derivative containing the CTP2-LBAA EPSPS gene was also completed (pMON17206) and introduced into tobacco.

The pMON979 plant transformation/expression vector was derived from pMON886 (described below) by replacing the neomycin phosphotransferase typell (KAN) gene in pMON886 with the 0.89 kb fragment containing the bacterial gentamicin-3-N-acetyltransferase type III (AAC(3)-III) gene (Hayford et al., 1988). The chimeric P-35S/AA(3)-III/NOS 3' gene encodes gentamicin resistance which permits selection of transformed plant cells. pMON979 also contains a 0.95 kb expression cassette consisting of the enhanced CaMV 35S promoter (Kay et al., 1987), several unique restriction sites, and the NOS 3' end (P-En-CaMV35S/NOS 3'). The rest of the pMON979 DNA segments are exactly the same as in pMON886.

Plasmid pMON886 is made up of the following segments of DNA. The first is a 0.93 kb *Ava*l to engineered-*Eco*RV fragment isolated from transposon Tn7 that encodes bacterial spectinomycin/streptomycin resistance (Spc/Str), which is a determinant for selection in *E. coli* and *Agrobacterium tumefaciens.* This is joined to the 1.61 kb segment of DNA encoding a chimeric kanamycin resistance which permits selection of transformed plant cells. The chimeric gene (P-35S/KAN/NOS 3') consists of the cauliflower mosaic virus (CaMV) 35S promoter, the neomycin phosphotransferase typell (KAN) gene, and the 3'-nontranslated region of the nopaline synthase gene (NOS 3') (Fraley et al., 1983). The next segment is the 0.75 kb *ori*V containing the origin of replication from the RK2 plasmid. It is joined to the 3.1 kb *Sal*l to Pvul segment of pBR322 (ori322) which provides the origin of replication for maintenance in *E. coli* and the *bom* site for the conjugational transfer into the *Agrobacterium tumefaciens* cells. The next segment is the 0.36 kb *Pvul* to *Bcl*I from pTiT37 that carries the nopaline-type T-DNA right border (Fraley et al., 1985).

The pMON977 vector is the same as pMON981 except for the presence of the P-En-CaMV35S promoter in place of the FMV35S promoter (see below).

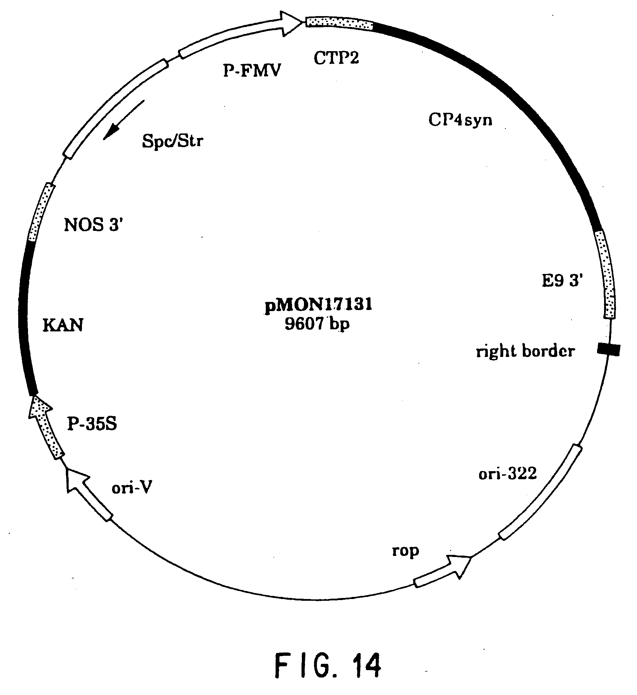

The pMON981 plasmid contains the following DNA segments: the 0.93 kb fragment isolated from transposon Tn7 encoding bacterial spectinomycin/streptomycin resistance [Spc/Str; a determinant for selection in *E. coli* and *Agrobacterium tumefaciens* (Fling et al., 1985)]: the chimeric kanamycin resistance gene engineered for plant expression to allow selection of the transformed tissue, consisting of the 0.35 kb cauliflower mosaic virus 35S promoter (P-35S) (Odell et al., 1985), the 0.83 kb neomycin phosphotransferase typell gene (KAN), and the 0.26 kb 3'-nontranslated region of the nopaline synthase gene (NOS 3') (Fraley et al., 1983): the 0.75 kb origin of replication from the RK2 plasmid *(ori*V*)* (Stalker et al., 1981); the 3.1 kb *Sal*I to *Pvu*I segment of pBR322 which provides the origin of replication for maintenance in *E. coli (ori-322)* and the *bom* site for the conjugational transfer into the *Agrobacterium tumefaciens* cells, and the 0.36 kb *Pvu*I to *Bcl*I fragment from the pTiT37 plasmid containing the nopaline-type T-DNA right border region (Fraley et al., 1985). The expression cassette consists of the 0.6 kb 35S promoter from the figwort mosaic virus (P-FMV35S) (Gowda et al., 1989) and the 0.7 kb 3' non-translated region of the pea rbcS-E9 gene (E9 3') (Coruzzi et al., 1984, and Morelli et al., 1985). The 0.6 kb Sspl fragment containing the FMV35S promoter (Figure 1) was engineered to place suitable cloning sites downstream of the transcriptional start site. The CTP2-CP4syn gene fusion was introduced into plant expression vectors (including pMON981, to form pMON17131; Figure 14) and transformed into tobacco, canola, potato, tomato, sugarbeet, cotton, lettuce, cucumber, oil seed rape, poplar, and *Arabidopsis.*

The plant vector containing the Class II EPSPS gene may be mobilized into any suitable *Agrobacterium* strain for transformation of the desired plant species. The plant vector may be mobilized into an ABI *Agrobacterium* strain. A suitable ABI strain is the A208 *Agrobacterium tumefaciens* carrying the disarmed Ti plasmid pTiC58 (pMP90RK) (Koncz and Schell, 1986). The Ti plasmid does not carry the T-DNA phytohormone genes and the strain is therefore unable to cause the crown gall disease. Mating of the plant vector into ABI was done by the triparental conjugation system using the helper plasmid pRK2013 (Ditta et al., 1980). When the plant tissue is incubated with the ABI : :plant vector conjugate, the vector is transferred to the plant cells by the virfunctions encoded by the disarmed pTiC58 plasmid. The vector opens at the T-DNA right border region, and the entire plant vector sequence may be inserted into the host plant chromosome. The pTiC58 Ti plasmid does not transfer to the plant cells but remains in the *Agrobacterium.*

### Class II EPSPS free DNA vectors

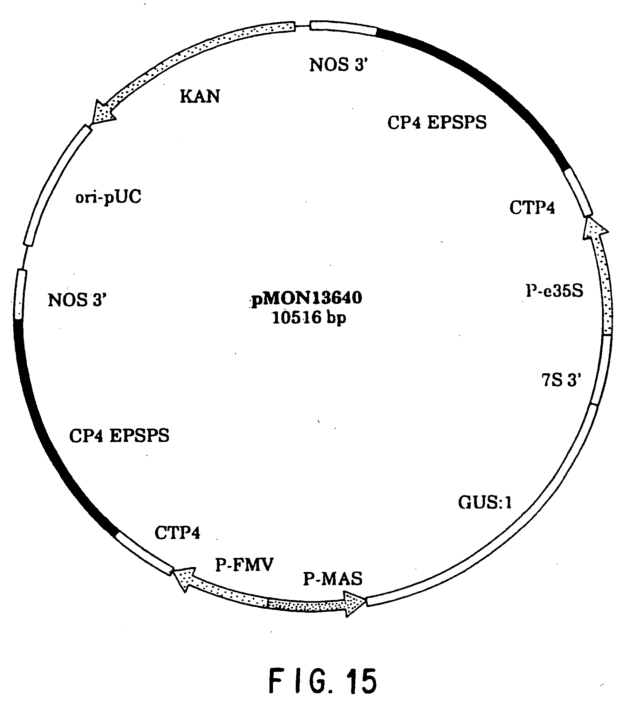

Class II EPSPS genes may also be introduced into plants through direct delivery methods. A number of direct delivery vectors were completed for the CP4 EPSPS gene. The vector pMON13640, a map of which is presented in Figure 15, is described here. The plasmid vector is based on a pUC plasmid (Vieira and Messing, 1987) containing, in this case, the *npt*II gene (kanamycin resistance; KAN) from Tn903 to provide a selectable marker in *E. coli.* The CTP4-EPSPS gene fusion is expressed from the P-FMV35S promoter and contains the NOS 3' polyadenylation sequence fragment and from a second cassette consisting of the E35S promoter, the CTP4-CP4 gene fusion and the NOS 3' sequences. The scoreable GUS marker gene (Jefferson et al. 1987) is expressedfrom the mannopine synthase promoter (P-MAS; Velten et al., 1984) and the soybean 7S storage protein gene 3' sequences (Schuler et al., 1982). Similar plasmids could also be made in which CTP-CP4 EPSPS fusions are expressed from the enhanced CaMV35S promoter or other plant promoters. Other vectors could be made that are suitable for free DNA delivery into plants and such are within the skill of the art and contemplated to be within the scope of this disclosure.

### PLANT REGENERATION

When expression of the Class II EPSPS gene is achieved in transformed cells (or protoplasts), the cells (or protoplasts) are regenerated into whole plants. Choice of methodology for the regeneration step is not critical, with suitable protocols being available for hosts from Leguminosae (alfalfa, soybean, clover, etc.), Umbelliferae (carrot, celery, parsnip), Cruciferae (cabbage, radish, rapeseed, etc.), Cucurbitaceae (melons and cucumber), Gramineae (wheat, rice, corn, etc.), Solanaceae (potato, tobacco, tomato, peppers), various floral crops as well as various trees such as poplar or apple, nut crops or vine plants such as grapes. See, e.g., Ammirato, 1984; Shimamoto, 1989; Fromm, 1990: Vasil, 1990.

The following examples are provided to better elucidate the practice of the present invention and should not be interpreted in any way to limit the scope of the present invention. Those skilled in the art will recognize that various modifications, truncations, etc. can be made to the methods and genes described herein while not departing from the spirit and scope of the present invention.

In the examples that follow, EPSPS activity in plants is assayed by the following method. Tissue samples were collected and immediately frozen in liquid nitrogen. One gram of young leaf tissue was frozen in a mortar with liquid nitrogen and ground to a fine powder with a pestle. The powder was then transferred to a second mortar, extraction buffer was added (1 ml/gram), and the sample was ground for an additional 45 seconds. The extraction buffer for Canola consists of 100 mM Tris, 1 mM EDTA, 10 % glycerol, 5 mM DTT, 1 mM BAM, 5 mM ascorbate, 1.0 mg/ml BSA, pH 7.5 (4°C). The extraction buffer for tobacco consists of 100 mM Tris, 10 mM EDTA, 35 mM KCI, 20 % glycerol, 5 mM DTT, 1 mM BAM, 5 mM ascorbate, 1.0 mg/ml BSA, pH 7.5 (4°C). The mixture was transferred to a microfuge tube and centrifuged for 5 minutes. The resulting supematants were desalted on spin G-50 (Pharmacia) columns, previously equilibrated with extraction buffer (without BSA), in 0.25 ml aliquots. The desalted extracts were assayed for EPSP synthase activity by radioactive HPLC assay. Protein concentrations in samples were determined by the BioRad microprotein assay with BSA as the standard.

Protein concentrations were determined using the BioRad Microprotein method. BSA was used to generate a standard curve ranging from 2 - 24 µg. Either 800 µl of standard or diluted sample was mixed with 200 µl of concentrated BioRad Bradford reagent. The samples were vortexed and read at A(595) after ~ 5 minutes and compared to the standard curve.

EPSPS enzyme assays contained HEPES (50 mM), shikimate-3-phosphate (2 mM), NH₄ molybdate (0.1 mM) and KF (5 mM), with or without glyphosate (0.5 or 1.0 mM). The assay mix (30 µl) and plant extract (10µl) were preincubated for 1 minute at 25°C and the reactions were initiated by adding ¹⁴C-PEP (1 mM). The reactions were quenched after 3 minutes with 50 µl of 90% EtOH/0.1 M HOAc, pH 4.5. The samples were spun at 6000 rpm and the resulting supernatants were analyzed for ¹⁴C-EPSP production by HPLC. Percent resistant EPSPS is calculated from the EPSPS activities with and without glyphosate.

The percent conversion of ¹⁴C labeled PEP to ¹⁴C EPSP was determined by HPLC radioassay using a C18 guard column (Brownlee) and an AX100 HPLC column (0.4 X 25 cm, Synchropak) with 0.28 M isocratic potassium phosphate eluant, pH 6.5, at 1 ml/min. Initial velocities were calculated by multiplying fractional turnover per unit time by the initial concentration of the labeled substrate (1 mM). The assay was linear with time up to ~ 3 minutes and 30% tumover to EPSPS. Samples were diluted with 10 mM Tris, 10% glycerol, 10 mM DTT, pH 7.5 (4°C) if necessary to obtain results within the linear range.

In these assays DL-dithiotheitol (DTT), benzamidine (BAM), and bovine serum albumin (BSA, essentially globulin free) were obtained from Sigma. Phospho*enol*pyruvate (PEP) was from Boehringer Mannheim and phospho*enol-*[1-¹⁴C]pyruvate (28 mCi/mmol) was from Amersham.

### EXAMPLE 1

Transformed tobacco plants have been generated with a number of the Class 11 EPSPS gene vectors containing the CP4 EPSPS DNA sequence as described above with suitable expression of the EPSPS. These transformed plants exhibit glyphosate tolerance imparted by the Class II CP4 EPSPS.

Transformation of tobacco employs the tobacco leaf disc transformation protocol which utilizes healthy leaf tissue about 1 month old. After a 15-20 minutes surface sterilization with 10% Clorox plus a surfactant, the leaves are rinsed 3 times in sterile water. Using a sterile paper punch, leaf discs are punched and placed upside down on MS104 media (MS salts 4.3 g/l, sucrose 30 g/l, B5 vitamins 500X 2 ml/l, NAA 0.1 mg/l, and BA 1.0 mg/l) for a 1 day preculture.

The discs are then inoculated with an overnight culture of a disarmed *Agrobacterium* ABI strain containing the subject vector that had been diluted 1/5 (ie: about 0.6 OD). The inoculation is done by placing the discs in centrifuge tubes with the culture. After 30 to 60 seconds, the liquid is drained off and the discs were blotted between sterile filter paper. The discs are then placed upside down on MS104 feeder plates with a filter disc to co-culture.

After 2-3 days of co-culture, the discs are transferred, still upside down, to selection plates with MS104 media. After 2-3 weeks, callus tissue formed, and individual clumps are separated from the leaf discs. Shoots are cleanly cut from the callus when they are large enough to be distinguished from stems. The shoots are placed on hormone-free rooting media (MSO: MS salts 4.3 g/l, sucrose 30 g/l, and B5 vitamins 500X 2 ml/l) with selection for the appropriate antibiotic resistance. Root formation occurred in 1-2 weeks. Any leaf callus assays are preferably done on rooted shoots while still sterile. Rooted shoots are then placed in soil and kept in a high humidity environment (ie: plastic containers or bags). The shoots are hardened off by gradually exposing them to ambient humidity conditions.

### Expression of CP4 EPSPS protein in transformed plants

Tobacco cells were transformed with a number of plant vectors containing the native CP4 EPSPS gene, and using different promoters and/or CTP's. Preliminary evidence for expression of the gene was given by the ability of the leaf tissue from antibiotic selected transformed shoots to recallus on glyphosate. In some cases, glyphosate tolerant callus was selected directly following transformation. The level of expression of the CP4 EPSPS was determined by the level of glyphosate tolerant EPSPS activity (assayed in the presence of 0.5 mM glyphosate) or by Western blot analysis using a goat anti-CP4 EPSPS antibody. The Western blots were quantitated by densitometer tracing and comparison to a standard curve established using purified CP4 EPSPS. These data are presented as % soluble leaf protein. The data from a number of transformed plant lines and transformation vectors are presented in Table VI below.

**Table VI**

| **Expression of CP4 EPSPS in transformed tobacco tissue** | | |
|---|---|---|
| Vector | Plant # | CP4 EPSPS (% leaf protein) |
| pMON17110 | 25313 | 0.02 |
| pMON17110 | 25329 | 0.04 |
| pMON17116 | 25095 | 0.02 |
| pMON17119 | 25106 | 0.09 |
| pMON17119 | 25762 | 0.09 |
| pMON17119 | 25767 | 0.03 |

| | | |
|---|---|---|
| Glyphosate tolerant EPSPS activity was also demonstrated in leaf extracts for these plants. | | |

Glyphosate tolerance has also been demonstrated at the whole plant level in transformed tobacco plants. In tobacco, Rₒ transformants of CTP2-CP4 EPSPS were sprayed at 0.4 Ib/acre (0.448 kg/hectare), a rate sufficient to kill control non-transformed tobacco plants corresponding to a rating of 3, 1 and 0 at days 7, 14 and 28, respectively, and were analyzed vegetatively and reproductively (Table VII).

**Table VII**

| **Glyphosate tolerance in R**_{**o**} **tobacco CP4 transformants** | | | | |
|---|---|---|---|---|
| Spray rate = 0.4 lb/acre (0.448 kg/hectare) | | | | |
| Vector/Plant # | Score* | | | |
| | Vegetative | | | Fertile |
| | day7 | day 14 | day 28 | |
| pMON17110/25313 | 6 | 4 | 2 | no |
| pMON17110/25329 | 9 | 10 | 10 | yes |
| pMON17119/25106 | 9 | 9 | 10 | yes |

| | | | | |
|---|---|---|---|---|
| Plants are evaluated on a numerical scoring system of 0-10 0 where a vegetative score of 10 represents no damage relative to non-sprayed controls and 0 represents a dead plant. Reproductive scores (Fertile) are determined at 28 days after spraying and are evaluated as to whether or not the plant is fertile. | | | | |

### EXAMPLE 2

Canola plants were transformed with the pMON17110, pMON17116, and pMON17131 vectors and a number of plant lines of the transformed canola were obtained which exhibit glyphosate tolerance.

### Plant Material

Seedlings of *Brassica napus* cv *Westar* were established in 2 inch (~5 cm) pots containing Metro Mix 350. They were grown in a growth chamber at 24°C, 16/8 hour photoperiod, light intensity of 400 uEm⁻²sec⁻¹ (HID lamps). They were fertilized with Peters 20-10-20 General Purpose Special. After 2 1/2 weeks they were transplanted to 6 inch (~ 15 cm) pots and grown in a growth chamber at 15/10°C day/night temperature, 16/8 hour photoperiod, light intensity of 800 uEm⁻²sec⁻¹ (HID lamps). They were fertilized with Peters 15-30-15 Hi-Phos Special.

### Transformation/Selection/Regeneration

Four terminal internodes from plants just prior to bolting or in the process of bofting but before flowering were removed and surfaced sterilized in 70% v/v ethanol for 1 minute, 2% w/v sodium hypochlorite for 20 minutes and rinsed 3 times with sterile deionized water. Stems with leaves attached could be refrigerated in moist plastic bags for up to 72 hours prior to sterilization. Six to seven stem segments were cut into 5mm discs with a Redco Vegetable Slicer 200 maintaining orientation of basal end.

The *Agrobacterium* was grown overnight on a rotator at 24°C in 2mls of Luria Broth containing 50mg/l kanamycin, 24mg/l chloramphenicol and 100mg/l spectinomycin. A 1:10 dilution was made in MS (Murashige and Skoog) media giving approximately 9x10⁸ cells per ml. This was confirmed with optical density readings at 660 mu. The stem discs (explants) were inoculated with 1.0ml of *Agrobacterium* and the excess was aspirated from the explants.

The explants were placed basal side down in petri plates containing 1/10X standard MS salts, B5 vitamins, 3% sucrose, 0.8% agar, pH 5.7, 1.0mg/l 6-benzyladenine (BA). The plates were layered with 1.5ml of media containing MS salts, B5 vitamins, 3% sucrose, pH 5.7, 4.0mg/l p-chlorophenoxyacetic acid, 0.005mg/l kinetin and covered with sterile filter paper.

Following a 2 to 3 day co-culture, the explants were transferred to deep dish petri plates containing MS salts, B5 vitamins, 3% sucrose, 0.8% agar, pH 5.7, 1mg/l BA, 500mg/l carbenicillin, 50mg/l cefotaxime, 200 mg/l kanamycin or 175mg/l gentamicin for selection. Seven explants were placed on each plate. After 3 weeks they were transferred to fresh media, 5 explants per plate. The explants were cultured in a growth room at 25°C, continuous light (Cool White).

### Expression Assay

After 3 weeks shoots were excised from the explants. Leaf recallusing assays were initiated to confirm modification of R₀ shoots. Three tiny pieces of leaf tissue were placed on recallusing media containing MS salts, B5 vitamins, 3% sucrose, 0.8% agar, pH 5.7, 5.0mg/l BA, 0.5mg/l naphthalene acetic acid (NAA), 500mg/l carbenicillin, 50mg/l cefotaxime and 200mg/l kanamycin or gentamicin or 0.5mM glyphosate. The leaf assays were incubated in a growth room under the same conditions as explant culture. After 3 weeks the leaf recallusing assays were scored for herbicide tolerance (callus or green leaf tissue) or sensitivity (bleaching).

### Transplantation

At the time of excision, the shoot stems were dipped in Rootone® and placed in 2 inch (~5 cm) pots containing Metro-Mix 350 and placed in a closed humid environment. They were placed in a growth chamber at 24°C, 16/8 hour photoperiod, 400 uEm⁻¹sec⁻²(HID lamps) for a hardening-off period of approximately 3 weeks.

The seed harvested from Rₒ plants is R₁ seed which gives rise to R₁ plants. To evaluate the glyphosate tolerance of an Rₒ plant, its progeny are evaluated. Because an Rₒ plant is assumed to be hemizygous at each insert location, selfing results in maximum genotypic segregation in the R₁. Because each insert acts as a dominant allele, in the absence of linkage and assuming only one hemizygous insert is required for tolerance expression, one insert would segregate 3:1, two inserts, 15:1, three inserts 63:1, etc. Therefore, relatively few R₁ plants need be grown to find at least one resistant phenotype.

Seed from an Rₒ plant is harvested, threshed, and dried before planting in a glyphosate spray test. Various techniques have been used to grow the plants for R₁ spray evaluations. Tests are conducted in both greenhouses and growth chambers. Two planting systems are used: ~ 10 cm pots or plant trays containing 32 or 36 cells. Soil used for planting is either Metro 350 plus three types of slow release fertilizer or plant Metro 350. Irrigation is either overhead in greenhouses or sub-irrigation in growth chambers. Fertilizer is applied as required in irrigation water. Temperature regimes appropriate for canola were maintained. A sixteen hour photoperiod was maintained. At the onset of flowering, plants are transplanted to ~15 cm pots for seed production.

A spray "batch" consists of several sets of R₁ progenies all sprayed on the same date. Some batches may also include evaluations of otherthan R₁ plants. Each batch also includes sprayed and unsprayed non-transgenic genotypes representing the genotypes in the particular batch which were putatively transformed. Also included in a batch is one or more non-segregating transformed genotypes previously identified as having some resistance.

Two-six plants from each individual Rₒ progeny are not sprayed and serve as controls to compare and measure the glyphosate tolerance, as well as to assess any variability not induced by the glyphosate. When the other plants reach the 2-4 leaf stage, usually 10 to 20 days after planting, glyphosate is applied at rates varying from 0.28 to 1.12 kg/ha, depending on objectives of the study. Low rate technology using low volumes has been adopted. A laboratory track sprayer has been calibrated to deliver a rate equivalent to field conditions.

A scale of 0 to 10 is used to rate the sprayed plants for vegetative resistance. The scale is relative to the unsprayed plants from the same Rₒ plant. A 0 is death, while a 10 represents no visible difference from the unsprayed plant. A higher number between 0 and 10 represents progressively less damage as compared to the unsprayed plant. Plants are scored at 7, 14, and 28 days after treatment (DAT), or until bolting, and a line is given the average score of the sprayed plants within an Rₒ plant family.

Six integers are used to qualitatively describe the degree of reproductive damage from glyphosate:

| | |
|---|---|
| 0: | No floral bud development |
| 2: | Floral buds present, but aborted prior to opening |
| 4: | Flowers open, but no anthers, or anthers fail to extrude past petals |
| 6: | Sterile anthers |
| 8: | Partially sterile anthers |
| 10: | Fully fertile flowers |

Plants are scored using this scale at or shortly after initiation of flowering, depending on the rate of floral structure development.

### Expression of EPSPS in Canola

After the 3 week period, the transformed canola plants were assayed for the presence of glyphosate tolerant EPSPS activity (assayed in the presence of glyphosate at 0.5mM). The results are shown in Table VIII.

**Table VIII**

| **Expression of CP4 EPSPS in transformed Canola plants** | | |
|---|---|---|
| Vector Control | Plant # | % resistant EPSPS activity of leaf extract (at 0.5 mM glyphosate) |
| | | 0 |
| pMON17110 | 41 | 47 |
| pMON17110 | 52 | 28 |
| pMON17110 | 71 | 82 |
| pMON17110 | 104 | 75 |
| pMON17110 | 172 | 84 |
| pMON17110 | 177 | 85 |
| pMON17110 | 252 | 29* |
| pMON17110 | 350 | 49 |
| pMON17116 | 40 | 25 |
| pMON17116 | 99 | 87 |
| pMON17116 | 175 | 94 |
| pMON17116 | 178 | 43 |
| pMON17116 | 182 | 18 |
| pMON17116 | 252 | 69 |
| pMON17116 | 298 | 44* |
| pMON17116 | 332 | 89 |
| pMON17116 | 383 | 97 |
| pMON17116 | 395 | 52 |

| | | |
|---|---|---|
| assayed in the presence of 1.0 mM glyphosate | | |

R₁ transformants of canola were then grown in a growth chamber and sprayed with glyphosate at 0.56 kg/ha (kilogram/hectare) and rated vegetatively. These results are shown in Table IXA - IXC. It is to be noted that expression of glyphosate resistant EPSPS in all tissues is preferred to observe optimal glyphosate tolerance phenotype in these transgenic plants. In the Tables below, only expression results obtained with leaf tissue are described.

**Table IXA**

| **Glyphosate tolerance in Class II EPSPS canola R**_{**1**} **transformants** | | | |
|---|---|---|---|
| (pMON17110 = P-E35S; pMON17116 = P-FMV35S; R1 plants; Spray rate = 0.56 kg/ha) | | | |
| | % resistant | Vegetative Score** | |
| Vector/Plant No. | EPSPS* | day 7 | day 14 |
| Control Westar | 0 | 5 | 3 |
| pMON17110/41 | 47 | 6 | 7 |
| pMON17110171 | 82 | 6 | 7 |
| pMON17110/177 | 85 | 9 | 10 |
| pMON17116/40 | 25 | 9 | 9 |
| pMON17116/99 | 87 | 9 | 10 |
| pMON17116/175 | 94 | 9 | 10 |
| pMON17116/178 | 43 | 6 | 3 |
| pMON17116/182 | 18 | 9 | 10 |
| pMON17116/383 | 97 | 9 | 10 |

| | | | |
|---|---|---|---|
| * % resistant EPSPS activity in the presence of 0.5 mM glyphosate | | | |
| ** A vegetative score of 10 indicates no damage, a score of 0 is given to a dead plant. | | | |

**Table IXB**

| **Glyphosate tolerance in Class II EPSPS canola R**_{**1**} **transformants** | | |
|---|---|---|
| (pMON17131 = P-FMV35S; R1 plants, Spray rate = 0.84 kg/ha) | | |
| Vector/Plant No. | Vegetative score** | Reproductive score |
| | day 14 | day 28 |
| 17131/78 | 10 | 10 |
| 17131/102 | 9 | 10 |
| 17131/115 | 9 | 10 |
| 17131/116 | 9 | 10 |
| 17131/157 | 9 | 10 |
| 17131/169 | 10 | 10 |
| 17131/255 | 10 | 10 |
| control Westar | 1 | 0 |

| | | |
|---|---|---|
| ** A vegetative score of 10 indicates no damage, a score of 0 is given to a dead plant. | | |

**Table IXC**

| **Glyphosate tolerance in Class I EPSPS canola transformants** | | | |
|---|---|---|---|
| (P-E35S: R2 Plants: Spray rate = 0.28 kg/ha) | | | |
| | % resistant | Vegetative Score** | |
| Vector/Plant No. | EPSPS* | day 7 | day 14 |
| Control Westar | 0 | 4 | 2 |
| pMON899/715 | 96 | 5 | 6 |
| pMON899/744 | 95 | 8 | 8 |
| pMON899/794 | 86 | 6 | 4 |
| pMON899/818 | 81 | 7 | 8 |
| pMON899/885 | 57 | 7 | 6 |

| | | | |
|---|---|---|---|
| * % resistant EPSPS activity in the presence of 0.5 mM glyphosate | | | |
| ** A vegetative score of 10 indicates no damage, a score of 0 is given to a dead plant. | | | |

The data obtained for the Class II EPSPS transformants may be compared to glyphosate tolerant Class I EPSP transformants in which the same promoter is used to express the EPSPS genes and in which the level of glyphosate tolerant EPSPS activity was comparable for the two types of transformants. A comparison of the data of pMON17110 [in Table IXA] and pMON17131 [Table IXB] with that for pMON899 [in Table IXC: the Class I gene in pMON899 is that from *A. thaliana* (Klee et al., 1987) in which the glycine at position 101 was changed to an alanine] illustrates that the Class II EPSPS is at least as good as that of the Class I EPSPS. An improvement in vegetative tolerance of Class II EPSPS is apparent when one takes into account that the Class II plants were sprayed at twice the rate and were tested as R₁ plants.

### EXAMPLE 3

Soybean plants were transformed with the pMON13640 (Figure 15) vector and a number of plant lines of the transformed soybean were obtained which exhibit glyphosate tolerance.

Soybean plants are transformed with pMON13640 by the method of microprojectile injection using particle gun technology as described in Christou et al. (1988). The seed harvested from Rₒ plants is R₁ seed which gives rise to R₁ plants. To evaluate the glyphosate tolerance of an Rₒ plant, its progeny are evaluated. Because an Rₒ plant is assumed to be hemizygous at each insert location, selfing results in maximum genotypic segregation in the R₁. Because each insert acts as a dominant allele, in the absence of linkage and assuming only one hemizygous insert is required for tolerance expression, one insert would segregate 3:1, two inserts, 15:1, three inserts 63:1, etc. Therefore, relatively few R₁ plants need be grown to find at least one resistant phenotype.

Seed from an Rₒ soybean plant is harvested, and dried before planting in a glyphosate spray test. Seeds are planted into 4 inch (~5cm) square pots containing Metro 350. Twenty seedlings from each Ro plant is considered adequate for testing. Plants are maintained and grown in a greenhouse environment. A 12.5-14 hour photoperiod and temperatures of 30°C day and 24°C night is regulated. Water soluble Peters Pete Lite fertilizer is applied as needed.

A spray "batch" consists of several sets of R₁ progenies all sprayed on the same date. Some batches may also include evaluations of other than R₁ plants. Each batch also includes sprayed and unsprayed non-transgenic genotypes representing the genotypes in the particular batch which were putatively transformed. Also included in a batch is one or more non-segregating transformed genotypes previously identified as having some resistance.

One to two plants from each individual R₀ progeny are not sprayed and serve as controls to compare and measure the glyphosate tolerance, as well as to assess any variability not induced by the glyphosate. When the other plants reach the first trifoliate leaf stage, usually 2-3 weeks after planting, glyphosate is applied at a rate equivalent of 128 oz./acre (8.895kg/ha) of Roundup®. A laboratory track sprayer has been calibrated to deliver a rate equivalent to those conditions.

A vegetative score of 0 to 10 is used. The score is relative to the unsprayed progenies from the same Rₒ plant. A 0 is death, while a 10 represents no visible difference from the unsprayed plant. A higher number between 0 and 10 represents progressively less damage as compared to the unsprayed plant. Plants are scored at 7, 14, and 28 days after treatment (DAT). The data from the analysis of one set of transformed and control soybean plants are described on Table X and show that the CP4 EPSPS gene imparts glyphosate tolerance in soybean also.

**Table X**

| **Glyphosate tolerance in Class I EPSPS soybean transformants** | | | |
|---|---|---|---|
| (P-E35S, P-FMV35S; RO plants; Spray rate = 128 oz./acre) | | | |
| Vector/Plant No. | Vegetative score | | |
| | day 7 | day 14 | day 28 |
| 13640/40-11 | 5 6 | | 7 |
| 13640/40-3 | 9 | 10 | 10 |
| 13640/40-7 | 4 | 7 | 7 |
| control A5403 | 2 | 1 | 0 |
| controlA5403 | 1 | 1 | 0 |

### EXAMPLE 4

The CP4 EPSPS gene may be used to select transformed plant material directly on media containing glyphosate. The ability to select and to identify transformed plant material depends, in most cases, on the use of a dominant selectable marker gene to enable the preferential and continued growth of the transformed tissues in the presence of a normally inhibitory substance. Antibiotic resistance and herbicide tolerance genes have been used almost exclusively as such dominant selectable marker genes in the presence of the corresponding antibiotic or herbicide. The nptII/kanamycin selection scheme is probably the most frequently used. It has been demonstrated that CP4 EPSPS is also a useful and perhaps superior selectable marker/selection scheme for producing and identifying transformed plants.

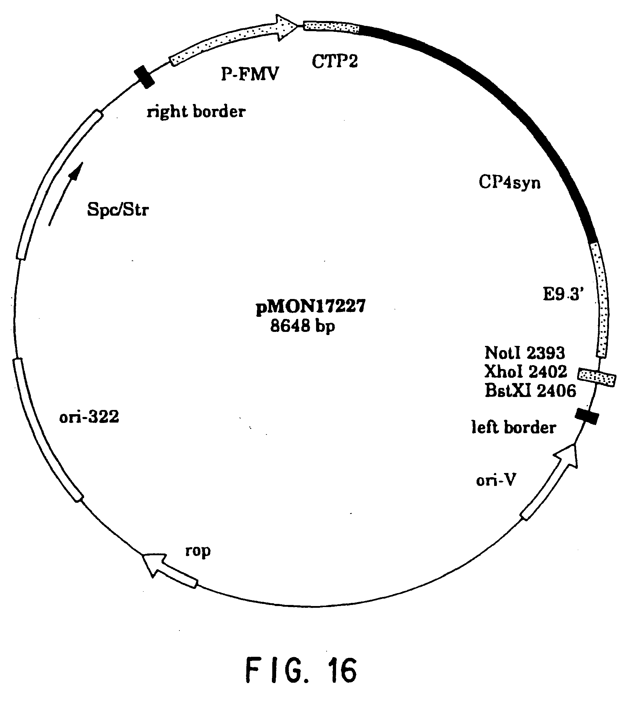

A plant transformation vector that may be used in this scheme is pMON17227 (Figure 16). This plasmid resembles many of the other plasmids described infra and is essentially composed of the previously described bacterial replicon system that enables this plasmid to replicate in *E. coli* and to be introduced into and to replicate in *Agrobacterium,* the bacterial selectable marker gene (Spc/Str), and located between the T-DNA right border and left border is the CTP2-CP4 synthetic gene in the FMV35S promoter-E9 3' cassette. This plasmid also has single sites for a number of restriction enzymes, located within the borders and outside of the expression cassette. This makes it possible to easily add other genes and genetic elements to the vector for introduction into plants.

The protocol for direct selection of transformed plants on glyphosate is outlined for tobacco. Explants are prepared for pre-culture as in the standard procedure as described in Example 1: surface sterilization of leaves from 1 month old tobacco plants (15 minutes in 10% clorox + surfactant: 3X dH₂O washes): explants are cut in 0.5 x 0.5 cm squares, removing leaf edges, mid-rib, tip, and petiole end for uniform tissue type: explants are placed in single layer, upside down, on MS104 plates + 2 ml 4COO5K media to moisten surface: pre-culture 1-2 days. Explants are inoculated using overnight culture of *Agrobacterium* containing the plant transformation plasmid that is adjusted to a titer of 1.2 X 109 bacteria/ml with 4COO5K media. Explants are placed into a centrifuge tube, the *Agrobacterium* suspension is added and the mixture of bacteria and explants is "Vortexed" on maximum setting for 25 seconds to ensure even penetration of bacteria. The bacteria are poured off and the explants are blotted between layers of dry sterile filter paper to remove excess bacteria. The blotted explants are placed upside down on MS104 plates + 2ml 4C005K media + filter disc. Co-culture is 2-3 days. The explants are transferred to MS104 + Carbenicillin 1000 mg/l + cefotaxime 100 mg/l for 3 days (delayed phase). The explants are then transferred to MS104 + glyphosate 0.05 mM + Carbenicillin 1000 mg/l + cefotaxime 100 mg/l for selection phase. At 4-6 weeks shoots are cut from callus and placed on MSO + Carbenicillin 500 mg/l rooting media. Roots form in 3-5 days, at which time leaf pieces can be taken from rooted plates to confirm glyphosate tolerance and that the material is transformed.

The presence of the CP4 EPSPS protein in these transformed tissues has been confirmed by immunoblot analysis of leaf discs. The data from one experiment with pMON17227 is presented in the following: 139 shoots formed on glyphosate from 400 explants inoculated with *Agrobacterium* ABI/pMON17227; 97 of these were positive on recallusing on glyphosate. These data indicate a transformation rate of 24 per 100 explants, which makes this a highly efficient and time saving transformation procedure for plants. Similar transformation frequencies have been obtained with pMON17131 and direct selection of transformants on glyphosate with the CP4 EPSPS genes has also been shown in other plant species, including *Arabidopsis,* potato, tomato, cotton, lettuce, and sugarbeet.

The pMON17227 plasmid contains single restriction enzyme recognition cleavage sites (Notl, Xhol, and BstXI) between the CP4 glyphosate selection region and the left border of the vector for the cloning of additional genes and to facilitate the introduction of these genes into plants.

### EXAMPLE 5

The CP4 EPSPS gene has also been introduced into Black Mexican Sweet (BMS) corn cells with expression of the protein and glyphosate resistance detected in callus.

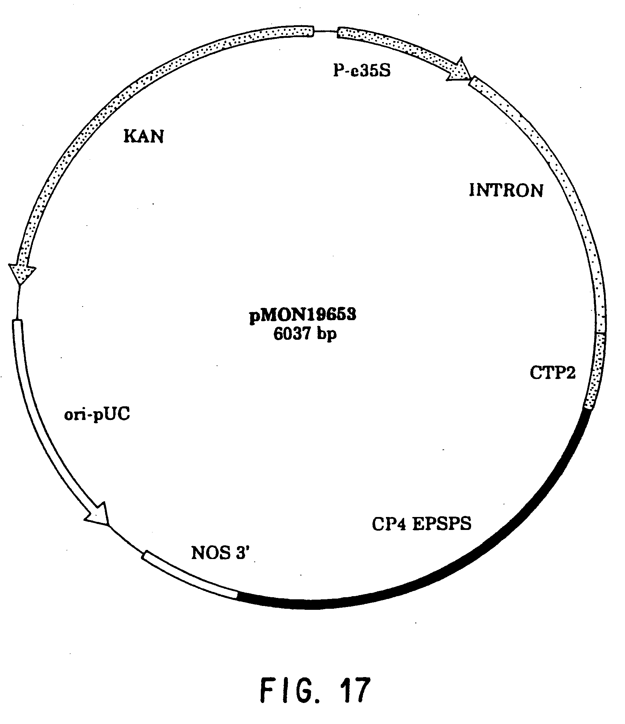

The backbone for this plasmid was a derivative of the high copy plasmid pUC119 (Viera and Messing, 1987). The 1.3Kb Fspl-Dral pUC11 9 fragment containing the origin of replication was fused to the 1.3Kb Smal-Hindlll filled fragment from pKC7 (Rao and Rogers, 1979) which contains the neomycin phosphotransferase type II gene to confer bacterial kanamycin resistance. This plasmid was used to construct a monocot expression cassette vector containing the 0.6kb cauliflower mosaic virus (CaMV) 35S RNA promoter with a duplication of the -90 to -300 region (Kay et al., 1987), an 0.8kb fragment containing an intron from a maize gene in the 5' untranslated leader region, followed by a polylinker and the 3' termination sequences from the nopaline synthase (NOS) gene (Fraley et al., 1983). A 1.7Kb fragment containing the 300bp chloroplast transit peptide from the *Arabidopsis* EPSP synthase fused in frame to the 1.4Kb coding sequence for the bacterial CP4 EPSP synthase was inserted into the monocot expression cassette in the polylinker between the intron and the NOS termination sequence to form the plasmid pMON1 9653 (Figure 17).

pMON1 9653 DNA was introduced into *Black Mexican Sweet* (BMS) cells by co-bombardment with EC9, a plasmid containing a sulfonylurea-resistant form of the maize acetolactate synthase gene. 2.5mg of each plasmid was coated onto tungsten particles and introduced into log-phase BMS cells using a PDS-1 000 particle gun essentially as described (Klein et al., 1989). Transformants are selected on MS medium containing 20ppb chlorsulfuron. After initial selection on chlorsulfuron, the calli can be assayed directly by Western blot. Glyphosate tolerance can be assessed by transferring the calli to medium containing 5mM glyphosate. As shown in Table XI, CP4 EPSPS confers glyphosate tolerance to com callus.

**Table XI.**

| **Expression of CP4 in BMS Corn Callus - pMON 19653** | |
|---|---|
| Line | CP4 expression (% extracted protein) |
| 284 | 0.006 % |
| 287 | 0.036 |
| 290 | 0.061 |
| 295 | 0.073 |
| 299 | 0.113 |
| 309 | 0.042 |
| 313 | 0.003 |

To measure CP4 EPSPS expression in corn callus, the following procedure was used: BMS callus (3 g wet weight) was dried on filter paper (Whatman#1) under vacuum, reweighed, and extraction buffer (500 µl/g dry weight: 100 mM Tris, 1 mM EDTA, 10% glycerol) was added. The tissue was homogenized with a Wheaton overhead stirrer for 30 seconds at 2.8 power setting. After centrifugation (3 minutes, Eppendorf microfuge), the supernatant was removed and the protein was quantitated (BioRad Protein Assay). Samples (50 µg/well) were loaded on an SDS PAGE gel (Jule, 3-17%) along with CP4 EPSPS standard (10 ng), electrophoresed, and transferred to nitrocellulose similarly to a previously described method (Padgette, 1987). The nitrocellulose blot was probed with goat anti-CP4 EPSPS IgG, and developed with I-125 Protein G. The radioactive blot was visualized by autoradiography Results were quantitated by densitometry on an LKB UltraScan XL laser densitomer and are tabulated below in Table X.

**Table XII.**

| **Glyphosate resistance in BMS Corn Callus using pMON 19653** | | | |
|---|---|---|---|
| Vector | Experiment | # chlorsulfuron-resistant lines | # cross-resistant to Glyphosate |
| 19653 | 253 | 120 | 81/120=67.5% |
| 19653 | 254 | 80 | 37/80=46% |
| EC9 control | 253/254 | 8 | 0/8=0% |

Improvements in the expression of Class I EPSPS could also be achieved by expressing the gene using stronger plant promoters, using better 3' polyadenylation signal sequences, optimizing the sequences around the initiation codon for ribosome loading and translation initiation, or by combination of these or other expression or regulatory sequences or factors. It would also be beneficial to transform the desired plant with a Class I EPSPS gene in conjunction with another glyphosate tolerant EPSPS gene or a gene capable of degrading glyphosate in order to enhance the glyphosate tolerance of the transformed plant.

From the foregoing, it will be seen that this invention is one well adapted to attain all the ends and objects hereinabove set forth together with advantages which are obvious and which are inherent to the invention.

It will be understood that certain features and subcombinations are of utility and may be employed without reference to other features and subcombinations. This is contemplated by and is within the scope of the claims.

Since many possible embodiments may be made of the invention without departing from the scope thereof, it is to be understood that all matter herein set forth or shown in the accompanying drawings is to be interpreted as illustrative and not in a limiting sense.

### EXAMPLE 6

The LBAA Class II EPSPS gene has been introduced into plants and also imparts glyphosate tolerance. Data on tobacco transformed with pMON17206 (infra) are presented in Table XIII.

**Table XIII -**

| **Tobacco Glyphosate Spray Test (pMON17206; E35S - CTP2-LBaa EPSPS; 0,4 lbs/ac)** | |
|---|---|
| Line | 7 Day Rating |
| 33358 | 9 |
| 34586 | 9 |
| 33328 | 9 |
| 34606 | 9 |
| 33377 | 9 |
| 34611 | 10 |
| 34607 | 10 |
| 34601 | 9 |
| 34589 | 9 |
| Samsum (Control) | 4 |

### BIBLIOGRAPHY

Alton, N.K. and Vapnek, D. (1979) Nature 282:864-869.

Ammirato, P.V. et al. Handbook of Plant Cell Culture - Crop Species. Macmillan Publ. Co. (1984).

Arnon, D.I. Plant Physiol. 24:1-15 (1949).

Bachmann, B. J. et al., Microb. Rev., 44:1-56 (1980).

Bartlett, S.G., Grossman, A.R., and Chua, N.H. (1982) in Methods in Chloroplast Molecular Biology, pp. 1081-1091. M. Edelman, R.B., Hallick, and Chua, N.H.,eds.

Bevan, M. (1984) Nucleic Acids Res. 12 (22): 8711-8721.

Birnboim, H. C. and Doly, J. (1979) A rapid alkaline extraction procedure for screening recombinant plasmid DNA. Nucl. Acids. Res. 7:1513-1525.

Boyer, H. W. and Rolland-Dussoix, D. (1969) A complementation analysis of the restriction and modification of DNA in *Escherichia coli.* J. Mol. Biol. 41:459.

Christou, P., D. E. McCabe, and W.F. Swain (1988) Stable transformation of Soybean Callus by DNA-Coated Gold Particles. Plant Physiol. 87:671-674.

Coruzzi, G., Broglie, R., Edwards, C., and Chua, N.H. (1984). Tissue-specific and light-regulated expression of a pea nuclear gene encoding the small subunit of ribulose-1,5-bisphosphate carboxylase. EMBO J 3:1671.

della-Cioppa, G., Bauer, S. C., Klein, B. K, Shah, D. M., Fraley, R. T. and Kishore G. K. (1986) Translocation of the precursor of 5-*enol*pyruvylshikimate-3-phosphate synthase into chloroplasts of higher plants *in vitro.* Proc. Natl. Acad Sci. USA 83: 6873-6877.

della-Cioppa, G., Bauer, S. C., Taylor, M. T., Rochester, D. E., Klein, B. K., Shah, D. M., Fraley, R. T. and Kishore G. M. (1987) Targeting a herbicide-resistant enzyme from *Escherichia coli* to chloroplasts of higher plants. Bio/Technology 5:579.584.

Devereux, J., Haeberli, P. and Smithies, O. (1984) A comprehensive set of sequence analysis programs for the VAX. Nucl. Acids. Res. 12:387-395.

Ditta, G., Stanfield, S., Corbin, D., and Helinski, D.R. (1980) Broad host range DNA cloning system for Gram-Negative bacteria: construction of a gene bank of *Rhizobium meliloti.* Proc Natl Acad Sci USA 77,7347-7351.

Duncan, K., Edwards, R.M., Coggins, J.R. (1987) The pentafunctional *aroM* enzyme of *Saccharomyces cerevisiae* is a mosaic of monofunctional domains. Biochem. J. 246: 375-386.

Dunn, J.J. and Studier, F.W., (1983) J. Mol. Biol. 166:477-535.

Fitzgibbon, J. E. (1988) *Pseudomonas* sp. strain PG2982: uptake of glyphosate and cloning of a gene which confers increased resistance to glyphosate. Ph. D. Dissertation, Louisiana State University.

Fitzgibbon, E. F. and Braymer, H. D. (1990) Cloning of a gene from *Pseudomonas* sp. PG2982 conferring increased glyphosate resistance Appl. Environ. Microbiol. 56: 3382-3388.

Fling, M.E., Kopf, J., and Richards, C. (1985). Nucleotide sequence of the transposon Tn7 gene encoding an aminoglycoside-modifying enzyme, 3°(9)-O-nucleotidyltransferase. Nucleic Acids Research 13 no.19, 7095-7106.

Fraley, R.T., Rogers, S.G., Horsch, R.B., Sanders, P.R. Flick, J.S., Adams, S.P., Bittner, M.L., Brand, L.A., Fink, C.L., Fry, J.S., Galluppi, G.R., Goldberg, S.B., Hoffman, N.L., and Woo, S.C. 1983. Expression of bacterial genes in plant cells. Proc. Natl. Acad. Sci. USA 80:4803-4807.

Fraley, R. T., Rogers, S. G., Horsch, R. B., Eichholtz D. A., Flick, J. S., Fink, C. L., Hoffmann, N. L. and Sanders, P. R. (1985) The SEV system: a new disarmed Ti plasmid vector system for plant transformation.

Fromm, M., (1990) UCLA Symposium on Molecular Strategies for Crop Improvement, April 16-22, 1990. Keystone, CO.

Gasser, C. S., Winter, J. A. Hironaka, C. M. and Shah, D. M. (1988) Structure, expression, and evolution of the 5-enolpyruvylshikimate 3-phosphate synthase genes of petunia and tomato. J. Biol. Chem. 263: 4280-4289.

Gowda, S., Wu, F.C., and Shepard, R.J. (1989). Identification of promoter sequences for the major RNA transcripts of figwort mosaic and peanut chlorotic streak viruses (caulimovirus group). Journal of Cellular Biochemistry supplement 13D, 301 (Abstract).

Hallas, L. E., Hahn, E. M. and Korndorfer, C. (1988) Characterization of microbial traits associated with glyphosate biodegradation in industrial activated sludge. J. Industrial Microbiol. 3: 377-385.

Hayford, M. B., Medford, J. I., Hoffman, N. L., Rogers, S. G. and Klee, H. J. (1988) Development of a plant transformation selection system based on expression of genes encoding gentamicin acetyltransferases. Plant Physiol. 86: 1216-1222.

Herrera-Estrella, L., et al. (1983) Nature 303:209 Horsch, R.B. and H. Klee. (1986) Proc. Natl. Acad. Sci. U.S.A. 83:4428-32.

Heitkamp, M. A., Hallas, L. and Adams, W. J. (1990) Biotreatment of industrial wastewater with immobilized microorganisms - Presented in Session 11, Paper S40, Society for Industrial Microbiology Annual Meeting, Orlando, Florida, July 29-August 3, 1990.

Hohn, B. and Collins J. (1980) A small cosmid for efficient cloning of large DNA fragments. Gene 11: 291-298.

Hunkapiller, M. W., Hewick, R. M., Dreyer, R. J., and Hood, L. (1983) *Methods Enzymol.* 91, 399-413.

Jefferson, R.A., Kavanaugh, T.A. and Bevan, M.W., EMBO J., 6:3901-3907 (1987).

Kay, R., Chan, A., Daly, M. and McPherson, J. 1987. Duplication of the CaMV 35S promoter sequence creates a strong enhancer for plants. Science 236, 1299-1302.

Kishore, G., Shah, D., Padgette, S., della-Cioppa, G., Gasser, C., Re, D., Hironaka, C., Taylor, M., Wibbenmeyer, J., Eichholtz, D., Hayford, M., Hoffman, N., Delannay, X., Horsch, R., Klee, H., Rogers, S., Rochester, D., Brundage, L., Sanders, P. and Fraley, R. T. (1988) 5-Enolpyruvylshikimate 3-phosphate synthase: From Biochemistry to genetic engineering of glyphosate tolerance, in Biotechnology for Crop Protection ACS Symposium series No. 379. Eds. Hedlin P. A., Menn, J. J. and Hollingsworth, R. M. pp. 37-48.

Kishore, G. and Shah, D. (1988) Ann. Rev. Biochem. 57:627-663.

Kishore, G. M., Brundage, L., Kolk, K, Padgette, S. R., Rochester, D., Huynh, Q. K. and della-Cioppa, G. (1986) Fed. Proc. 45: 1506.

Klee, H.J., et al. (1985) Bio/Technology 3:637-42.

Klee, H. J., Muskopf, Y. M. and Gasser, C. S. (1987) Cloning of an *Arabidopsis thaliana* gene encoding 5-enolpyruvyl-shikimate-3-phosphate synthase: sequence analysis and manipulation to obtain glyphosate tolerant plants. Mol. Gen. Genet. 210: 437-442.

Klein, T.M., Kornstein, L., Sanford, J.C., and Fromm, M.E. 1989. Genetic transformation of maize cells by particle bombardment. Plant Phys. 91:440-444.
Koncz, C. and Schell, J. (1986) The promoter of T_{L}-DNA gene 5 controls the tissue-specific expression of chimeric genes carried by a novel type of *Agrobacterium* binary vector. Mol. Gen. Genet. 204:383-396.

Kunkel,T. A., Roberts, J. D. and Zakour, R. A. (1987) Rapid and efficient site-specific mutagenesis without phenotypic selection. Methods Enzymol. 154:367.

Laemmli, U.K., "Cleavage of structural proteins during the assembly of the head of the bacteriophage T4" Nature, 227:680 (1970).

Maniatis, T.. Fritsch, E. F. and Sambrook, J. (1982) Molecular Cloning: a laboratory manual. Cold Spring Harbor Laboratory, Cold Spring Harbor, New York.

Maskell, D.J., Morrissey, P. and Dougan, G. (1988) Cloning and nucleotide sequence of the aroA gene of *Bordetella pertussis.* J. Bacteriol. 170:2467-2471.

Miller, J. H. (1972). Experiments in Molecular Genetics. Cold Spring Harbor Laboratory, Cold Spring Harbor, New York.

Moore, J. K., Braymer, H. D. and Larson, A. D. (1983) Isolation of a *Pseudomonas* sp. which utilizes the phosphonate herbicide glyphosate. Appl. Environ. Microbiol. 46: 316-320.

Morelli, G., Nagy, F., Fraley, R.T., Rogers, S.G., and Chua, N. H. (1985). A short conserved sequence is involved in the light-inducibility of a gene encoding ribulose 1,5-bisphosphate carboxylase small subunit of pea. Nature 315, 200-204.

Odell, J.T., Nagy, F., and Chua, N.H. (1985). Identification of DNA sequences required for activity of the cauliflower mosaic virus 35S promoter. Nature 313, 810-812.

Olins, P. O., Devine, C. S., Rangwala, S. H. and Kavka, K. S. (1988) Gene 73: 227-235.

Padgette, S. R., Huynh, Q. K., Borgmeyer, J., Shah, D. M., Brand, L. A., Re, D. B., Bishop, B. F., Rogers, S. G., Fraley, R. T., and Kishore, G. (1987) Bacterial expression and isolation of *Petunia hybrida* 5-*enol*-pyruvylshikimate-3-phosphate synthase. Arch. Biochem. Biophys. 258, 564-573.

Padgette, S. R., Huynh, Q. K., Aykent, S., Sammons, R. D., Sikorski, J. A., and Kishore, G. M. (1988) J. Biol. Chem. 263, 1798-1802.

Quinn, J. P., Peden, J. M. M. and Dick, E. (1988) Glyphosate tolerance and utilization by the microflora of soils treated with the herbicide. Appl. Microbiol. Biotechnol. 29: 511-516.

Rao, R.N. and Rogers, S.G. 1979. Plasmid pKC7: A vector containing ten restriction endonuclease sites suitable for cloning DNA segments. Gene 7:79.

Rogers, S.G., Brand, L.A. Holder, S.B. Sharps, E.S. and Brackin, M.J. (1983) Amplification of the *aroA* gene from *E. coli* results in tolerance to the herbicide glyphosate. Appl. Environ. Microbiol. 46:37-43.

Sambrook, J., Fritsch, E.F. and Maniatis, T., Molecular Cloning: A Laboratory Manual, Cold Spring Harbor Laboratory Press, Cold Spring Harbor, New York (1989).

Schuler, M. A., Schmitt, E. S. and Beachy, R.N. (1982) Nucleic Acids Res. 10:8225-8244.

Schulz, A., Kruper, A. and Amrhein, N. (1985) Differential sensitivity of bacterial 5-enolpyruvylshikimate-3-phosphate synthases to the herbicide glyphosate. FEMS Microbiol. Lett. 28: 297-301.

Schulz, A., Sost, D. and Amrhein, D. (1984) Arch. Microbiol. 137: 121-123.

Shah, D., Horsch, R., Klee, H., Kishore, G., Winter, J., Turner, N., Hironaka, C., Sanders, P., Gasser, C., Aykent, S., Siegal, N., Rogers, S., and Fraley, R. (1986). Engineering herbicide tolerance in transgenic plants. Science 233, 478-481.

Shimamoto, K. et al. (1989) Natureu338:274-276.

Sost, D., Schulz, A. and Amrhein, N. (1984) FEBS Lett. 173: 238-241.

Sost, D. and Amrhein, N. (1990) Substitution of Gly-96 to Ala in the 5-enolpyruvylshikimate 3-phosphate synthase of *Klebsiella pneumoniae* results in greatly reduced affinity for the herbicide glyphosate. Arch. Biochem. Biophys. 282: 433-436.

Stalker, D.M., Thomas, C.M., and Helinski, D.R. (1981). Nucleotide sequence of the region of the origin of replication of the broad host range plasmid RK2. Mol Gen Genet 181: 8-12.

Stalker, D. M., Hiatt, W. R. and Comai, L. (1985) A single amino acid substitution in the enzyme 5-enolpyruvylshikimate 3-phosphate synthase confers resistance to glyphosate. J. Biol. Chem. 260: 4724-4728.

Tabor, S. and Richardson, C. C. (1985) A bacteriophage T7 RNA polymerase/promoter system for controlled exclusive expression of specific genes. Proc. Natl. Acad. Sci. USA 82: 1074-1078.

Talbot, H. W., Johnson, L. M. and Munnecke, D. M. (1984) Glyphosate utilization by *Pseudomonas* sp. and *Alcaligenes* sp. isolated from environmental sources. Current Microbiol. 10: 255-260.

Talmadge, K., and Gilbert, W., "Construction of plasmid vectors with unique Pstl cloning sites in the signal sequence coding region" Gene, (12) 235-241 (1980).

Vasil, V., F. Redway and I. Vasil., Bio/Technology 8:429-434 (1990).

Velten, J., Velten, R., Hain, R. and Schell, J. (1984) EMBO J. 3:2723-2730.

Viera, J. and Messing, J. 1987. Production of single-stranded plasmid DNA. Methods Enzym. 153:3-11.

Wibbenmeyer, J., Brundage, L., Padgette, S. R., Likos, J. J., and Kishore, G. M. (1988) Biochem. Biophys. Res. Comm. 153, 760-766.

Wong, E. Y., Seetharam, R., Kotts, C. E., Heeren, R. A., Klein, B. K., Braford, S. R., Mathis, K. J., Bishop, B. F., Siegel, N. R., Smith, C. E. and Tacon, W. C. (1988) Expression of excreted insulin-like growth factor-1 in *Escherichia coli*. Gene 68: 193-203.

## Claims

1. An isolated DNA sequence encoding a Class II EPSPS enzyme, said enzyme being an EPSPS enzyme having a Kₘ for phosphoenolpyruvate (PEP) between 1-150 µM and a Kᵢ(glyphosate)/Kₘ(PEP) ratio between 3-500, which enzyme is capable of reacting with antibodies raised against a Class II EPSPS enzyme selected from the group consisting of the enzymes of SEQ ID NO:3 and SEO ID NO:5.

2. A DNA sequence of claim 1 wherein said Kₘ for phosphoenolpyruvate is between 2-25 µM.

3. A DNA sequence of claim 1 wherein said Kᵢ/Kₘ ratio is between 6-250.

4. An isolated DNA sequence encoding a protein which exhibits EPSPS activity wherein said protein is capable of reacting with antibodies raised against a Class II EPSPS enzyme selected from the group consisting of the enzymes of SEQ ID NO:3 and SEQ ID NO:5.

5. The DNA sequence of Claim 4 wherein said antibodies are raised against a Class II EPSPS enzyme of SEQ ID NO:3.

6. A DNA sequence encoding a Class II EPSPS enzyme selected from the group consisting of SEQ ID NO:3 and SEQ ID NO:5.

7. A recombinant, double-stranded DNA molecule compnsing in sequence:
a) a promoter which functions in plant cells to cause the production of an RNA sequence;
b) a structural DNA sequence that causes the production of an RNA sequence which encodes a Class II EPSPS enzyme capable of reacting with antibodies raised against a Class II EPSPS enzyme selected from the group consisting of the enzymes of SEQ ID No:3 and SEQ ID No:5: and
c) a 3' non-translated region which functions in plant cells to cause the addition of a stretch of polyadenyl nucleotides to the 3' end of the RNA sequence
where the promoter is heterologous with respect to the structural DNA sequence and adapted to cause sufficient expression of the fusion polypeptide to enhance the glyphosate tolerance of a plant cell transformed with said DNA molecule.

8. The DNA molecule of Claim 7 in which said structural DNA sequence encodes a fusion polypeptide comprising an amino-terminal chloroplast transit peptide and a Class II EPSPS enzyme.

9. The DNA molecule of Claim 8 wherein said structural DNA sequence encoding a Class II EPSPS enzyme is selected from the group consisting of SEQ ID NO:2, SEQ ID NO:4 and SEQ ID NO:6.

10. The DNA molecule of Claim 9 wherein said sequence is from SEQ ID NO:2.

11. A DNA molecule of Claim 8 in which the promoter is a plant DNA virus promoter.

12. A DNA molecule of Claim 11 in which the promoter is selected from the group consisting of CaMV35S and FMV35S promoters.

13. A DNA molecule of Claim 7 in which said structural DNA encodes a Class II EPSPS enzyme selected from the group consisting of SEQ ID NO:3 and SEQ ID NO:5.

14. A method of producing genetically transformed plants which are tolerant toward glyphosate herbicide, comprising the steps of.
a) inserting into the genome of a plant cell a recombinant, double-stranded DNA molecule comprising:
i) a promoter which functions in plant cells to cause the production of an RNA sequence,
ii) a structural DNA sequence that causes the production of an RNA sequence which encodes a 5 fusion polypeptide comprising an amino terminal chloroplast transit peptide and a Class II EPSPS enzyme capable of reacting with antibodies raised against a Class II EPSPS enzyme selected from the group consisting of the enzymes of SEQ ID NO:3 and SEQ ID NO:5,
iii) a 3' non-translated DNA sequence which functions in plant cells to cause the addition of a stretch of polyadenyl nucleotides to the 3' end of the RNA sequence
where the promoter is heterologous with respect to the structural DNA sequence and adapted to cause sufficient expression of the fusion polypeptide to enhance the glyphosate tolerance of a plant cell transformed with said gene;
b) obtaining a transformed plant cell; and
c) regenerating from the transformed plant cell a genetically transformed plant which has increased tolerance to glyphosate herbicide.

15. The method of Claim 14 wherein said structural DNA sequence encoding a Class II EPSPS enzyme is selected from the group consisting of SEQ ID NO:2, SEQ ID NO:4, and SEQ ID NO:6.

16. The DNA molecule of Claim 15 wherein said sequence is that as set forth in SEQ ID NO:2.

17. A method of Claim 14 in which the promoter is from a plant DNA virus.

18. A method of Claim 17 in which the promoter is selected from the group consisting of CaMV35S and FMV35S promoters.

19. A method of claim 14 in which said structural DNA encodes a Class II EPSPS enzyme selected from the group consisting of SEQ ID NO:3 and SEQ ID NO:5.

20. A glyphosate tolerant plant cell comprising a DNA molecule of Claims 8, 9, 12 or 13.

21. A glyphosate tolerant plant cell of Claim 20 in which the promoter is a plant DNA virus promoter.

22. A glyphosate tolerant plant cell of Claim 21 in which the promoter is selected from the group consisting of CaMV35S and FMV35S promoters.

23. A glyphosate tolerant plant cell of Claim 20 selected from the group consisting of com, wheat, rice, soybean, cotton, sugarbeet, oilseed rape, canola, flax, sunflower, potato, tobacco, tomato, alfalfa, poplar, pine, apple and grape.

24. A glyphosate tolerant plant comprising plant cells of Claim 20.

25. A glyphosate tolerant plant of Claim 24 in which the promoter is from DNA plant virus promoter.

26. A glyphosate tolerant plant of Claim 25 in which the promoter is selected from the group consisting of CaMV35S and FMV35S promoters.

27. A glyphosate tolerant plant of Claim 26 selected from the group consisting of com, wheat, rice, soybean, cotton, sugarbeet, oilseed rape, canola, flax, sunflower, potato, tobacco, tomato, alfalfa, poplar, pine, apple and grape.

28. A method for selectively controlling weeds in a field containing a crop having planted crop seeds or plants comprising the steps of:
a) planting said crop seeds or plants which are glyphosate tolerant as a result of a recombinant double-stranded DNA molecule being inserted into said crop seed or plant, said DNA molecule having:
a promoter which functions in plant cells to cause the production of an RNA sequence,
ii) a structural DNA sequence that causes the production of an RNA sequence which encodes a polypeptide which comprises an amino terminal chloroplast transit peptide and a Class II EPSPS enzyme capable of reacting with antibodies raised against a Class II EPSPS enzyme selected from the group consisting of the enzymes of SEQ ID NO:3 and SEQ ID NO:5,
iii) a 3' non-translated DNA sequence which functions in plant cells to cause the addition of a stretch of
polyadenyl nucleotides to the 3' end of the RNA sequence
where the promoter is heterologous with respect to the structural DNA sequence and adapted to cause sufficient expression of the fusion polypeptide to enhance the glyphosate tolerance of a plant cell transformed with said gene: and
b) applying to said crop and weeds in said field a sufficient amount of glyphosate herbicide to control said weeds without significantly affecting said crop.

29. The method of Claim 28 wherein said structural DNA sequence encoding a Class II EPSPS enzyme is selected from the sequences as set forth in SEQ ID NO:2. SEQ ID NO:4 or SEQ ID NO:6.

30. A method of Claim 29 in which said DNA molecule contains a structural DNA sequence from SEQ ID NO:2.

31. A method of Claim 30 in which said DNA molecule further comprises a promoter selected from the group consisting of the CaMV35SS and FMV35S promoters.

32. A method of Claim 31 in which the crop plant is selected from the group consisting of com, wheat, rice, soybean, cotton, sugarbeet, oilseed rape, canola, flax, sunflower, potato, tobacco, tomato, alfalfa, poplar, pine, apple and grape.

33. The method of claim 28 wherein said structural DNA sequence encodes a Class II EPSPS enzyme selected from the group consisting of SEQ ID NO:3 and SEQ ID NO:5.

## Patentansprüche

1. Isolierte DNA-Sequenz, welche für ein EPSPS-Enzym der Klasse II codiert, welches Enzym ein EPSPS-Enzym mit einer Kₘ für Phosphoenolpyruvat (PEP) zwischen 1 und 150 µM und einem Kᵢ(Glyphosat)/Kₘ(PEP)-Verhältnis zwischen 3 und 500 ist, welches Enzym mit Antikörpern gegen ein EPSPS-Enzym der Klasse II, ausgewählt aus der Gruppe bestehend aus den Enzymen der SEQ ID NO: 3 und SEQ ID NO: 5, reagieren kann.

2. DNA-Sequenz nach Anspruch 1, worin die Kₘ für Phosphoenolpyruvat zwischen 2 und 25 µM ist.

3. DNA-Sequenz nach Anspruch 1, worin das Kᵢ/Kₘ-Verhältnis zwischen 6 und 250 ist.

4. Isolierte DNA-Sequenz, die für ein Protein codiert, welches EPSPS-Aktivität zeigt, wobei das Protein mit Antikörpern gegen ein EPSPS-Enzym der Klasse II, ausgewählt aus der Gruppe bestehend aus den Enzymen der SEQ ID NO: 3 und SEQ ID NO: 5, reagieren kann.

5. DNA-Sequenz nach Anspruch 4, wobei die Antikörper gegen ein EPSPS-Enzym der Klasse II der SEQ ID NO: 3 sind.

6. DNA-Sequenz, die für ein EPSPS-Enzym der Klasse II, ausgewählt aus der Gruppe bestehend aus SEQ ID NO: 3 und SEQ ID NO: 5 codiert.

7. Rekombinantes, doppelsträngiges DNA-Molekül, welches in Sequenz aufweist:
a) ein Promotor, der in Pflanzenzellen zur Bewirkung der Produktion einer RNA-Sequenz fungiert;
b) eine strukturelle DNA-Sequenz, die die Produktion einer RNA-Sequenz bewirkt, welche für ein EPSPS-Enzym der Klasse II codiert, welches mit Antikörpern gegen ein EPSPS-Enzym der Klasse II, ausgewählt aus der Gruppe bestehend aus den Enzymen SEQ ID NO: 3 und SEQ ID NO: 5, reagieren kann, und
c) einen 3' nicht-translatierten Bereich, welcher in Pflanzenzellen zur Bewirkung der Addition eines Abschnittes von Polyadenylnucleotiden am 3'-Ende der RNA-Sequenz fungiert,
wobei der Promotor in bezug auf die strukturelle DNA-Sequenz heterolog ist und so beschaffen ist, daß er eine ausreichende Expression des Fusionspolypeptids bewirkt, um die Glyphosat-Toleranz einer mit diesem DNA-Molekül transformierten Pflanzenzelle zu verbessern.

8. DNA-Molekül nach Anspruch 7, in welchem die strukturelle DNA-Sequenz für ein Fusionspolypeptid codiert, das ein Amino-terminales Chloroplasten-Transitpeptid und ein EPSPS-Enzym der Klasse II aufweist.

9. DNA-Molekül nach Anspruch 8, wobei die für ein EPSPS-Enzym der Klasse II kodierende strukturelle DNA-Sequenz ausgewählt ist aus der Gruppe bestehend aus SEQ ID NO: 2, SEQ ID NO: 4 und SEQ ID NO: 6.

10. DNA-Molekül nach Anspruch 9, wobei diese Sequenz aus SEQ ID NO: 2 ist.

11. DNA-Molekül nach Anspruch 8, wobei der Promotor ein Pflanzen-DNA-Virus-Promotor ist.

12. DNA-Molekül nach Anspruch 11, in welchem der Promotor ausgewählt ist aus der Gruppe bestehend aus CaMV35S und FMV35S-Promotoren.

13. DNA-Molekül nach Anspruch 7, in welchem diese strukturelle DNA für ein EPSPS-Enzym, ausgewählt aus der Gruppe bestehend aus SEQ ID NO: 3 und SEQ ID NO: 5, codiert.

14. Verfahren zur Herstellung genetisch transformierter Pflanzen, die eine Toleranz gegenüber Glyphosat-Herbizid aufweisen, welches die Schritte umfaßt:
a) Inserieren eines rekombinanten, doppelsträngigen DNA-Moleküls in das Genom einer Pflanzenzelle, wobei das DNA-Molekül umfaßt:
i) einen Promotor, der in Pflanzenzellen zur Bewirkung der Produktion einer RNA-Sequenz fungiert,
ii) eine strukturelle DNA-Sequenz, welche die Produktion einer RNA-Sequenz bewirkt, die für ein Fusionspolypeptid codiert, welches ein Amino-terminales Chloroplasten-Transitpeptid und ein EPSPS-Enzym der Klasse II umfaßt, das mit Antikörpern gegen ein EPSPS-Enzym, ausgewählt aus der Gruppe bestehend aus den Enzymen der SEQ ID NO: 3 und SEQ ID NO: 5, reagieren kann,
iii) eine 3'-nicht-translatierte DNA-Sequenz, die in Pflanzenzellen zur Bewirkung der Addition eines Abschnitts von Polyadenylnucleotiden an das 3'-Ende der RNA-Sequenz fungiert,
wobei der Promotor in bezug auf die strukturelle DNA-Sequenz heteolog ist und so beschaffen ist, daß er eine ausreichende Expression des Fusionspolypeptids bewirkt, um die Glyphosat-Toleranz einer mit diesem Gen transformierten Pflanzenzelle zu verbessern;
b) Erhalten einer transformierten Pflanzenzelle; und
c) Regenerieren einer genetisch transformierten Pflanze aus der transformierten Pflanzenzelle, welche Pflanze eine erhöhte Toleranz gegenüber Glyphosat-Herbizid aufweist.

15. Verfahren nach Anspruch 14, worin die für ein EPSPS-Enzym der Klasse II codierende DNA-Sequenz ausgewählt ist aus der Gruppe bestehend aus SEQ ID NO: 2, SEQ ID NO: 4 und SEQ ID NO: 6.

16. DNA-Molekül nach Anspruch 15, worin die Sequenz jene ist, wie sie in SEQ ID NO: 2 angeführt ist.

17. Verfahren nach Anspruch 14, in welchem der Promotor aus einem Pflanzen-DNA-Virus stammt.

18. Verfahren nach Anspruch 17, in welchem der Promotor ausgewählt ist aus der Gruppe bestehend aus CaMV35S- und FMV35S-Promotoren.

19. Verfahren nach Anspruch 14, in welchem die strukturelle DNA für ein EPSPS-Enzym der Klasse II, ausgewählt aus der Gruppe bestehend aus SEQ ID NO: 3 und SEQ ID NO: 5, codiert.

20. Glyphosat-tolerante Pflanzenzelle umfassend ein DNA-Molekül der Ansprüche 8, 9, 12 oder 13.

21. Glyphosat-tolerante Pflanzenzelle nach Anspruch 20, in welcher der Promotor ein Pflanzen-DNA-virus-Promotor ist.

22. Glyphosat-tolerante Pflanzenzelle nach Anspruch 21, in welcher der Promotor ausgewählt ist aus der Gruppe bestehend aus CaMV35S- und FMV35S-Promotoren.

23. Glyphosat-tolerante Pflanzenzelle nach Anspruch 20, ausgewählt aus der Gruppe bestehend aus Mais, Weizen, Reis, Sojabohne, Baumwolle, Zuckerrübe, Ölsaatenraps, Canola, Flachs, Sonnenblume, Kartoffel, Tabak, Tomate, Luzerne, Pappel, Kiefer, Apfel und Traube.

24. Glyphosat-tolerante Pflanze umfassend Pflanzenzellen nach Anspruch 20.

25. Glyphosat-tolerante Pflanze nach Anspruch 24, in welcher der Promotor von einem DNA-Pflanzen-Virus-Promotor stammt.

26. Glyphosat-tolerante Pflanze nach Anspruch 25, in welcher der Promotor ausgewählt ist aus der Gruppe bestehend aus CaMV35S- und FMV35S-Promotoren.

27. Glyphosat-tolerante Pflanze nach Anspruch 24, ausgewählt aus der Gruppe bestehend aus Mais, Weizen, Reis, Sojabohne, Baumwolle, Zuckerrübe, Ölsaatenraps, Canola, Flachs, Sonnenblume, Kartoffel, Tabak, Tomate, Luzerne, Pappel, Kiefer, Apfel und Traube.

28. Verfahren zur selektiven Unkrautbekämpfung auf einem Feld, welches eine Bebauung mit gepflanzten Samen oder Pflanzen von Nutzpflanzen enthält, umfassend die Schritte
a) Pflanzung dieser Nutzpflanzen-Samen oder Pflanzen, die Glyphosat-tolerant sind, weil ein rekombinantes doppelsträngiges DNA-Molekül in den Samen oder die Pflanze der Nutzpflanze inseriert ist, wobei das DNA-Molekül aufweist:
i) einen Promotor, der in Pflanzenzellen zur Bewirkung der Produktion einer RNA-Sequenz fungiert,
ii) eine strukturelle DNA-Sequenz, welche die Produktion einer RNA-Sequenz bewirkt, die für ein Polypeptid codiert, welches ein Amino-terminales Chloroplasten-Transitpeptid und ein EPSPS-Enzym der Klasse II umfaßt, das mit Antikörpern gegen ein EPSPS-Enzym der Klasse II, ausgewählt aus der Gruppe bestehend aus den Enzymen der SEQ ID NO: 3 und SEQ ID NO: 5, reagieren kann,
iii) eine 3'-nicht-translatierte DNA Sequenz, die in Pflanzenzellen zur Bewirkung der Addition eines Abschnitts von Polyadenylnucleotiden an das 3'-Ende der RNA-Sequenz fungiert,
wobei der Promotor in bezug auf die struturelle DNA-Sequenz heterolog ist und so beschaffen ist, daß er eine ausreichende Expression des Fusionspolypeptids bewirkt, um die Glyphosat-Toleranz einer mit diesem Gen transformierten Pflanzenzelle zu verbessern; und
b) Auftragen einer ausreichenden Menge von Glyphosat-Herbizid auf die Nutzpflanzen und das Unkraut in diesem Feld, um dieses Unkraut zu bekämpfen, ohne die Nutzpflanzen wesentlich zu beeinträchtigen.

29. Verfahren nach Anspruch 28, wobei die für ein EPSPS-Enzym der Klasse II codierende strukturelle DNA-Sequenz ausgewählt ist aus den Sequenzen, wie in SEQ ID NO: 2, SEQ ID NO: 4 oder SEQ ID NO: 6 angeführt.

30. Verfahren nach Anspruch 29, in welchem das DNA-Molekül eine strukturelle DNA-Sequenz von SEQ ID NO: 2 enthält.

31. Verfahren nach Anspruch 30, in welchem das DNA-Molekül weiters einen Promotor, ausgewählt aus der Gruppe bestehend aus den CaMV35SS- und FMV35S-Promotoren, umfaßt.

32. Verfahren nach Anspruch 31, in welchem die Nutzpflanze ausgewählt aus der Gruppe bestehend aus Mais, Weizen, Reis, Sojabohne, Baumwolle, Zuckerrübe, Ölsaatenraps, Canola, Flachs, Sonnenblume, Kartoffel, Tabak, Tomate, Luzerne, Pappel, Kiefer, Apfel und Traube.

33. Verfahren nach Anspruch 28, wobei die strukturelle DNA-Sequenz für ein EPSPS-Enzym der Klasse II, ausgewählt aus der Gruppe bestehend aus SEQ ID NO: 3 und SEQ ID NO: 5, codiert.

## Revendications

1. Séquence d'ADN isolée codant pour une enzyme EPSPS de la Classe II, cette enzyme étant une enzyme EPSPS ayant un Kₘ pour le phosphoénolpyruvate (PEP) compris entre 1 et 150 µM et un rapport Kᵢ(glyphosate)/Kₘ (PEP) compris entre 3 et 500, laquelle enzyme est capable de réagir avec des anticorps formés contre une enzyme EPSPS de la Classe II choisie parmi les enzymes de SEQ ID No :3 et SEQ ID NO :5.

2. Séquence d'ADN selon la revendication 1, dans laquelle ce Kₘ pour le phosphoénolpyruvate est compris entre 2 et 25 µM.

3. Séquence d'ADN selon la revendication 1, dans laquelle ce rapport Kᵢ/Kₘ est compris entre 6 et 250.

4. Séquence d'ADN isolée codant pour une protéine qui présente une activité d'EPSPS dans laquelle cette protéine est capable de réagir avec des anticorps formés contre une enzyme EPSPS de la Classe II choisie parmi les enzymes de SEQ ID NO :3 et SEQ ID NO :5.

5. Séquence d'ADN selon la revendication 4, dans laquelle ces anticorps sont formés contre une enzyme EPSPS de la Classe II de SEQ I NO :3.

6. Séquence d'ADN codant pour une enzyme EPSPS de la Classe II choisie parmi SEQ ID NO :3 et SEQ ID NO :5.

7. Molécule d'ADN à double brin, recombinante, comprenant successivement :
a) un promoteur qui fonctionne dans les cellules végétales en provoquant la production d'une séquence d'ARN ;
b) une séquence d'ADN structural qui provoque la production d'une séquence d'ARN qui code pour une enzyme EPSPS de la Classe II capable de réagir avec des anticorps formés contre une enzyme EPSPS de la Classe II choisie parmi les enzymes SEQ ID NO :3 et SEQ ID NO :5 ;
c) une région non traduite 3' qui fonctionne dans les cellules végétales en provoquant l'addition d'un segment de polyadényle nucléotides à l'extrémité 3' de la séquence d'ARN,
dans laquelle le promoteur est hétérologue par rapport à là séquence d'ADN structural et capable de provoquer une expression du polypeptide de fusion suffisante pour augmenter la tolérance au glyphosate d'une cellule végétale transformée par cette molécule d'ADN.

8. Molécule d'ADN selon la revendication 7, dans laquelle cette séquence d'ADN structural code pour un polypeptide de fusion comprenant un peptide de transit dans les chloroplastes amino-terminal et une enzyme EPSPS de la Classe II.

9. Molécule d'ADN selon la revendication 8, dans laquelle cette séquence d'ADN structural codant pour une enzyme EPSPS de la Classe II est choisie parmi SEQ ID NO :2, SEQ ID NO :4 et SEQE ID NO :6.

10. Molécule d'ADN selon revendication 9, dans laquelle cette séquence provient de SEQ ID NO :2.

11. Molécule d'ADN selon la revendication 8, dans laquelle le promoteur est un promoteur de virus à ADN végétal.

12. Molécule d'ADN selon la revendication 11, dans laquelle le promoteur est choisi parmi les promoteurs CaMV35S et FMV35S.

13. Molécule d'ADN selon la revendication 7, dans laquelle cet ADN structural code pour une enzyme EPSPS de la Classe II choisie parmi SEQ ID NO :3 et SEQ ID NO :5.

14. Procédé de production de plantes transformées génétiquement qui sont tolérantes vis-à-vis de l'herbicide glyphosate, comprenant les étapes consistant à
a) insérer dans le génome d'une cellule végétale une molécule d'ADN à double brin, recombinante, comprenant :
i) un promoteur qui fonctionne dans les cellules végétales en provoquant la production d'une séquence d'ARN,
ii) une séquence d'ADN structural qui provoque la production d'une séquence d'ARN codant pour un polypeptide de fusion qui comprend un peptide de transit dans les chloroplastes amino-terminal et une enzyme EPSPS de la Classe II capable de réagir avec des anticorps formés contre une enzyme EPSPS de la Classe II choisie parmi les enzymes SEQ ID NO :3 et SEQ ID NO :5,
iii) une séquence d'ADN non traduite 3' qui fonctionne dans les cellules végétales en provoquant l'addition d'un segment de polyadényle nucléotides à l'extrémité 3' de la séquence d'ARN
dans laquelle le promoteur hétérologue par rapport à la séquence d'ADN structural et est capable de provoquer une expression du polypeptide de fusion suffisante pour augmenter la tolérance au glyphosate d'une cellule végétale transformé par ce gène ;
b) obtenir une cellule végétale transformée ; et
c) régénérer à partir de la cellule végétale transformée une plante transformée génétiquement qui présente une tolérance améliorée pour l'herbicide glyphosate.

15. Procédé selon la revendication 14, dans lequel cette séquence d'ADN structural codant pour une enzyme EPSPS de la Classe II est choisie parmi SEQ ID NO :2, SEQ ID NO :4 et SEQ ID NO :6.

16. Molécule d'ADN selon la revendication 15, dans laquelle cette séquence est celle indiquée dans SEQ ID NO :2.

17. Procédé selon la revendication 14, dans lequel le promoteur provient d'un virus à ADN végétal.

18. Procédé selon la revendication 17, dans lequel le promoteur est choisi parmi les promoteurs CaMV35S et FMV35S.

19. Procédé selon la revendication 14, dans lequel cet ADN structural code pour une enzyme EPSPS de la Classe II choisie parmi SEQ ID NO :3 et SEQ ID NO :5.

20. Cellule végétale tolérant le glyphosate comprenant une molécule d'ADN selon les revendications 8, 9, 12 ou 13.

21. Cellule végétale tolérant le glyphosate selon la revendication 20, dans laquelle le promoteur est un promoteur de virus à ADN végétal.

22. Cellule végétale tolérant le glyphosate selon la revendication 21, dans laquelle le promoteur est choisi parmi les promoteurs CaMV35S et FMV35S.

23. Cellule végétale tolérant le glyphosate selon la revendication 20, choisie parmi le maïs, le froment, le riz, le soja, le coton, la betterave à sucre, le colza oléagineux, le canola, le lin, le tournesol, la pomme de terre, le tabac, la tomate, la luzerne, le peuplier, le pin, le pommier et la vigne.

24. Plante tolérant le glyphosate comprenant des cellules végétales selon la revendication 20.

25. Plante tolérant le glyphosate selon la revendication 24,
dans laquelle le promoteur est un promoteur provenant d'un virus végétal à ADN.

26. Plante tolérant le glyphosate selon la revendication 25,
dans laquelle le promoteur est choisi parmi les promoteurs CaMV35S et FMV35S.

27. Plante tolérant le glyphosate selon la revendication 24, choisie parmi le maïs, le froment, le riz, le soja, le coton, la betterave à sucre le colza oléagineux, le canola, le lin, le tournesol, la pomme de terre, le tabac, la tomate, la luzerne, le peuplier, le pin, le pommier et la vigne.

28. Procédé pour lutter sélectivement contre les mauvaises herbes dans un champ contenant une récolte dans laquelle sont plantés des semences ou plants, comprenant les étapes consistant à :
a) planter ces semences ou plants de récolte qui sont tolérants vis-à-vis du glyphosate sous l'effet d'une molécule d'ADN à double brin, recombinante, insérée dans cette semence ou plant de récolte, cette molécule d'ADN ayant
i) un promoteur qui fonctionne dans les cellules végétales en provoquant la production d'une séquence d'ARN,
ii) une séquence d'ADN structural qui provoque la production d'une séquence d'ARN codant pour un polypeptide qui comprend un peptide de transit dans les chloroplastes amino-terminal et une enzyme EPSPS de la Classe II capable de réagir avec des anticorps formés contre une enzyme EPSPS de la Classe II choisie parmi les enzymes SEQ ID NO :3 et SEQ ID NO :5,
iii) une séquence d'ADN non traduite 3' qui fonctionne dans les cellules végétales en provoquant l'addition d'un segment de polyadényle nucléotides à l'extrémité de la séquence d'ARN.
dans laquelle le promoteur est hétérologue par rapport à la séquence d'ADN structural et est capable de provoquer une expression du polypeptide de fusion suffisante pour améliorer la tolérance au glyphosate d'une cellule végétale transformée par ce gène ; et
b) appliquer à cette récolte et à ces semences dans ce champ une quantité d'herbicide glyphosate suffisante pour lutter contre ces mauvaises herbes sans affecter significativement cette récolte.

29. Procédé selon la revendication 28, dans lequel cette séquence d'ADN structural codant pour une enzyme EPSPS de la Classe II est choisie parmi les séquence indiqués dans SEQ ID NO :2 SEQ ID NO :4 ou SEQ ID NO :6.

30. Procédé selon la revendication 29, dans lequel cette molécule d'ADN contient une séquence d'ADN structural provenant de SEQ ID NO :2.

31. Procédé selon la revendication 30, dans lequel cette molécule d'ADN comprend en outre un promoteur choisi parmi les promoteurs CaM35SS et FMV35S.

32. Procédé selon la revendication 31, dans lequel la plante de récolte est choisie parmi le maïs, le froment, le riz, le soja, le coton, la betterave à sucre, le colza oléagineux, la canola, le lin, le tournesol, la pomme de terre, le tabac, la tomate, la luzerne, le peuplier, le pin, le pommier et la vigne.

33. Procédé selon la revendication 28, dans lequel cette séquence d'ADN structural code pour une enzyme EPSPS de la Classe II choisie parmi SEQ ID NO :3 et SEQ ID NO :5.
